# EUROPEAN PATENT APPLICATION

(11) **EP 4 420 684 A1**
(43) Date of publication of application: **28.08.2024**
(21) Application number: 22932705.1
(22) Date of filing: 25.03.2022
(51) Int. Cl.: A61K 47/68, C07D 487/04, C07D 209/60, A61K 39/395, A61P 35/00

(54) **DNA TOXIC DIMER COMPOUND AND CONJUGATE THEREOF**

(71) Applicant: Systimmune, Inc., Redmond, WA 98052 (US)
(72) Inventor: ZHU, Yi, ChengDu, Sichuan 611130 (CN); WAN, Weili, ChengDu, Sichuan 611130 (CN); ZHUO, Shi, ChengDu, Sichuan 611130 (CN); ZHANG, Yiying, ChengDu, Sichuan 611130 (CN); ZHU, Guili, ChengDu, Sichuan 611130 (CN); YU, Tianzi, ChengDu, Sichuan 611130 (CN)
(74) Representative: Hobson, David James
(86) International application number: PCT/CN2022/082986
(87) International publication number: WO 2023/178641

(57) **Abstract**

A DNA toxic dimer compound and its conjugate or a pharmaceutically acceptable salt thereof, method of making and methods of using the compounds or conjugates in preventing or treating cancer. The conjugate may specifically bind to receptors highly expressed in tumor cells. It has good water solubility, stability, and homogeneity, and may be used to prevent or treat tumors or other diseases.

## Description

### TECHNICAL FIELD

The application relates to novel cytotoxic compounds and drugs comprising such cytotoxic compounds and cell-binding agents. More specifically, the application relates to novel benzodiazepine dimer compounds, their derivatives, intermediates, pharmaceutically acceptable salts, and conjugates thereof. The compounds can be used as drugs, in particular as antitumor drugs.

### BACKGROUND

Ligand-drug conjugate (ADC), as novel targeted drugs, generally includes three components: an antibody or antibody-like ligand, a small molecule drug, and a linker that couples the ligand to the drug. ADCs utilize the specific recognition of an antigen by an antibody to transport the drug molecules to the vicinity of the target cell and effectively release the drug molecules for therapeutic purposes. In August 2011, the U.S. Food and Drug Administration (FDA) approved ADECTEIS^{™}, a new ADC developed by Seattle Genetics, for the treatment of Hodgkin's Lymphoma and relapsed anaplastic large cell lymphoma (ALCL), which has demonstrated safety and efficacy of such drugs in clinical use. With the development of ADC drugs, there is a need for more effective small-molecule drugs with novel mechanisms of action.

Benzodiazepine derivatives, with the ability to recognize and bind to specific DNA sequences, are highly potent interstrand cross-linkers that react with guanine in the minor groove of DNA to form DNA adducts, interfering with the processing of DNA, hence their use as antitumor agents is recognized. (Rahman et al. (2009) Jour. Amer. Chem. Soc. 131(38):13756-13766; Thurston et al. (1994) Chem. Rev., 94:433-465; Bose et al. (1992) J. Am. Chem. Soc. 114:4939-4941; Gregson et al. (2004) Jour. Med.Chem. 47(5):1161-1174).

DNA minor groove alkylating agents of the 1 -(chloromethyl)-2,3-dihydro-1H-benzo[e]indole (CBI) class are potent cytotoxins (Atwell et al. (1999) J. Med. Chem., 42:3400) and have been used as effector units in multiple classes of precursor drugs designed for cancer therapy. CBI and benzodiazepine derivatives have been linked together by alkyl chains (CN105636612 A).

Imidazo[1,2-a] pyridine derivatives are potent DNA-binding units that have been used in the synthesis of derivatives of the antitumor antibiotic betacarbamycins (duocarmycins) and have shown very potent cytotoxicity (Ronald C.Elgersma et al. (2015) Mol.Pharmaceutics.12. 1813-1835).

Benzodiazepine derivatives in all prior art disclosure are extremely toxic and are toxic at very low doses, therefore, there is a strong need for an improved benzodiazepine derivatives that has lower toxicity, still be therapeutically active, and has a high therapeutic window.

### SUMMARY

The present application aims to provide a cytotoxic benzodiazepine dimer derivative with a good therapeutic window and an antibody conjugate thereof. The novel conjugate of cytotoxic benzodiazepine dimer derivatives of small molecule drugs has two functional groups, chloromethyl (CBI) and imine (PBD). Chloromethyl is a precursor drug structure that enters the body to form a ternary ring structure, which can further undergo DNA alkylation. The presence of these two functional groups enhances DNA cross-linking. The inventors unexpectedly found that such benzodiazepineADC drugs have good safety and efficient anti-tumor activity.

The present application discloses a ligand-drug conjugate as shown in Formula I, or pharmaceutically acceptable salts, deuterated derivatives, and solvates thereof:

Ab-L-D (I)

wherein:
Ab is a ligand unit, selected from antibodies, antibody fragments, targeting proteins or Fc-fusion proteins;
L is a linker unit between D and Ab;
D is a drug unit, selected from the following structures: 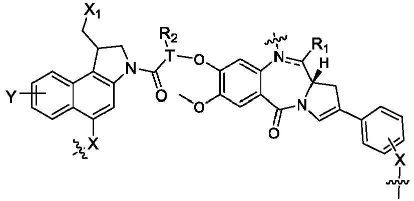 or 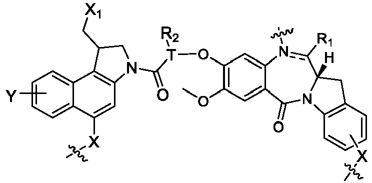
wherein:
   the wavy lines indicate sites wherein the drug unit is connected to L and only one of the three sites is connected to L;
   R₁ is H, deuterium, OH or an ether indicated by OR₃, a sulfite SO₃⁻ or OSO₃⁻, wherein R₃ is selected from the straight, branched or cyclic alkyl, alkenyl or alkynyl groups of C₁-C₁₀;
   the double line -̅ -̅ -̅ between N and C indicates a single or double bond provided that, when it is a double bond, N is not connected to L at N and R₁ is H; when it is a single bond, N is connected to L at N; R₁ is selected from OH or an ether, sulfite SO₃- or OSO₃- indicated by OR₃, wherein R₃ is selected from a straight, branched,
   or cyclic alkyl, alkenyl, or alkynyl group of C₁-C₁₀;
   R₂ is an H or alkyl group;
   T is selected from C₂-C₁₂ alkyl, Z, (C₁-C₆ alkylidene)-Z-(C₁-C₆ alkylidene), (C₁-C₆ alkylidene)-Z-(C₁-C₆ alkylidene)-Z-(C₁-C₆ alkylidene), (C₁-C₆ alkylidene)-Z-(C₁-C₆ alkylidene), or (C₁-C₆ alkylidene)-Z-(C₁-C₆ alkylidene);
   wherein:
      Z is selected from O, S, NR₄, aryl or heteroaryl; wherein R₄ is selected from H, P(O)₃H₂ or C(O)NR₅R₆; wherein R₅ and R₆ are selected from H, C₁-C₆ alkyl, one or more F-substituted C₁-C₆ alkyls, or wherein R₅ and R₆ form a five-membered or six-membered heterocyclic group;
      alkylidene, alkenylidene, aryl or heteroaryl are selected from F, OH, O(C₁-C₆ alkyl), NH₂, NHCH₃, N(CH₃)₂ or C₁-C₆ alkyl-substituted, wherein the alkyl group is substituted with one or more F;
      Y is selected from one or more H or C₁-C₄ alkyl groups;
      X is selected from-O-, -N-, -S-, -OC(O)-CR₇R₈-(CR₉R₁₀)m-O-, -OC(O)-CR₇R₈-(CR₉R₁₀)m-NH-, -OC(O)-CR₇R₈-(CR₉R₁₀)m-S-, -NHC(O)-CR₇R₈-(CR₉R₁₀)m-O-, - NHC(O)-CR₇R₈-(CR₉R₁₀)m-NH- or-NHC(O)-CR₇R₈-(CR₉R₁₀)m-S-;
      wherein:
         R₇, R₈ are independently a hydrogen atom, a deuterium atom, a halogen, an alkyl group, a deuteroalkyl group, a haloalkyl group, a cycloalkyl group, a cycloalkyl alkyl group, an alkoxyalkyl group, a heterocyclyl group, an aryl group, a substituent aryl group or a heteroaryl group, respectively; alternatively, R₇, R₈ and the carbon atoms connected thereto constitute a C₃-C₆ cycloalkyl group, a cycloalkyl alkyl group or a heterocyclyl group;
         R₉, R₁₀ are identical or different and are independently hydrogen, deuterium, halogen, alkyl, haloalkyl, deuteroalkyl, alkoxy, hydroxy, amino, cyano, nitro, hydroxyalkyl, cycloalkyl, or heterocycloalkyl, respectively; alternatively, R₉, R₁₀ and the carbon atoms connected thereto constitute a C₃-C₆ cycloalkyl, cycloalkylalkyl, or heterocycloalkyl;
         m is chosen from the integers 0-4; and
         X₁ is selected from halogen or OSO₂R₁₁, wherein R₁₁ is selected from H, C₁-C₄ hydrocarbyl, phenyl or substituted phenyl.

In one embodiment, Ab is an antibody, which is configured to form a linkage bond with the connecting unit through its heteroatom, said antibody being selected from murine antibodies, chimeric antibodies, humanized antibodies, fully human antibodies, antibody fragments, bispecific antibodies, or multispecific antibodies.

In a further combodiment, the antibody, or antigen-binding fragment thereof, is selected from, without limitation: anti-EGFRvIII antibody, anti-DLL-3 antibody, anti-PSMA antibody, anti-CD70 antibody, anti-MUC16 antibody, anti-ENPP3 antibody, anti-TDGF1 antibody, anti-ETBRantibody, anti-MSLNantibody,anti-TIM-1 antibody, anti-LRRC15 antibody, anti-LIV-1 antibody, anti-CanAg/AFP antibody, anti-cladin 18.2 antibody, anti-Mesothelin antibody, anti-HER2 (ErbB2) antibody, anti-EGFR antibody, anti-c-MET antibody, anti-SLITRK6 antibody, anti-KIT/CD117 antibody, anti-STEAP1 antibody, Anti-SLAMF7/CS1 antibody, anti-NaPi2B/SLC34A2 antibody, anti-GPNMB antibody, anti-HER3(ErbB3) antibody, anti-MUC1/CD227 antibody, anti-AXL antibody, anti-CD166 antibody, anti-B7-H3(CD276) antibody, anti-PTK7/CCK4 Antibodies, anti-PRLR antibodies, anti-EFNA4 antibodies, anti-5T4 antibodies, anti-NOTCH3 antibodies, anti-Nectin 4 antibodies, anti-TROP-2 antibodies, anti-CD142 antibodies, anti-CA6 antibodies, anti-GPR20 antibodies, anti-CD174 antibodies, anti-CD71 antibodies, anti- EphA2 antibody, anti-LYPD3 antibody, anti-FGFR2 antibody, anti-FGFR3 antibody, anti-FRα antibody, anti-CEACAMs antibody, anti-GCC antibody, anti-Integrin Av antibody, anti-CAIX antibody, anti-P-cadherin antibody, anti-GD3 antibody, anti-Cadherin 6 antibody, anti-LAMP1 antibody, anti-FLT3 antibody, anti-BCMAantibody, anti-CD79b antibody, anti-CD19 antibody, anti-CD33 antibody, anti-CD56 antibody, anti-CD74 antibody, anti-CD22 antibody, anti-CD30 antibody, anti-CD37 antibody, anti-CD47 antibody, anti-CD138 antibody, anti-One or more of CD352 antibody, anti-CD25 antibody, or anti-CD123 antibody.

The application further provides a DNA-toxic dimer compound and its conjugate or pharmaceutically acceptable salts, deuterated derivatives and solvates thereof. In one embodiment, the L is cleavable or non-cleavable.

A DNA-toxic dimer compound and its conjugates or pharmaceutically acceptable salts, deuterated derivatives, and solvates thereof, wherein the pharmaceutically acceptable salt comprises a sodium, potassium, calcium, or magnesium salt formed with an acidic functional group in the structural formula; or an acetate, trifluoroacetate, citrate, oxalate, tartrate, malate, nitrate, chloride, bromide, iodide, sulfate, bisulfate, phosphate, lactate, oleate, ascorbate, salicylate, formate, glutamate, methanesulfonate, ethanesulfonate, benzenesulfonate, or p-toluenesulfonate formed with a basic functional group in the structure.

A DNA-toxic dimer compound and its conjugate or pharmaceutically acceptable salts, deuterated derivatives and solvates thereof, prepared for use as therapeutic or prophylactic oncology drugs.

In one embodiment, the tumor is breast cancer, ovarian cancer, cervical cancer, uterine cancer, prostate cancer, renal cancer, urethral cancer, bladder cancer, liver cancer, gastric cancer, endometrial cancer, salivary gland cancer, esophageal cancer, lung cancer, colon cancer, rectal cancer, colorectal cancer, bone cancer, skin cancer, thyroid cancer, pancreatic cancer, melanoma, glioma, neuroblastoma, glioblastoma multiforme, sarcoma, lymphoma and leukemia, as well as other solid tumors or hematological tumors.

The present application further provides compounds of general formula II or III that are linked to Ab, or pharmaceutically acceptable salts or solvates thereof: 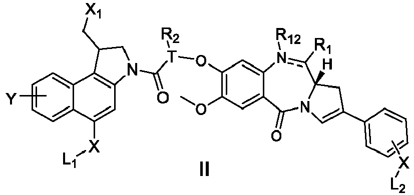 or 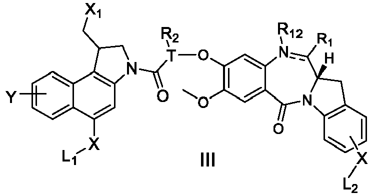 wherein:
R₁ is H, OH or an ether indicated by OR₃, a sulfite SO₃⁻ or OSO₃⁻, wherein R₃ is selected from the straight, branched or cyclic alkyl, alkenyl or alkynyl groups of C₁-C₁₀;
The double line -̅ -̅ -̅ between N and C denotes a single or double bond provided that, when it is a double bond, R₁₂ is absent and R₁ is H; when it is a single bond, R₁₂ is-C(O)O-L₃, wherein L₃ is the linking unit; and R₁ is selected from OH, an ether as denoted by OR₃, a sulphite SO₃-, or an OSO₃-, wherein R₃ is selected from a straight-chained, branched, or cyclic alkyl, alkenyl, or alkynyl group of C₁-C₁₀;
R₂ is an H or alkyl substituent;
T is selected from C₂-C₁₂ alkyl, Z, (C₁-C₆ alkylidene)-Z-(C₁-C₆ alkylidene), (C₁-C₆ alkylidene)-Z-(C₁-C₆ alkylidene)-Z-(C₁-C₆ alkylidene), (C₁-C₆ alkylidene)-Z-(C₁-C₆ alkylidene), or (C₁-C₆ alkylidene)-Z-(C₁-C₆ alkylidene);
wherein:
   Z is selected from O, S, NR₄, aryl and heteroaryl; wherein R₄ is selected from H, P(O)₃H₂, C(O)NR₅R₆, wherein R₅ and R₆ are selected from H, C₁-C₆ alkyl, one or more F-substituted C₁-C₆ alkyls or wherein R₅ and R₆ form a five-membered or six-membered heterocyclic group;
   Alkylidene, alkenylidene, arylidene and heteroarylidene are selected from F, OH, O(C₁-C₆ alkylidene), NH₂, NHCH₃, N(CH₃)₂ and C₁-C₆ alkylidene-substituted, wherein the alkylidene is substituted with one or more F;
   Y is selected from one or more H or C₁-C₄ alkyl groups;
   X selected from-O-,-N-,-S-,-OC(O)-CR₇R₈-(CR₇R₁₀)m-O-,-OC(O)-CR₇R₈-(CR₉R₁₀)m-NH-, -OC(O)-CR₇R₈-(CR₉R₈)m-S-, -NHC(O)-CR₇R₈-(CR₉R₁₀)m-O-, - NHC(O)-CR₇R₈-(CR₉R₁₀)m-NH- or-NHC(O)-CR₇R₈-(CR₉R₁₀)m-S-;
   wherein:
      R₇, R₈ are independently a hydrogen atom, a deuterium atom, a halogen, an alkyl group, a deuteroalkyl group, a haloalkyl group, a cycloalkyl group, a cycloalkyl alkyl group, an alkoxyalkyl group, a heterocyclyl group, an aryl group, a substituted aryl group or a heteroaryl group, respectively; alternatively, R₇, R₈ and the carbon atoms connected thereto constitute a C₃-C₆ cycloalkyl group, a cycloalkyl alkyl group or a heterocyclyl group;
      R₉, R₁₀ are identical or different and are independently hydrogen, deuterium, halogen, alkyl, haloalkyl, deuteroalkyl, alkoxy, hydroxy, amino, cyano, nitro, hydroxyalkyl, cycloalkyl, or heterocycloalkyl, respectively; alternatively, R₉, R₁₀ and the carbon atoms connected thereto constitute a C₃-C₆ cycloalkyl, cycloalkylalkyl, or heterocycloalkyl;
      m is chosen from the integers 0-4;
      X₁ is selected from halogen or OSO₂R₁₁, wherein R₁₁ is selected from the hydrocarbon group of H, C₁-C₄, phenyl or substituted phenyl;
      L₁, L₂ are linker units or substituents.

In one embodiment, T is selected from the alkylene groups of C₂-C₁₂.

More preferably, T is

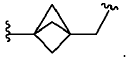

In one embodiment, X is-O-,-N- or-NHC(O)-CR₇R₈-(CR₉R₁₀)m-O-.

In one embodiment, L₃ is: 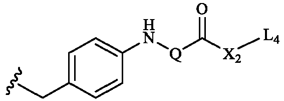 wherein: a connection to-C(O)O- occurs at the wavy line; L₄ is a linker unit, connected to the ligand unit.

In one embodiment, L₄ is non-limitatively selected from: 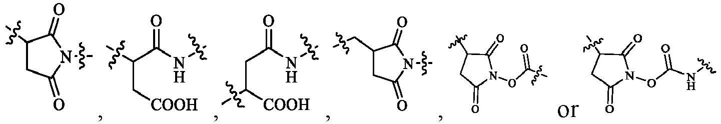 wherein: at the left wavy line, a carbon is connected to the ligand unit, and at the right wavy line, a nitrogen or ester carbonyl is connected to X₂.

In one embodiment, Q is: 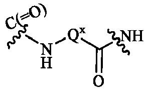 wherein Q^{x} is an amino acid residue or a peptide residue comprising an amino acid.

In one embodiment, X₂ is: 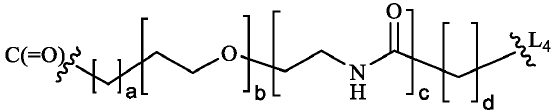 where a is selected from the integers 0-5, b is selected from the integers 0-16, c is selected from the integers 0-1, and d is selected from the integers 0-5.

In one embodiment, L₃ is selected without limitation from: 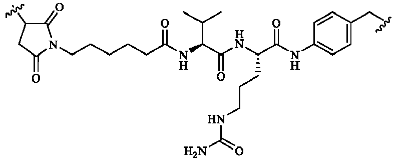 or 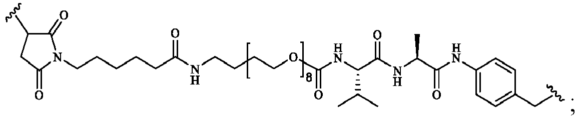 wherein: at the left wavy line, the butanediimide is connected to the ligand unit, and at the right wavy line, a connection to-C(O)O- occurs.

In one embodiment, the compounds of general Formula II or III, or pharmaceutically acceptable salts or solvates thereof, L₁ and L₂ are independently selected from:
structure A: a hydrogen atom, C(O)NR'R", wherein R' and R" are selected from H, C₁-C₆ alkyl, one or more F-substituted C₁-C₆ alkyls, respectively; or wherein R' and R" form a five-membered or six-membered heterocyclic group; and
structure B: L₄-L₅-, L₄-L₆- or L₄-L₇-L₈-L₉-, wherein L₄, L₅, L₆, L₇, L₈, and L₉ are connecting units, L₄ is connected to the ligand unit, and L₅, L₆, and L₉ are connected to X.

In one embodiment:
when there is a single bond between N and C, i.e., R₁₂ is present, L₁, L₂ are independently selected from structure A;
when there is a double bondbetween N and C i.e. R₁₂ is not present, L₁ is structure A or B, then L₂ is structure B or A.

In one embodiment, L₅ is-((CH₂)sO)r(CH₂)sX₃L₁₀- or-((CH₂)sO)r(CH₂)sX₄L₁₀-;

L₆ is-((CH₂)sO)r(CH₂)s-;

L₁₀ is-(CH₂)s- or-((CH₂)sNHC(=O)X₅X₆ C(=O)(CH₂)s-;

wherein:
X₃ is elected without limitation from the following group: 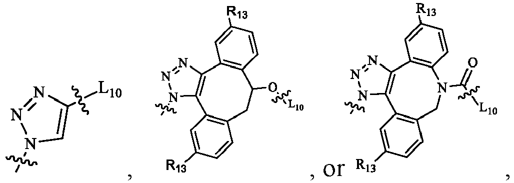
wherein R₁₃ is independently selected from a hydrogen atom, a C₁-C₆ hydrocarbon group, a halogen atom or a hydroxyl group;
X₄ is selected without limitation: 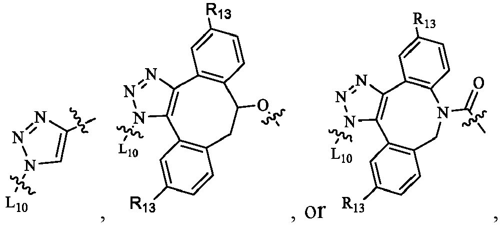
wherein R₁₃ is independently selected from a hydrogen atom, a C₁-C₆ hydrocarbon group, a halogen atom or a hydroxyl group;
X₅ is elected without limitation from the following group: 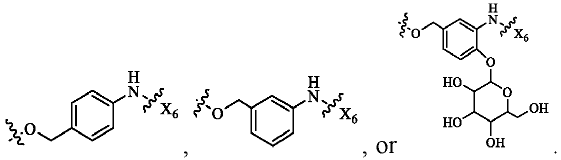
X₆ is selected from peptide residues consisting of amino acids, selected without limitation from: 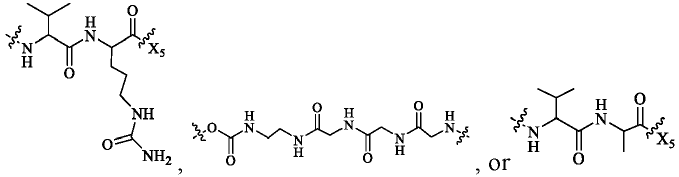
s is chosen from integers 1-10, and r is chosen from integers 1-14.

In one embodiment, L₇ is-NC(R₁₄R₁₅)C(O),-NR₁₆(CH₂)ₒC(O)-,-NR₁₆(CH₂CH₂O)ₒCH₂C(O)-,-S(CH₂)ₚC(O)-, or a chemical bond, wherein o is selected from an integer from 0-20; p is an integer from 0-20; R₁₄ is identical or different from R₁₅, and each is independently selected from a hydrogen atom, a deuterium atom, an alkyl, a substituted alkyl, a deuterioalkyl, a heteroalkyl carboxyl, amino, substituted amino; R₁₆ is selected from a hydrogen atom, a deuterium atom, a halogen, an alkyl, a substituted alkyl, a deuteroalkyl, a cycloalkylalkyl, an alkoxyalkyl, an aryl, a substituted aryl or a heteroaryl;
L₈ is selected from peptide residues derived from free amino acids;
L₉ is-NR₁₇(CR₁₈R₁₉) _{q}-,-C(O)NR₁₇-,-C(O)NR₁₇ (CH₂) _{q}-, or a chemical bond, wherein q is selected from an integer from 0 to 6; R₁₇, R₁₈ and R₁₉ are identical or different and are each independently selected from a hydrogen atom, a deuterium atom, a halogen, an alkyl group, a substituted alkyl group, a deuterated alkyl group, a cycloalkyl group, a cycloalkyl alkyl group, an alkoxyalkyl group, a heterocyclic group, an aryl group, a substituted aryl group, or a heteroaryl group.

In one embodiment, L₁, L₂ are independently selected from, without limitation, structure B from the following structures: 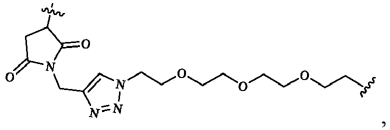 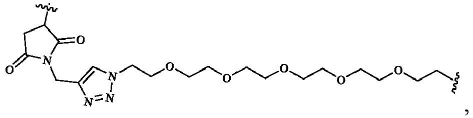 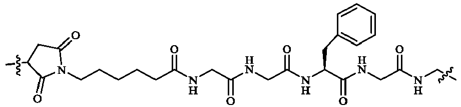 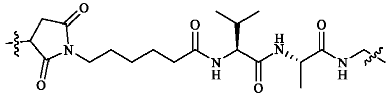 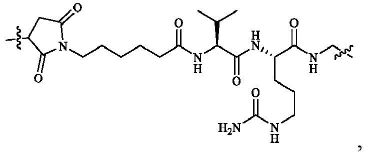 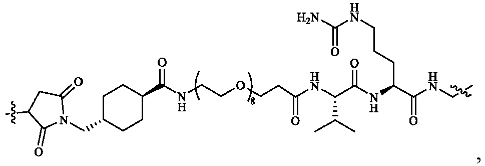 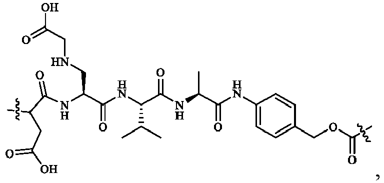 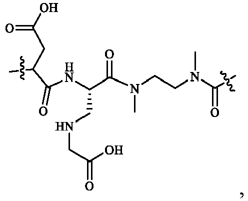 or 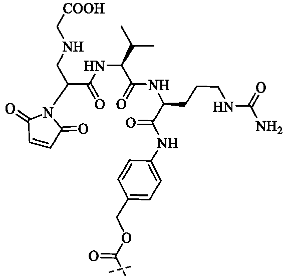 The

In a further embodiment, the compound of general formula II or III, or a pharmaceutically acceptable salt or solvate thereof, is selected, without limitation, from the following structures: 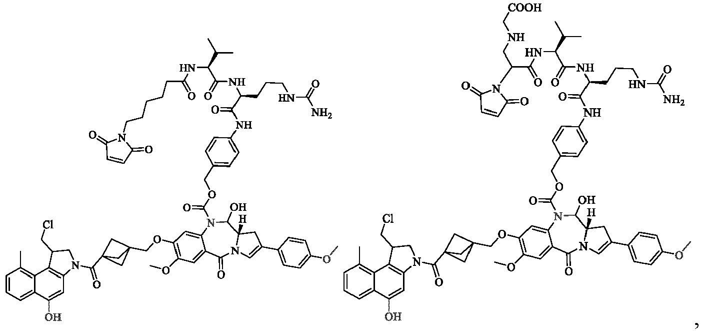 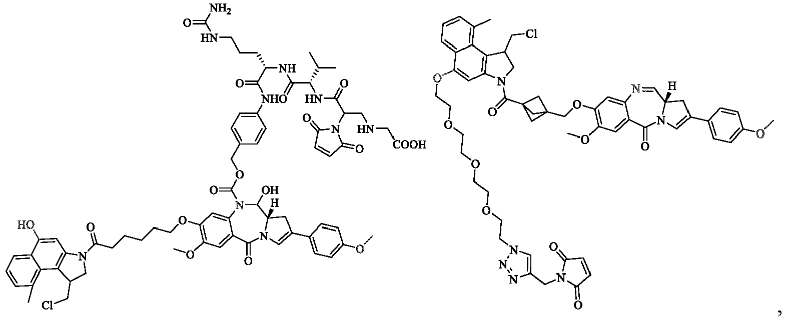 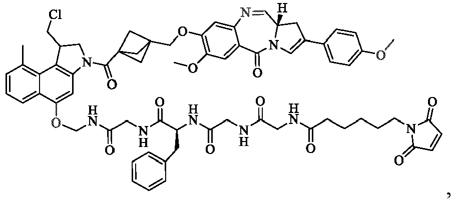 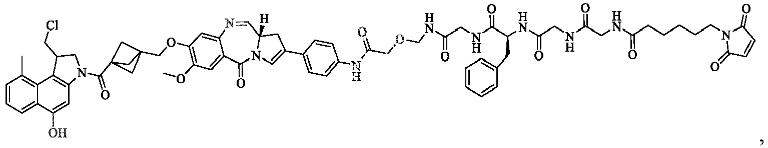 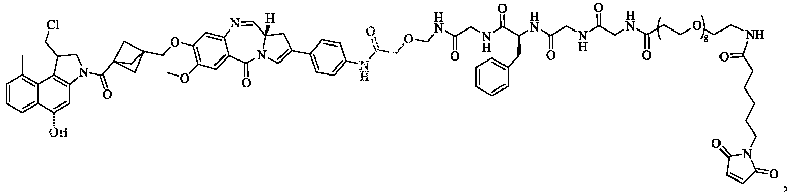 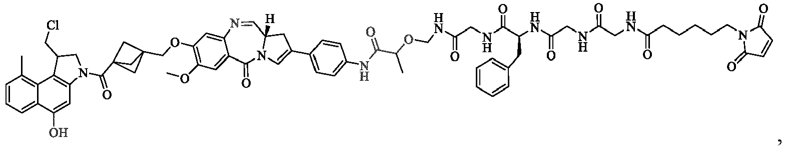 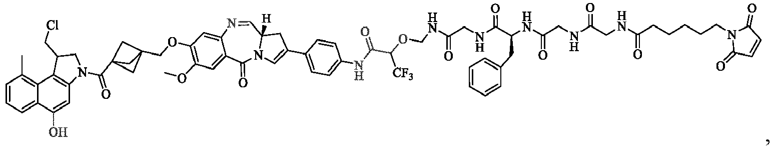 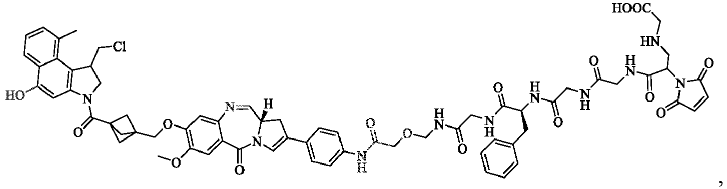 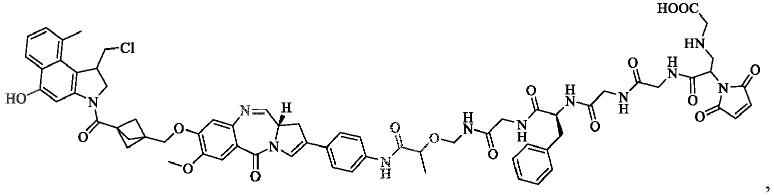 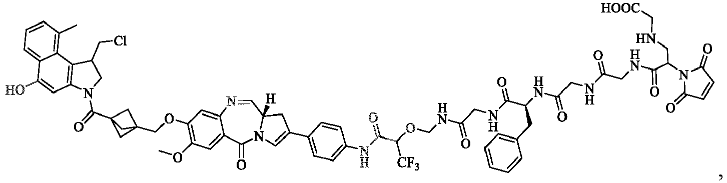 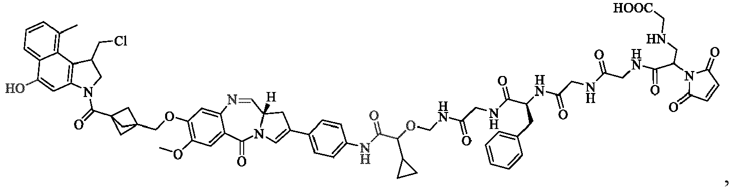 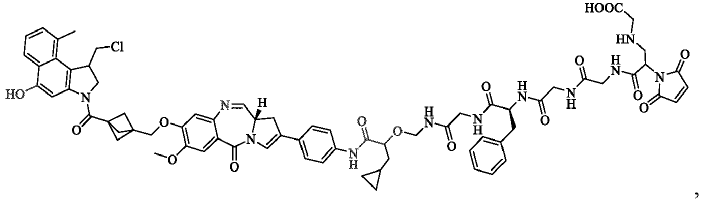 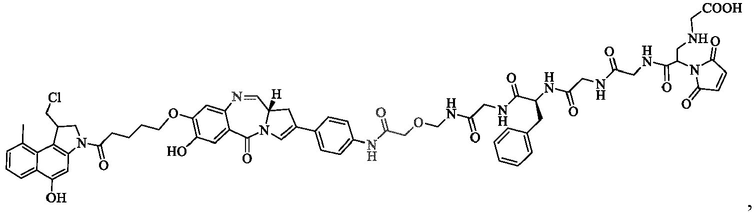 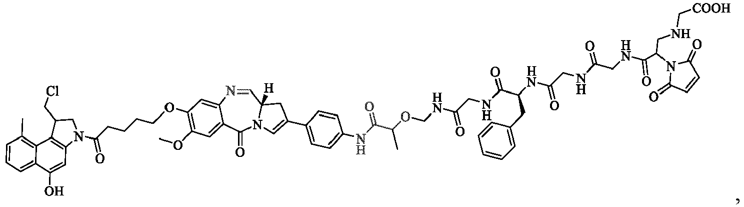 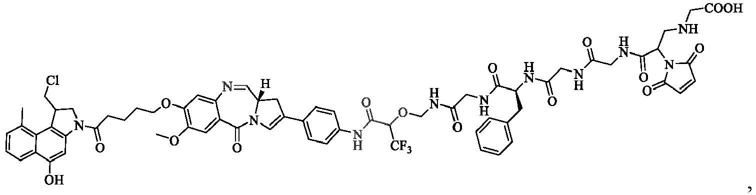 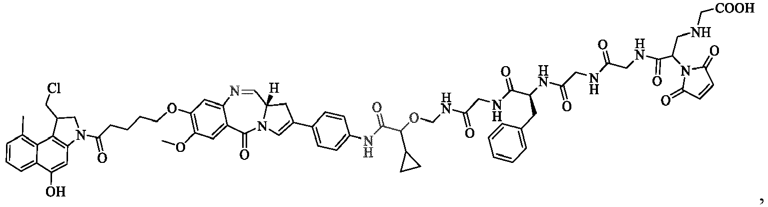 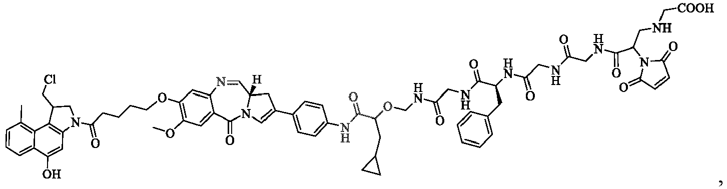 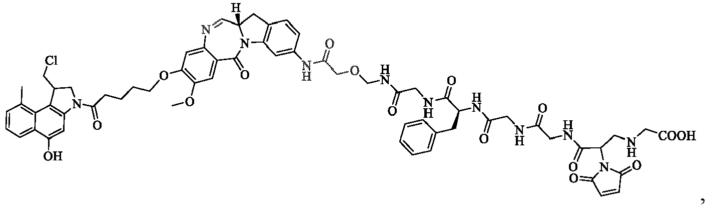 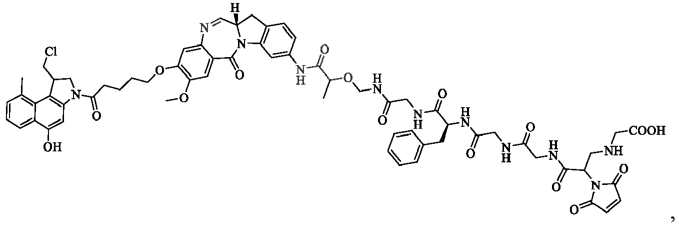 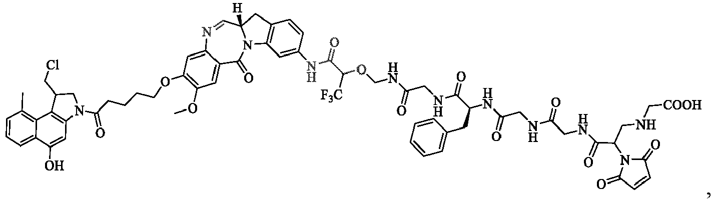 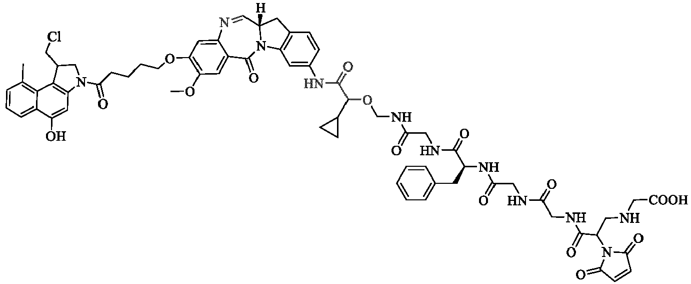 or

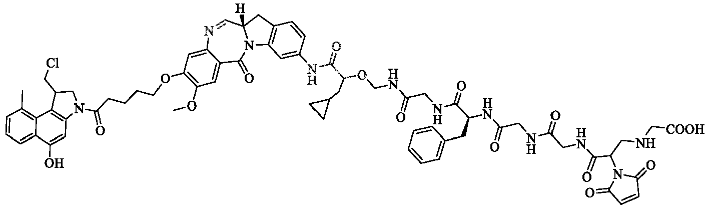

In one embodiment, the antibody-drug conjugate, or pharmaceutically acceptable salts, deuterated derivative, and solvates thereof, are selected without limitation from the following structures: 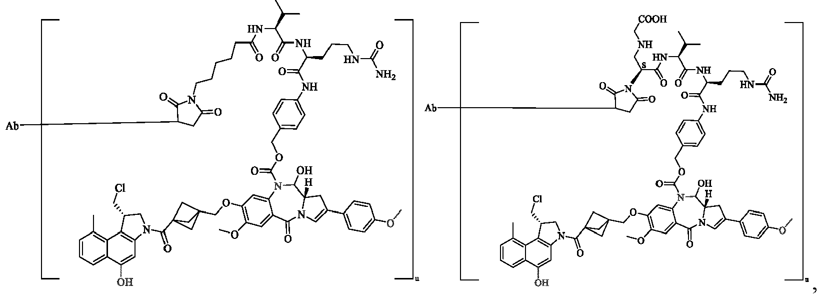 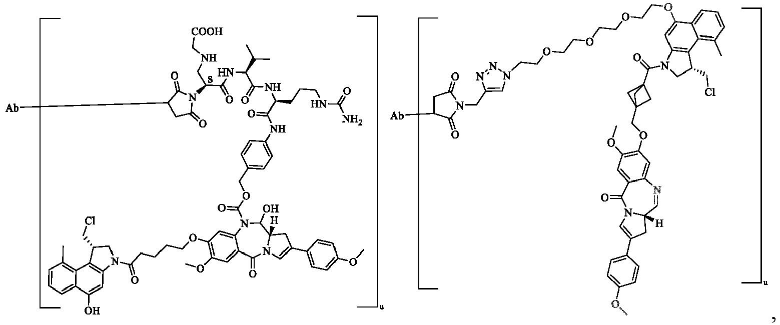 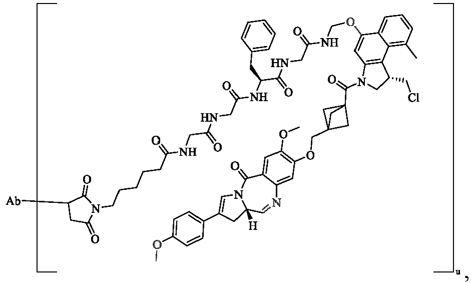 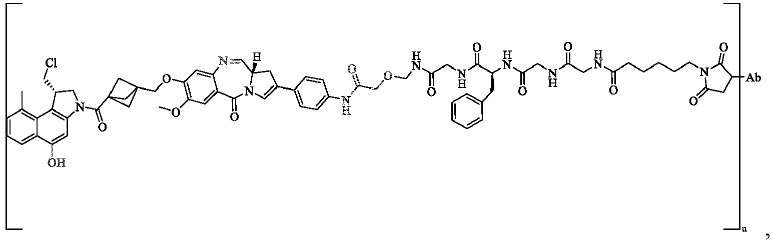 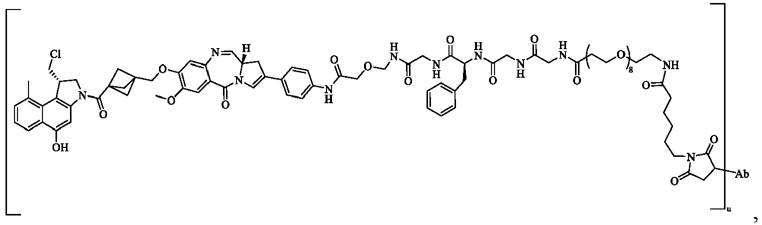 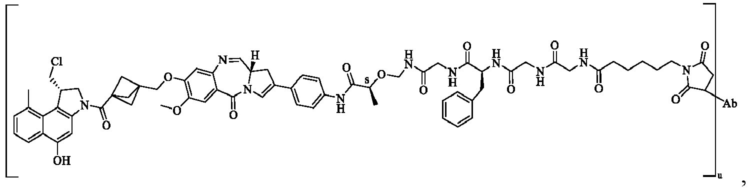 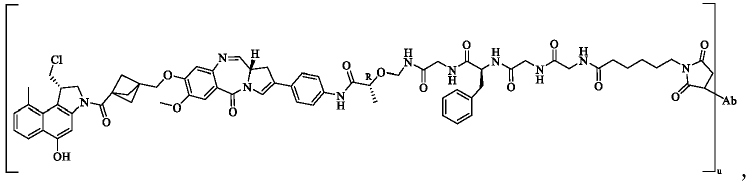 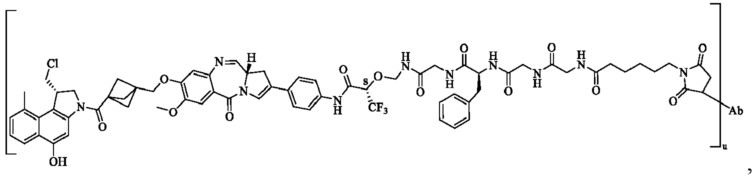 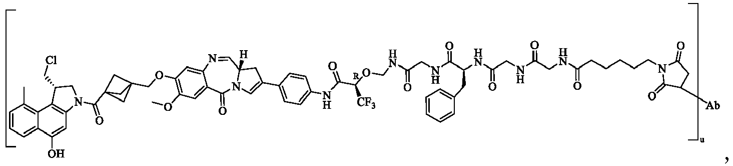 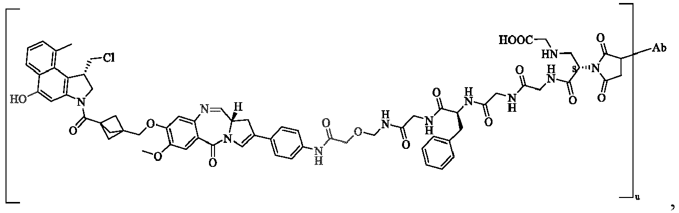 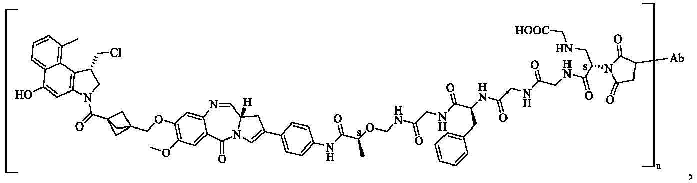 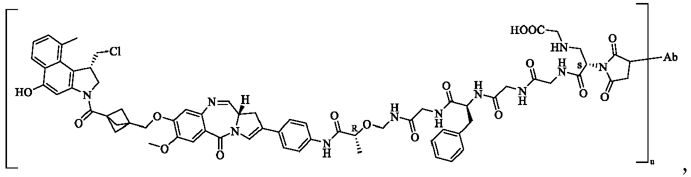 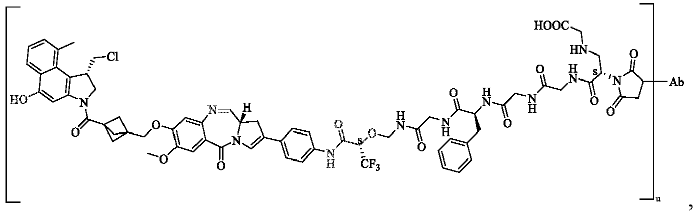 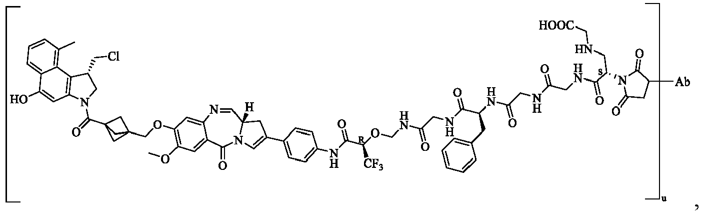 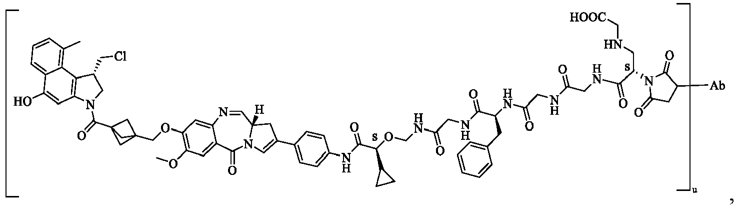 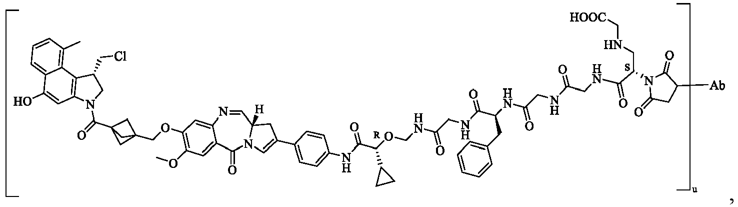 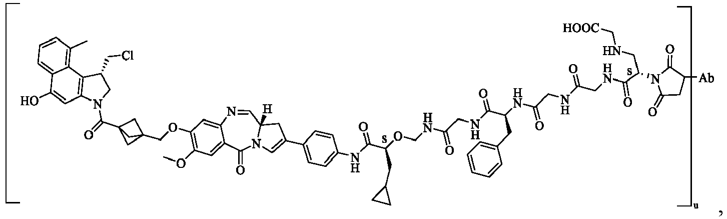 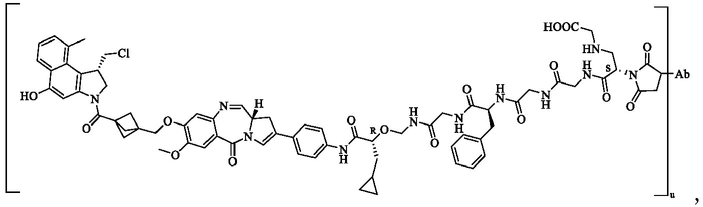 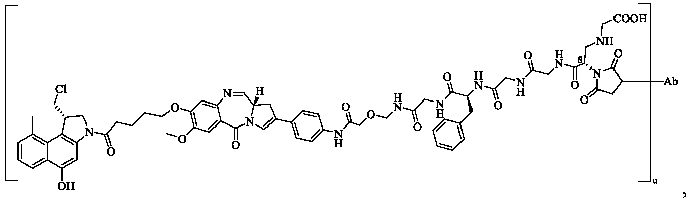 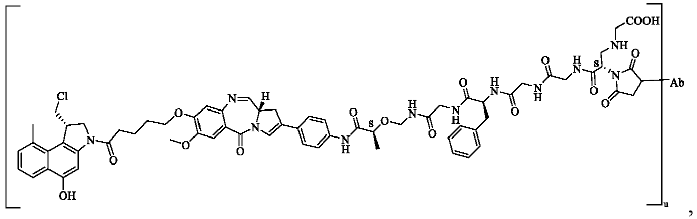 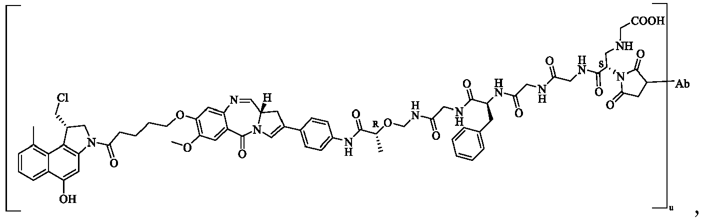 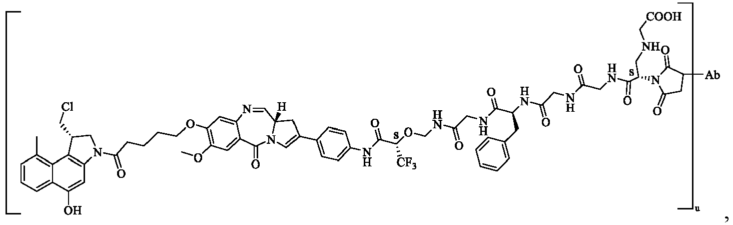 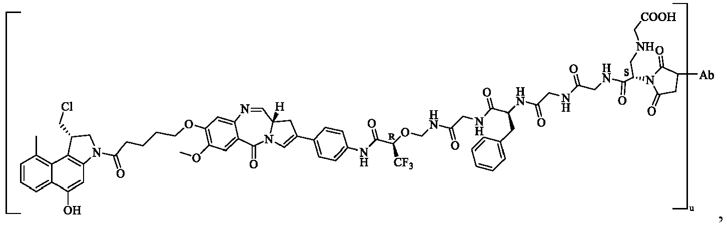 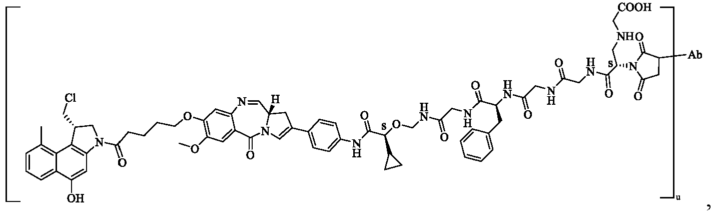 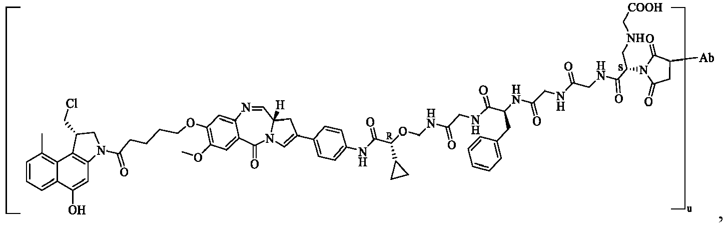 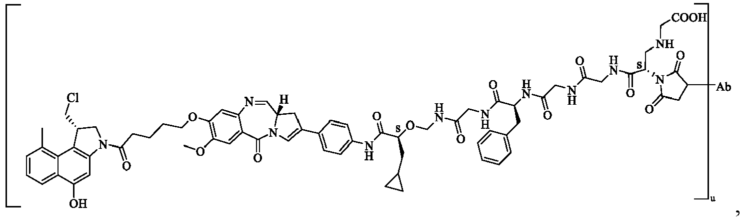 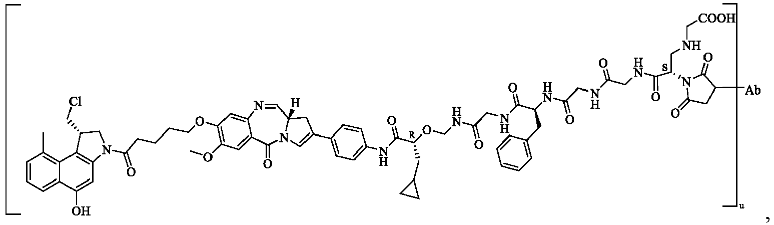 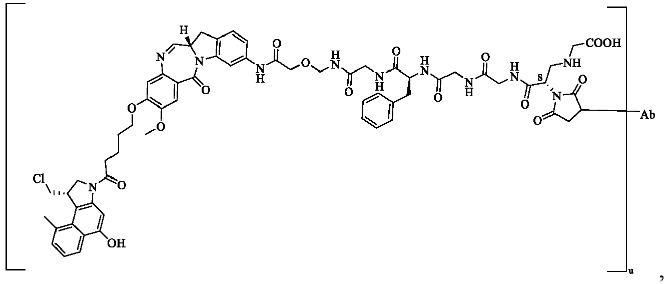 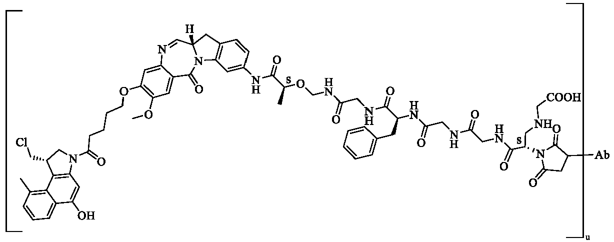 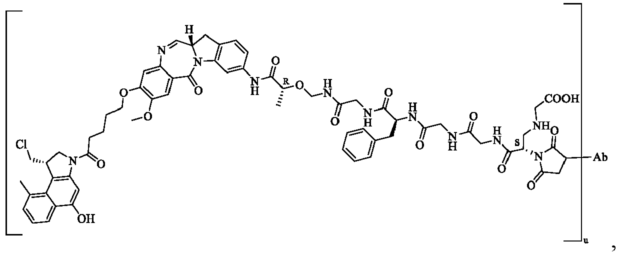 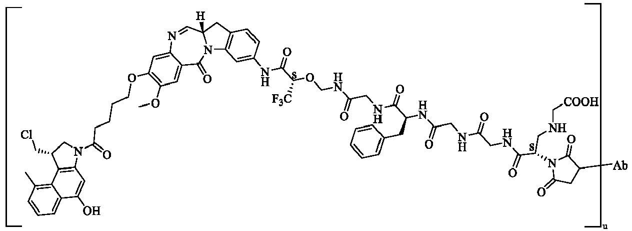 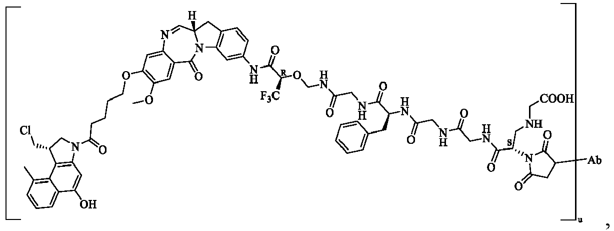 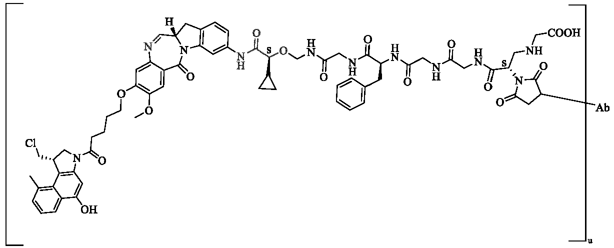 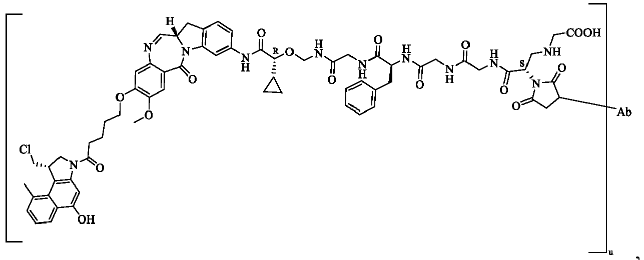 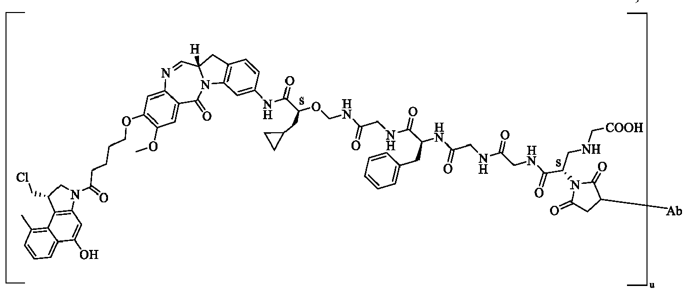 or 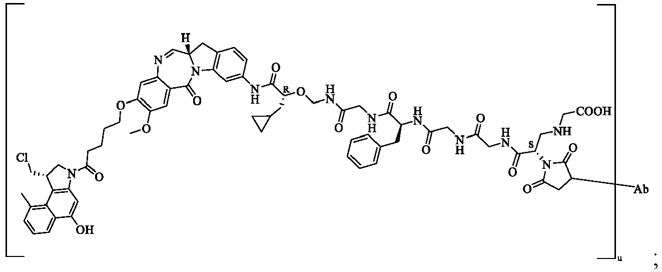 where u is selected from integers 1-10.

### DETAILED DESCRIPTION

### Abbreviations and definitions

Unless otherwise indicated, the following terms and phrases, as used herein, are intended to have the meanings set forth below. When a trade name is used herein, unless otherwise indicated in the context, the trade name includes the product formulation, generic drug and active ingredient of said trade name product.

Unless stated to the contrary, terms used in the claims and specification herein have the meanings set forth below.

The term "ligand" refers to a macromolecular compound that recognizes and binds to an antigen or receptor associated with a target cell. The ligand serves to present a drug to a target cell population bound to a ligand, which includes, but is not limited to, a protein-like hormone, a lectin, a growth factor, an antibody, or other molecule that binds to the cell. In the embodiments of the application, the ligand is denoted as Ab, and the ligand may form a linkage bond with a linking unit (i.e., a connecting unit) via a heteroatom on the ligand, preferably an antibody or an antigen-binding fragment thereof, said antibody being selectedfrom chimeric, humanized, fully human, or murine antibodies; preferably monoclonal antibodies.

A ligand unit is a targeting agentthatbinds specifically to a target component. Said ligand is capable of binding specifically to a cellular fraction or to a cellular component or to other target molecules of interest. The target portion or target is typically on the surface of the cell. In some aspects, the ligand unit serves to deliver the drug unit to a specific target cell population with which the ligand unit interacts. Ligands include but are not limited to, proteins, polypeptides and peptides, and non-proteins such as sugars. Suitable ligand units include, for example, antibodies, such as full-length (intact) antibodies and antigen-binding fragments thereof. In embodiments where the ligand unit is a non-antibody targeting reagent, it may be a peptide or polypeptide, or a non-protein molecule. Examples of such targeting reagents include interferons, lymphokines, hormones, growth and colony stimulating factors, vitamins, nutrient transporter molecules, or any other cell-binding molecule or substance. In some embodiments, the linker is covalently attached to the sulfur atom of the ligand. In some embodiments, the sulfur atom is a sulfur atom of a cysteine residue that forms an interchain disulfide bond of the antibody. In another embodiment, the sulfur atom is a sulfur atom of a cysteine residue that has been imported into the ligand unit, which forms an interchain disulfide bond of the antibody. In still another embodiment, the sulfur atom is a sulfur atom that has been introduced into a cysteine residue of the ligand unit (e.g., by targeted mutagenesis or chemical reaction). In yet another embodiment, the linker-bound sulfur atom is selected from a cysteine residue that forms the interchain disulfide bond of the antibody or a frontal cysteine residue that has been introduced into the ligand unit (e.g., by targeted mutagenesis or chemical reaction). In some embodiments, the EU index numbering system in accordance with Kabat {[Kabat E.A et al, (1991)] Sequences of proteins of Immunological Interest, Fifth Edition, NIH Publication 91-3242} is used.

As used herein, "antibody" or "antibody unit" includes, to the extent thereof, any part of an antibody structure. This unit may bind, reactively associate, or complex with a receptor, antigen, or other receptor unit possessed by the target cell population. The antibody may be any protein or protein-like molecule that binds, complexes, or reacts with a portion of the cell population to be treated or biologically modified. The antibodies comprising the antibody-drug conjugate of the present application maintain their original antigen-binding capacity in the wild type. Thus, the antibodies of the present application are capable of binding exclusively to antigens. Antigens involved include, for example, tumor-associated antigens (TAA), cell surface receptor proteins and other cell surface molecules, cell survival regulators, cell proliferation regulators, molecules associated with tissue growth and differentiation (such as are known or foreseen to be functional), lymphokines, cytokines, molecules involved in the regulation of cellular cycling, molecules involved in angiogenic molecules (if known or predicted to be functional). Tumor-associated factors may be cluster differentiation factors (e.g., CD proteins).

Antibodies applied in antibody drug conjugate include, but are not limited to, antibodies directed against cell surface receptors and tumor-associated antigens. Such tumor-associated antigens are well known in the industry and can be prepared by methods and information for antibody preparation well known in the industry. In order to develop effective cellular level targets that can be used for cancer diagnosis and treatment, researchers seek to find transmembrane or other tumor-associated peptides. These targets can be specifically expressed on the surface of one or more cancer cells with little or no expression on the surface of one or more non-cancer cells. Typically, such tumor-associated polypeptides are more overexpressed on the surface of cancer cells relative to the surface of non-cancer cells. Identification of such tumor-associated factors can greatly enhance the specific targeting properties of antibody-based cancer therapies. For convenience, information related to antigens known to the industry is labeled below, including name, other names, and genebank accession number. Nucleic acid and protein sequences corresponding to the tumor-associated antigens canbe found in publicly available databases, such as Genbank.The tumor-associated antigens targeted by the antibody include all amino acid sequence variants and isoforms having at least 70%, 80%, 85%, 90%, or 95% homology to the sequences identified in the references, or possessing biological properties and characteristics that are identical to the sequences of tumor-associated antigens in the cited literature. biological properties and characteristics of the tumor-associated antigen.

The term "inhibition" or "inhibition" means that a detectable amount is reduced or completely prevented.

The term "cancer" refers to a physiological condition or disease characterized by dysregulated cell growth. "Tumor" includes cancer cells.

The term "autoimmune disease" refers to diseases or disorders that originate in tissues or proteins that target an individual's own body.

The term "drug" refers to cytotoxic drugs, expressed as "d", which are chemical molecules that have a strong ability to disrupt the normal growth of tumor cells. Cytotoxic drugs can, in principle, kill tumor cells in sufficiently high concentrations, but due to a lack of specificity, they can kill tumor cells while causing apoptosis in normal cells, leading to serious side effects. The term includes toxins, such as small molecule toxins or enzymatically active toxins of bacterial, fungal, plant or animal origin, radioisotopes (e.g., radioisotopes of At²¹¹, I¹³¹, I¹²⁵, Y⁹⁰, Re¹⁸⁶, Re¹⁸⁸, Sm¹⁵³, Bi²¹², P³² and Lu¹⁷⁶), toxic drugs, chemotherapeutic drugs, antibiotics and nucleolytic enzymes, preferably toxic drugs.

The term "linker" or "linker fragment" or "linker unit" refers to a fragment or bond of a chemical structure that is attached to a ligand at one end and to the drug at the other end, or may be attached to other connectors and then to the drug.

Junctions, including extensions, spacers and amino acid units, can be synthesized by methods known in the art, such as those described in US2005 -0238649A1. The junctions can be "cleavable junctions" that facilitate the release of the drug in the cell. For example, acid-unstable junctions (e.g., hydrazone), protease-sensitive (e.g., peptidase-sensitive) junctions, photo-unstable junctions, dimethyl junctions, or disulfide-containing junctions can be used (Chari et al. Cancer Research 52:127-131, 1992); U.S. Patent No. 5, 208, 020.

According to the mechanism of intracellular drug release, as used herein, "linkers" or "linkers of antibody-drug Conjugate" can be categorized into two types: unbreakable linkers and breakable linkers. For antibody-drug Conjugate containing an unbreakable linker, the mechanism of drug release is as follows: after the coupler binds to the antigen and is endocytosed by the cell, the antibody is enzymatically cleaved in the lysosome, releasing an active molecule consisting of the small drug molecule, the linker, and the antibody amino acid residues. Theresulting changein the structure of the drug molecule does not diminish its cytotoxicity, but because the active molecule is electrically charged (amino acid residues), it cannot penetrate into neighboring cells. Therefore, such active drugs do not kill neighboring tumor cells that do not express the targeted antigen (antigen-negative cells) (bystander effect) (Ducry et al., 2010, Bioconjugate Chem. 21: 5-13).

The term "antibody-drug conjugate" refers to the attachment of an antibody to a biologically active drug by means of a stable linker unit. In the context of the present application "ligand-drug conjugate", preferably antibody-drug conjugate (ADC), refers to the attachment of a monoclonal antibody or antibody fragment to a biologically active toxic drug by means of a stabilized linker unit.

The three-letter codes and single-letter codes for amino acids used in this disclosure are as described in J. boil.Cem. 1968, 243, 3558.

The term "alkyl" refers to a saturated aliphatic hydrocarbon group, which is a straight or branched chain group comprising 1 to 20 carbon atoms, preferably an alkyl group comprising 1 to 12 carbon atoms, more preferably an alkyl group comprising 1 to 10 carbon atoms, and mostpreferably an alkyl group comprising 1 to 6 carbon atoms. Non-limiting examples include methyl, ethyl, n-propyl, isopropyl, n-butyl, isobutyl, tert-butyl, sec-butyl, n-pentyl, 1, 1-dimethylpropyl, 1, 2-dimethylpropyl, 2, 2-dimethylpropyl, 1 ethylpropyl, 2-methylbutyl, 3-methylbutyl, n-hexyl, 1 ethyl-2-methylpropyl, 1, 1, 2-trimethylpropyl, 1, 1-dimethylbutyl 1, 2-dimethylbutyl, 2, 2-dimethylbutyl, 1, 3-dimethylbutyl, 2-ethylbutyl, 2-methylpentyl, 3-methylpentyl, 4-methylpentyl, 2, 3-dimethylbutyl, n-heptyl, 2-methylhexyl, 3-methylhexyl, 4-methylhexyl, 5-methylhexyl, 2, 3-dimethylpentyl, 2, 4-dimethylpentyl, 2, 2-dimethylpentyl 2, 3-dimethylpentyl, 3, 3-dimethylpentyl, 2-ethylpentyl, 3-ethylpentyl, n-octyl, 2, 3-dimethylhexyl, 2, 4-dimethylhexyl, 2, 5-dimethylhexyl, 2, 2-dimethylhexyl, 3, 3-dimethylhexyl, 4, 4-dimethylhexyl, 2-ethylhexyl, 3-ethylhexyl, 4-ethylhexyl, 2-methyl-2-ethylpentyl, 2-methyl-3-ethylpentyl, n-nonyl, n-ethylpentyl, 2, 2-dimethylpentyl, n-nonyl, 2, 2-dimethylpentyl, n-nonyl, 2, 2-dimethylpentyl, 2, 2-methylpentyl, 2, 2-methylpentyl ethylpentyl, n-nonyl, 2-methyl-2-ethylhexyl, 2-methyl-3-ethylhexyl, 2, 2-diethylpentyl, n-decyl, 3, 3-diethylhexyl, 2, 2-diethylhexyl, and their various branched isomers, and the like. More preferred are lower alkyl groups containing from 1 to 6 carbon atoms, and non-limiting embodiments include methyl, ethyl, n-propyl, isopropyl, n-butyl, isobutyl, tert-butyl, sec-butyl, n-pentyl, 1, 1-dimethylpropyl, 1, 2-dimethylpropyl, 2, 2-dimethylpropyl, 1-ethylpropyl, 2-methylbutyl, 3-methylbutyl, hexyl, 1-ethyl-2-methylpropyl, 1, 1, 2-trimethylpropyl, 1, 1-dimethylbutyl, 1, 2-dimethylbutyl, 2, 2-dimethylbutyl, 1, 3-dimethylbutyl, 2-ethylbutyl, 2-methylpentyl, 3-methylpentyl, 4-methylpentyl, 2, 3-dimethylbutyl, and the like. The alkyl group may be substituted or non-substituted, and when substituted, the substituent group may be substituted at any available point of attachment, said substituent group preferably being one or more of the following groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkane thio, heterocycloalkylthio, and oxo group.

The term "substituted alkyl" means that the hydrogenin the alkyl group is replaced by a substituent group which, unless otherwise indicated in the text, may be a variety of groups selected from the following group: - halogen,-OR',-NR'R' ', -SR', -SiR'R"R‴,-OC(O)R', -C(O)R', -CO₂R', -CONR'R", -OC(O)NR'R‴, -NR"C(O)R", -NR'-C(O)NR"R‴, -NR"C(O)₂R", -NH-C(NH₂)=NH, -NR'C(NH₂)=NH, -NH-C(NH₂)NR', -S(O)R', -S(O)₂R', -S(O)₂NR'R", -NR'S(O)₂R", -CN and -NO₂, the number of substituents is from 0 to (2m'+1), where m' is the total number of carbon atoms in the group. R', R" and R‴ each independently refer to hydrogen, unsubstituted C₁₋₈ alkyl, unsubstituted aryl, aryl substituted by 1-3 halogens, unsubstituted C₁₋₈ alkyl, C₁₋₈ alkoxy or C₁₋₈ thioalkoxy, or unsubstituted aryl-C₁₋₄ alkyl. When attached to the same nitrogen atom, R' and R", along with the nitrogen atom, may form a 3-, 4-, 5-, 6- or 7-membered ring. For example,-NR'R" includes 1-pyrrolidinyl and 4-morpholinyl.

The term "heteroalkyl" means an alkyl group containing one or more heteroatoms selected from N, O or S, wherein alkyl is as defined above.

The term "alkylene" means a saturated straight or branched aliphatic hydrocarbon group having two residues derived from the removal of two hydrogen atoms from the same carbon atom or from two different carbon atoms of the parent alkane, which is a straight or branched group comprising from 1 to 20 carbon atoms, preferably from 1 to 12 carbon atoms, more preferably from 1 to 6 carbon atoms. Preferably alkylene groups containing from 1 to 12 carbon atoms, more preferably from 1 to 6 carbon atoms. Non-limiting examples of alkylene groups include, but are not limited to, methylene (-CH₂-, 1, 1-ethylidene (-CH(CH₃)-), 1, 2-ethylidene (-CH₂CH₂)-, 1, 1-propylidene (-CH(CH₂CH₃)-), 1, 2-propylidene (-CH₂CH(CH₃)-), 1, 3-propylidene (-CH₂CH₂CH₂-), 1, 4-butylene (-CH₂CH₂CH₂CH₂-) and 1, 5-butylidene (-CH₂CH₂CH₂CH₂CH₂-), among others. The alkylene group may be substituted or non-substituted, and when substituted, the substituent may be substituted atany available point of attachment, said substituent preferably being independently and optionally selected from alkyl, alkenyl, alkynyl, alkoxy, alkylsulfanyl, alkylamino, halo, mercapto, hydroxyl, nitro, cyano, cycloalkyl, heterocyclo, aryl, heteroaryl, cycloalkoxy, heterocycloalkoxy, cycloalkylthio, heterocycloalkylthio, and oxo.

The term "alkoxy" refers to-O-(alkyl) and-O-(cycloalkyl), wherein alkyl or cycloalkyl is defined above. Non-limiting examples of alkoxy include: methoxy, ethoxy, propoxy, butoxy, cyclopropoxy, cyclobutoxy, cyclopentyloxy, cyclohexyloxy. The alkoxy group may be optionally substituted or non-substituted, and when substituted, the substituent is preferably one or more of the following groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio.

The term "cycloalkyl" refers to a saturated or partially unsaturated monocyclic or polycyclic cyclic hydrocarbon substituent, wherein the cycloalkyl ring comprises 3 to 20 carbon atoms, preferably 3 to 12 carbon atoms, more preferably 3 to 10 carbon atoms, and most preferably 3 to 8 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, cycloheptenyl, cycloheptatrienyl, cyclooctyl, and the like; multicyclic cycloalkyl groups include cycloalkyl groups of spirocyclic, thickened and bridged rings.

The term "heterocyclyl" means a saturated or partially unsaturated monocyclic or polycyclic cyclic hydrocarbon substituent comprising from 3 to 20 ring atoms, wherein one or more of the ring atoms is a heteroatom selected from the group consisting of nitrogen, oxygen, or S(O)ₘ (wherein ₘ is an integer from 0 to 2), but does not include a ring portion of-O-O-, -O-S-, or-S-S-, and the remaining ring atoms are carbon. atoms are carbon. Preferably, it contains 3 to 12 ring atoms, 1 to 4 of which are heteroatoms; more preferably the cycloalkyl ring contains 3 to 10 ring atoms. Non -limiting examples of monocyclic heterocyclic groups include pyrrolidinyl, piperidinyl, piperazinyl, morpholinyl, thiomorpholinyl, homopiperazinyl, and the like. Polycyclic heterocyclic groups include spiro, thick and bridged heterocyclic groups.

The term "cycloalkylalkyl" means that the alkyl group is substituted with one or more cycloalkyl groups, preferably with a cycloalkyl group, wherein alkyl is as defined above and wherein cycloalkyl is as defined above.

The term "alkyl halide" means an alkyl group substituted with one or more halogens, wherein the alkyl group is as defined above.

The term "deuteroalkyl" means an alkyl group substituted with one or more deuterium atoms, wherein the alkyl group is as defined above.

The term "hydroxyl" refers to the-OH group.

The term "halogen" means fluorine, chlorine, bromine or iodine.

The term "amino" means -NH₂. The term "nitro" means-NO₂.

The term "amide group" means-C(O)N(alkyl) or (cycloalkyl), wherein alkyl, cycloalkyl is as defined above.

The term "carboxylate group" means-C(O)O(alkyl) or (cycloalkyl), wherein alkyl, cycloalkyl is as defined above.

The present application also includes various deuterated forms of Formula I. Each of the available hydrogen atoms attached to the carbon atom can be independently replaced by a deuterium atom. Those skilled in the art can refer to the relevant literature to synthesize deuterated forms of Formula I. Commercially available deuterated starting materials can be used in the preparation of deuterated forms of Formula I, or they can be synthesized by conventional techniques using deuterium reagents, non-limiting examples of deuterium reagents include: deuteroborane, tetrahydrofuran solution of trideuteroborane, lithium aluminum deuterohydride, deuterated ethyl iodide and deuterated methyl iodide, among others.

The term "antibody" refers to immunoglobulins, which are tetrapeptide chains consisting of two identical heavy chains and two identical light chains linked by interchain disulfide bonds. Immunoglobulins differ in the composition and order of amino acids in the constant region of the heavy chain, and therefore differ in their antigenicity. Accordingly, immunoglobulins can be categorized into five classes, or isoforms of immunoglobulins, namely IgM, IgD, IgG, IgAand IgE, with corresponding heavy chains of µ-chain, δ-chain, γ-chain, α-chain and ε-chain, respectively. The same class of Ig can be further divided into different subclasses according to the differences in the amino acid composition of its hinge region and the number and position of disulfide bonds of the heavy chain, such as IgG can be divided into IgG1, IgG2, IgG3, IgG4. The light chain is divided into κ-chain or λ-chain through the differences in the constant region. Each of the five classes of Ig may have either a *κ* chain or a A chain. The antibodies described in the present application are preferably specific antibodies against cell surface antigens on target cells, and non-limiting examples are the following antibodies: one or a few anti-EGFRvIII antibody, anti-DLL-3 antibody, anti-PSMA antibody, anti-CD70 antibody, anti-MUC16 antibody, anti-ENPP3 antibody, anti-TDGF1 antibody, anti-ETBRantibody, anti-MSLN antibody, anti-TIM-1 antibody, anti-LRRC15 antibody, anti-LIV-1 antibody, anti-CanAg/AFP antibody, anti-cladin 18.2 antibody, anti-Mesothelin antibody, anti-HER2 (ErbB2) antibody, anti-EGFR antibody, anti-c-MET antibody, anti-SLITRK6 antibody, anti-KIT/CD117 antibody, anti-STEAP1 antibody, Anti-SLAMF7/CS1 antibody, anti-NaPi2B/SLC34A2 antibody, anti-GPNMB antibody, anti-HER3(ErbB3) antibody, anti-MUC1/CD227 antibody, anti-AXL antibody, anti-CD166 antibody, anti-B7-H3(CD276) antibody, anti-PTK7/CCK4 Antibodies, anti-PRLR antibodies, anti-EFNA4 antibodies, anti-5T4 antibodies, anti-NOTCH3 antibodies, anti-Nectin 4 antibodies, anti-TROP-2 antibodies, anti-CD142 antibodies, anti-CA6 antibodies, anti-GPR20 antibodies, anti-CD174 antibodies, anti-CD71 antibodies, anti- EphA2 antibody, anti-LYPD3 antibody, anti-FGFR2 antibody, anti-FGFR3 antibody, anti-FRα antibody, anti-CEACAMs antibody, anti-GCC antibody, anti-Integrin Av antibody, anti-CAIX antibody, anti-P-cadherin antibody, anti-GD3 antibody, anti-Cadherin 6 antibody, anti-LAMP1 antibody, anti-FLT3 antibody, anti-BCMAantibody, anti-CD79b antibody, anti-CD19 antibody, anti-CD33 antibody, anti-CD56 antibody, anti-CD74 antibody, anti-CD22 antibody, anti-CD30 antibody, anti-CD37 antibody, anti-CD138 antibody, anti- One or more ofCD352 antibody, anti-CD25 antibody, or anti-CD123 antibody.

The term "solvate" or "solvent compound" refers to the ligand-drug conjugate of the present application forming a pharmaceutically acceptable solvate with one or more solvent molecules. Non-limiting examples of solvent molecules include water, ethanol, and acetonitrile, isopropyl alcohol, DMSO, and ethyl acetate.

The term "drug load" refers to the average amount of cytotoxic drug loaded on each antibody in formula I. It can also be expressed as a ratio of the amount of the drug to the amount of the antibody, and the drug load can be in the range of 0-12, preferably 1-10 cytotoxic drugs (D) attached to each antibody (Ab). In embodiments of the application, the drug load is denoted as n, which exemplarily may be the mean value of 1, 2, 3, 4, 5, 6, 7, 8, 9, 10. The average amount of drug per ADC molecule after conjugating reaction can be identified by conventional methods such as UV/visible spectroscopy, mass spectrometry, ELISA tests and HPLCCHaracterization.

In one embodiment of the present application, the cytotoxic drug is coupled to the open cysteine sulfhydryl-SH and/or the sulfhydryl-SH of the fixed-point mutated cysteine residue between the antibody chains by a linker unit, and generally the number of drug molecules that can be coupled to the antibody in the conjugating reaction will be less than or equal to a theoretical maximum.

The loading of ligand cytotoxic drug Conjugate can be controlled by non-limiting methods including:
(1) Controlling the molar ratio of the linking reagent to the monoclonal antibody.
(2) Control of reaction time and temperature.
(3) Selection of different reaction reagents.

Conventional preparations of pharmaceutical compositions are described in the Chinese Pharmacopoeia.

The term "pharmaceutically acceptable salt" or "pharmaceutically usable salt" means a salt of a ligand-drug conjugate of the present application, or a salt of a compound described in the present application, which is safe and efficacious when used in a mammal and is biologically active as desired. The ligand-drug conjugate of the present application contains at least one carboxyl group so that they can form salts with bases, and non-limiting examples of pharmaceutically acceptable salts include, for example, sodium, potassium, calcium or magnesium salts.

The term "pharmaceutically acceptable salt" or "pharmaceutically usable salt" means a salt of an antibody-drug conjugates of the present application, or a salt of a compound described herein, which is safe and efficacious when used in a mammal and is biologically active as desired. The ligand-drug conjugate of the present application contain at least one amino group and can therefore form salts with acids, non-limiting examples of pharmaceutically acceptable salts include: hydrochloride, hydrobromide, hydriodate, sulfate, bisulfate, citrate, acetate, succinate, ascorbate, oxalate, nitrate, pyrite, hydrophosphate, dihydrophosphate, salicylate, citric acid hydrochloride, tartaric acid salt, maleate, fumarate, formate, benzoate, methanesulfonate, ethanesulfonate, benzenesulfonate, p-toluenesulfonate.

"Acidic amino acid" refers to amino acids with an isoelectric point of less than 7. Acidic amino acid molecules tend to have one or more acidic groups, such as carboxyl groups, which can be effectively ionized into negative ionic forms in the structure to increase hydrophilicity. Acidic amino acids can be natural or unnatural.

The term "natural amino acids" refers to biologically synthesized amino acids. Natural amino acids are generally of the L-type, but there are a few exceptions, such as glycine, both natural and synthesized by living organisms.

"Unnatural amino acids" means amino acids obtained by synthetic means.

The application is further elaborated below in connection with specific embodiments which, it should be understood, are intended only to illustrate the application and are not intended to limit the scope of the application. Test methods for which specific conditions are not indicated in the following embodiments are generally in accordance with conventional conditions or in accordance with conditions recommended by the manufacturer. All percentages, proportions, ratios, or portions are by weight unless otherwise indicated.

### Example 1:

### Synthesis of compound A:

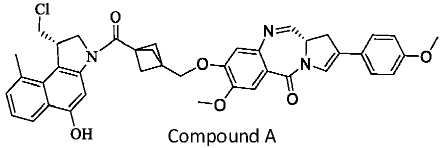

Compound **A was** synthesized according to the method provided in Example 14 "Synthesis of Compound 012" of Patent "CN109180681A".

### Example 2:

### Synthesis of compound B and compounds B1-9:

### 1. Preparation of compound B

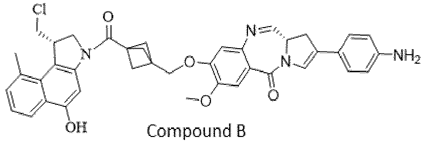

Compound **B** was synthesized according to the method provided in "General Step E: CBI-Benzodiazepine Derivative Synthesis Method I" of Patent "CN109180681A".

### 2. Preparation of compound B1

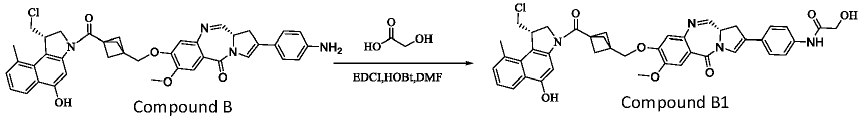

Compound **B** (50 mg, 0.072 mmol), hydroxyacetic acid (11 mg, 0.14 mmol), EDCI (20 mg, 0.10 mmol), HOBt (13.5 mg, 0.10 mmol) and 3 mLDMF were added to a 25 mL single-necked bottle, and the reaction was carried out at room temperature for 2h, and monitored by HPLC. At the end of the reaction, the reaction solution was directly purified by high performance liquid phase purification with HPLC to yield compound **B1** (42 mg) in 78 % yield, LC-MS: [M+H]⁺ = 747.2.

### 3. Preparation of compound B2

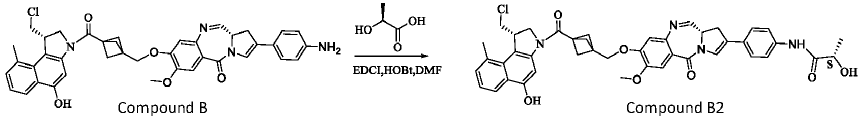

Referring to the synthesis method of compound **B1**, compound **B** (50 mg,0.072 mmol), L-lactic acid (12.6 mg,0.14 mmol), EDCI (20 mg,0.10 mmol), HOBt (13.5 mg,0.10 mmol), and 3 mLDMF were added to a 25 mL single-necked bottle, and the reaction was carried out at room temperature for 2h, and HPLC was used to monitor the reaction. At the end of the reaction, the reaction solution was directly purified by high-performance liquid phase purification with HPLC to obtain compound **B2** (37 mg) in 68 % yield, LC-MS: [M+H]⁺ = 761.2.

### 4. Preparation of compound B3

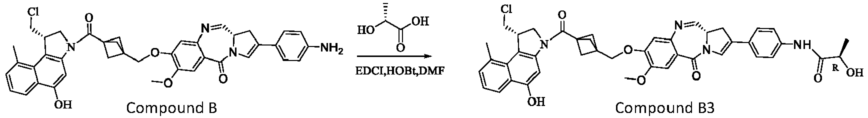

Referring to the synthesis of compound **B2**, L-lactic acid was replaced with D-lactic acid to obtain compound **B3**, LC-MS: [M+H]⁺ = 761.2.

### 5. Preparation of compounds B4 and B5

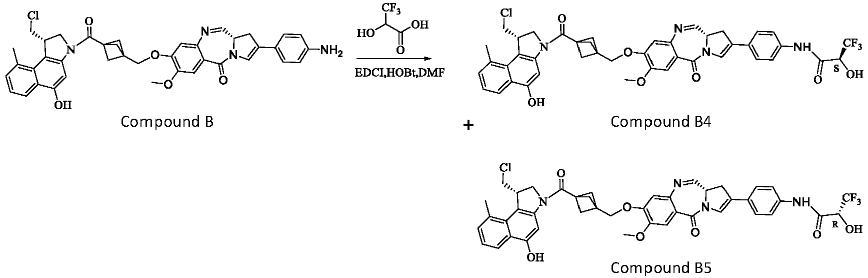

Compound **B** (60 mg, 0.087 mmol), 3,3,3-trifluorolactic acid (25 mg, 0.17 mmol), EDCI (25 mg, 0.13 mmol), HOBt (17.6 mg, 0.13 mmol), and 3 mLDMF were added to a 25 mL bottle, and the reaction was carried out at room temperature for 1.5 h, and the reaction was monitored by HPLC. At the end of the reaction, the reaction solution was directly purified by high-performance liquid phase purification with HPLC, and the product preparations were lyophilized to obtain compound **B4** (26 mg), compound **B5** (22 mg), LC-MS: [M+H]⁺ = 815.2, respectively.

### 6. Preparation of compounds B6 and B7

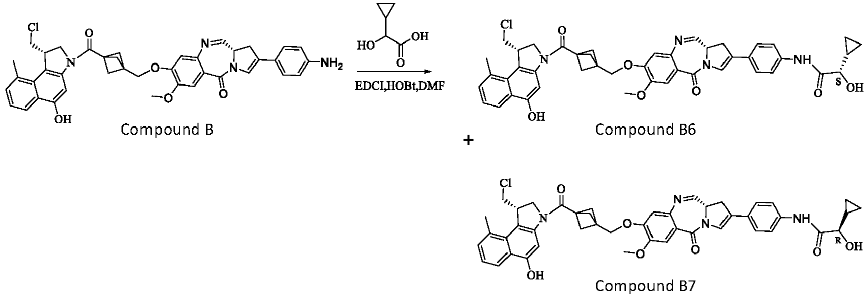

Referring to the synthesis of compounds **B4** and **B5**, the compounds **B6** and **B7** were obtained using high performance liquid phase purification, LC-MS: [M+H]⁺ =787.3.

### 7. Preparation of compounds B8 and B9

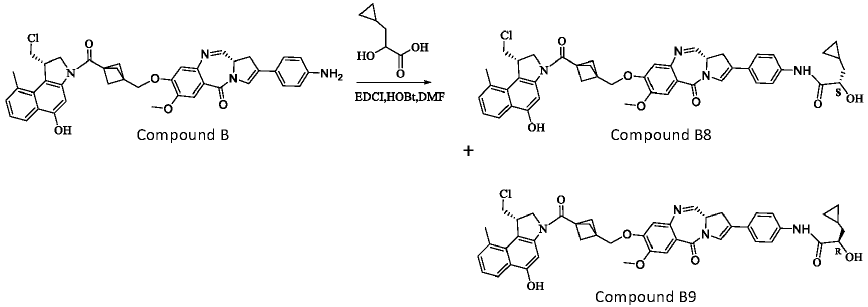

Referring to the synthesis of compounds **B4** and **B5**, compounds **B8** and **B9** were obtained by using high performance liquid phase purification, LC-MS: [M+H]⁺ =801.3.

### Example 3:

### Synthesis of compounds C and C1-9:

### 1. Preparation of compound C

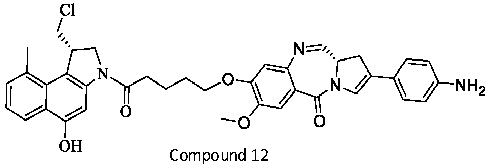

Compound **C** was synthesized according to the method provided in "General Step E: CBI-Benzodiazepine Derivative Synthesis Method II" of Patent "CN109180681A".

### 2. Preparation of compound C1

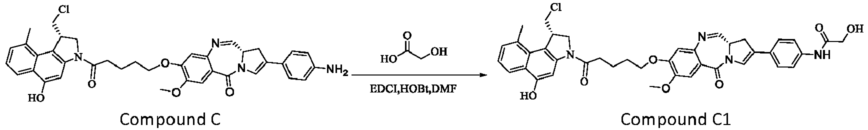

Compound **C** (50 mg, 0.075 mmol), hydroxyacetic acid (11.4 mg, 0.15 mmol), EDCI (21.6 mg, 0.11 mmol), HOBt(15.2 mg, 0.11 mmol) and 3 mLDMF were added to a 25 mL single-necked bottle, and the reaction was carried out at room temperature for 2h, and monitored by HPLC. At the end of the reaction, the reaction solution was directly purified by high performance liquid phase purification with HPLC to yield compound **C1** (35 mg) in 65% yield, LC-MS: [M+H]⁺ = 723.2.

### 3. Preparation of compound C2

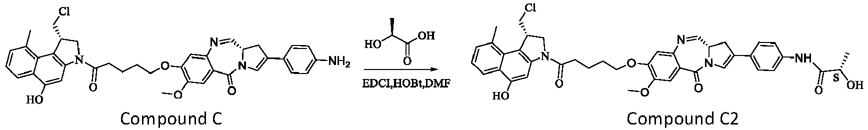

Referring to the synthesis method of compound **C1**, compound **C** (50 mg, 0.075 mmol), L-lactic acid (13 mg, 0.15 mmol), EDCI (21.6 mg, 0.11 mmol), HOBt(15.2 mg, 0.11 mmol), and 3 mLDMF were added to a 25 mL single-necked bottle, and the reaction was carried out for 2 h at room temperature, and the HPLC was monitored. Reaction. At the end of the reaction, the reaction solution was directly purified by high performance liquid phase purification with HPLC to yield compound **C2** (33.5 mg) in 63% yield, LC-MS: [M+H]⁺ = 737.2.

### 4. Preparation of compound C3:

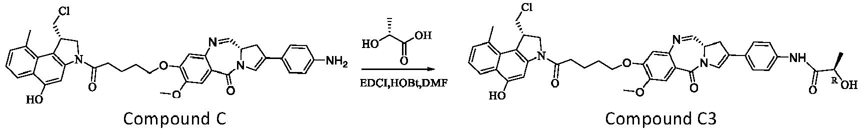

Referring to the synthesis of compound **C2**, L-lactic acid was replaced with D-lactic acid to obtain compound **C3**, LC-MS: [M+H]⁺ = 737.2.

### 5. Preparation of compounds C4 and C5:

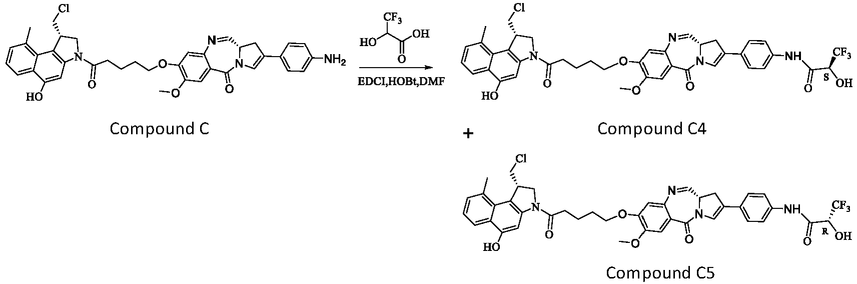

Compound **C** (60 mg, 0.09mmol), 3,3,3-trifluorolactic acid (26 mg, 0.18 mmol), EDCI (25 mg, 0.13 mmol), HOBt (17.6 mg, 0.13 mmol), and 3 mLDMF were added to a 25 mL single-necked bottle, and the reaction was carried out at room temperature for 1.5 h, and the reaction was monitored by HPLC. At the end of the reaction, the reaction solution was directly purified by high performance liquid phase purification with HPLC, and the product preparations were lyophilized to obtain compound **C4** (21mg), compound **C5** (25mg),LC-MS: [M+H]⁺ =791.2, respectively.

### 6. Preparation of compounds C6 and C7:

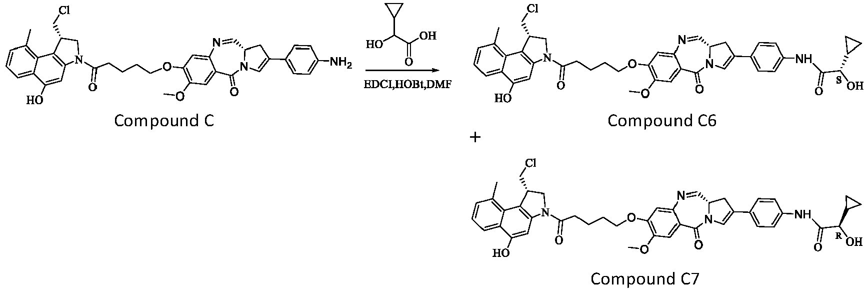

Referring to the synthesis of compounds **C4** and **C5**, compounds **C6** and **C7** were obtained by using high performance liquid phase purification, LC-MS: [M+H]⁺ =763.3.

### 7. Preparation of compounds C8 and C9:

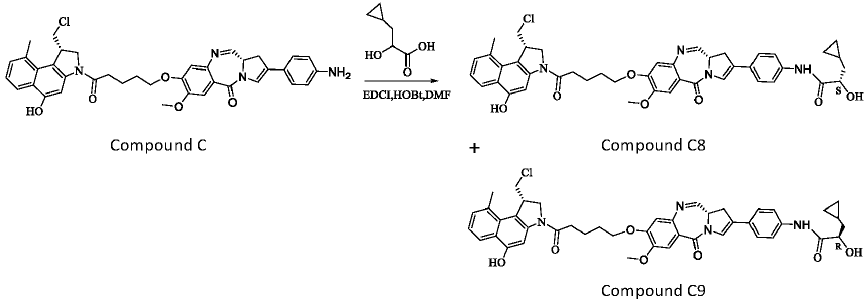

Referring to the synthesis of compounds **C4** and **C5**, compounds **C8** and **C9** were obtained by using high performance liquid phase purification, LC-MS: [M+H]⁺ =777.3.

### Example 4:

### Synthesis of compounds D1 and D1-9:

### 1. Preparation of compound D

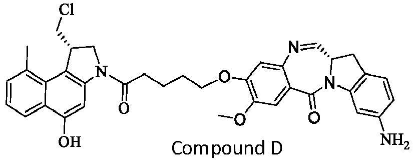

Compound **D** was synthesized by the method provided in "General Step A: Synthesis of Benzodiazepine Derivatives I and General Step E: CBI-Benzodiazepine Derivatives Synthesis Method II" of Patent "CN109180681A".

### 2. Preparation of compound D1

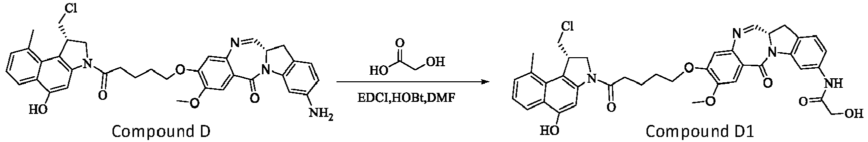

Compound **D** (50 mg, 0.078 mmol), hydroxyacetic acid (12 mg, 0.16 mmol), EDCI (22.4 mg, 0.12 mmol), HOBt (15.8 mg, 0.12 mmol) and 3 mLDMF were added to a 25 mL single-necked bottle and the reaction was carried out at room temperature for 2h, and monitored by HPLC. At the end of the reaction, the reaction solution was directly purified by high performance liquid phase purification with HPLC to yield compound **D1** (34 mg) in 62.5 % yield, LC-MS: [M+H]⁺ = 697.2.

### 3. Preparation of compound D2

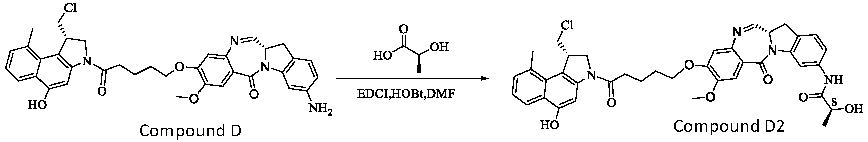

Compound **D** (50 mg, 0.078 mmol), L-lactic acid (14 mg, 0.16 mmol), EDCI(22.4 mg, 0.12 mmol), HOBt (15.8 mg, 0.12 mmol) and 3 mLDMF were added to a 25 mL single-necked bottle, and the reaction was carried out at room temperature for 2 h. The reaction was monitored by HPLC. At the end of the reaction, the reaction solution was directly purified by high performance liquid phase purification with HPLC to yield compound **D2** (33.8 mg) in 71 % yield, LC-MS: [M+H]⁺ = 711.2.

### 4. Preparation of compound D3

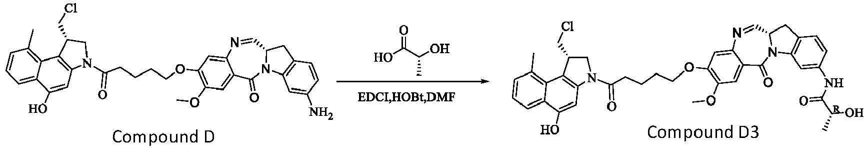

Referring to the synthesis of compound **D2**, D-lactic acid was substituted forL-lactic acid to obtain compound **D3**, LC-MS: [M+H]⁺ = 711.2.

### 4. Preparation of compounds D4 and D5

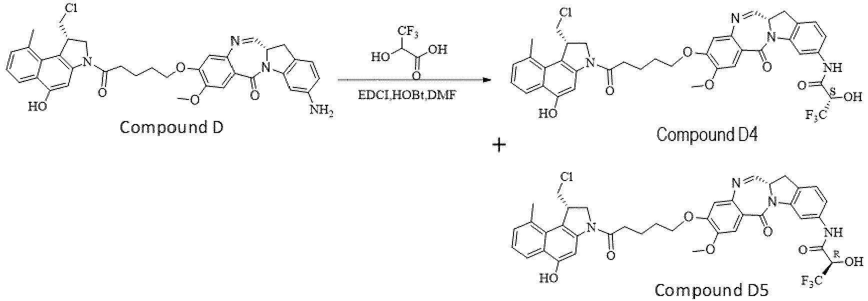

Compound **D** (60 mg, 0.094 mmol), 3,3,3-trifluorolactic acid (27 mg, 0.187mmol), EDCI (27 mg, 0.14 mmol), HOBt (19 mg, 0.14 mmol), and 3 mLDMF were added to a 25 mL single-necked bottle, and the reaction was carried out at room temperature for 1.5 h, and the reaction was monitored by HPLC. At the end of the reaction, the reaction solution was directly purified by high-performance liquid phase purification with HPLC, and the product preparations were lyophilized to obtain compound **D4** (27mg), compound **D5** (29mg), LC-MS: [M+H]⁺ =765.2, respectively.

### 6. Preparation of compounds D6 and D7

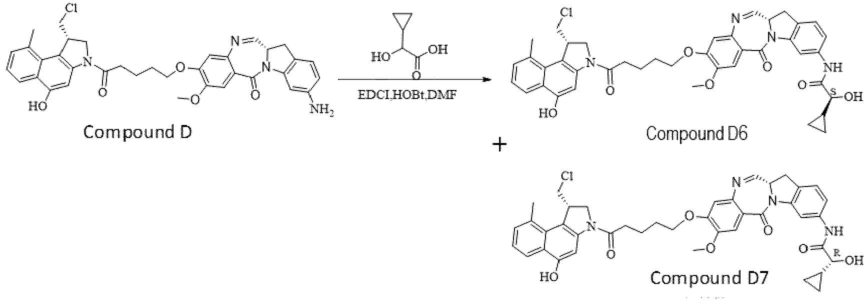

Referring to the synthesis of compounds **D4** and **D5**, compounds **D6** and **D7** were obtained by using high performance liquid phase purification, LC-MS: [M+H]⁺ =73 7.3.

### 7. Preparation of compounds D8 and D9

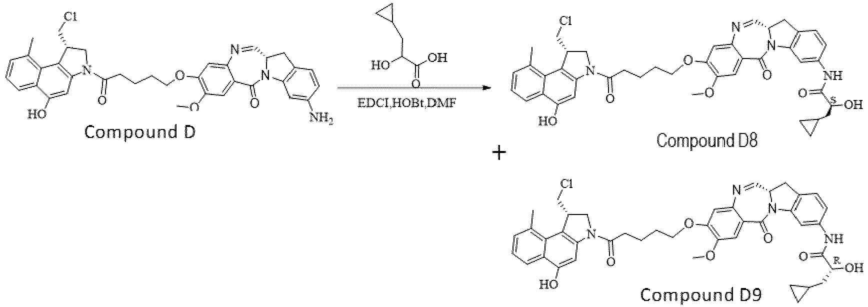

Referring to the synthesis of compounds **D4** and **D5**, compounds **D8** and **D9** were obtained by using high performance liquid phase purification, LC-MS: [M+H]⁺ =751.3.

### Example 5:

### Synthesis of compound M1:

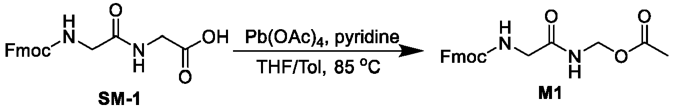

N-Fmoc-glycine-glycine(100 g, 28 mmol, 1.0 eq), lead tetraacetate (175 g, 395 mmol, 1.4 eq), 2 L tetrahydrofuran and 670 mL toluene were added to a 5 L single-necked bottle, stirred well, and the reaction was heated to 85°C for 2.5 h. The reaction was monitored by TLC, and the raw material was finished reacting, then it was cooled to room temperature, filtered, and the filtrate concentrated under reduced pressure, the residue was purified by column chromatography (PE:EA=5:1-2:1) to yield compound **M1** (87 g) in 84.4% yield, LC-MS: [M+NH ]₄⁺ =3 86.0.

### Example 6:

### Synthesis of compound M3:

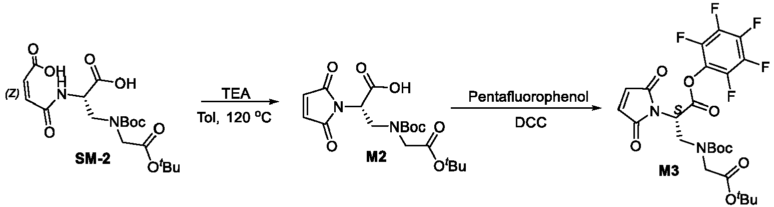

In a 1000 mL bottle, compound **SM-2** (synthesized according to the method published in patent CN108452321A) (40 g, 96 mmol, 1.0 eq), triethylamine (26.7 mL, 2.0 eq), toluene (400 mL) were added, and the reaction was carried out by refluxing the compound at a temperature of 120°C for 2 h. The reaction was monitored to be almost complete by TLC, and then spun off at a temperature of 50°C under reduced pressure. The solvent was removed by spinning under reduced pressure at 50°C. Dissolve with ethyl acetate (150 mL), water (40 mL), adjust pH to 2-3 with 1M HCl under ice bath stirring, and partition. The aqueous layer was extracted once more with ethyl acetate, the organic layers were combined and dried by adding anhydrous sodium sulfate. After filtration, a light yellow oily crude was obtained by concentration, and the crude was purified by column chromatography (DCM:MeOH=40:1) to obtain 26.6 g of compound **M2**; LC-MS: [M+H]⁺ =399.3.

In a 1L single-necked bottle, compound **M2** (26.5 g, 60.5 mmol, 1.0 eq), pentafluorophenol (12.2 g, 66.5 mmol, 1.1 eq), DCC (13.7 g, 66.5 mmol, 1.1 eq) and THF (300 mL) were added, and the reaction was carried out at room temperature for 30 min (monitored by TLC), and insoluble material was filtered off. The reaction solution was purified directly by preparation, and the preparative solution was concentrated by pumping water under reduced pressure at 35°C to remove acetonitrile, and lyophilized to obtain 31.5 g of compound **M3** in 64% yield; LC-MS: [M+H]⁺ =565.1.

### Example 7:

### Synthesis of compound 1:

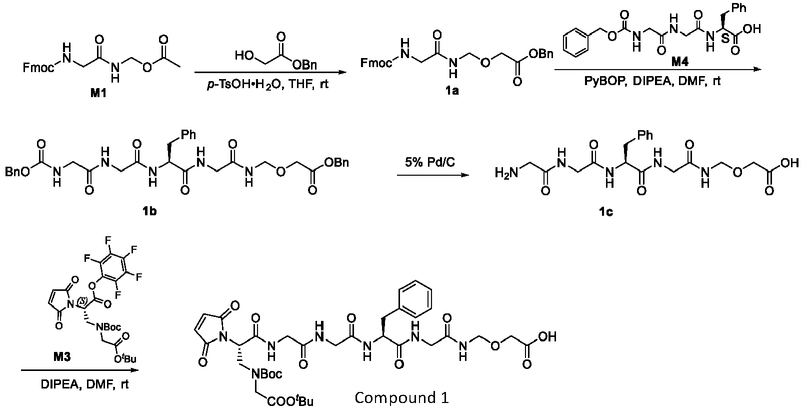

### Step 1: Synthesis of compound 1a

In a 250 mL bottle, add **M1** (6 g, 16.3 mmol), 100 mL THF, p-toluenesulfonic acid monohydrate (0.31 g, 1.63 mmol), stirring and cooled to 0°C, dropwise addition of benzyl hydroxyacetate (5.4 g, 32.6 mmol), dropwise natural warming to room temperature reaction (the reaction is about 2-4 h), TLC monitoring. At the end of the reaction, saturated NaHCO₃ solution was added, extracted with ethyl acetate, washed with saturated sodium chloride solution, dried with anhydrous sodium sulfate, filtered, concentrated, and the residue was purified by silica gel column (PE:EA=10:1-5:1-1:1) to obtain Compound **1a** (4 g) in 52% yield; LC-MS: [M+H]⁺ =475.2.

### Step 2: Synthesis of compound 1b

In a 25 mL single-necked bottle, compound **1a** (2 g, 4.2 mmol) was added, 10 mL DMF, stirred at 0 °C, DBU (766 mg, 5.04 mmol) was added, and the reaction was carried out for 1 h. The completion of Fmoc deprotection was monitored by TLC and set aside;
Another 25 mL single-necked bottle was filled with compound **M4** (prepared by the method published in patent CN111051330 A) (1.73 g, 4.2 mmol), PyBOP (2.61 g, 5.04 mmol), HOBt (680 mg, 5.04 mmol), and 10 mL of DMF, and DIPEA (830 uL, 5.04 mmol) was added to the reaction bottle under an ice-water bath, and stirring was continued for 30 min. 5.04 mmol), continue stirring for 30 min, the above reaction solution was added to the reaction flask, and the reaction was raised to room temperature. After HPLC monitoring the end of the reaction, the reaction solution was purified by preparative liquid phase to obtain the product preparation, the preparation was extracted by dichloromethane, washed with saturated sodium chloride solution, dried with anhydrous sodium sulfate, filtered, and the filtrate was concentrated under reduced pressure to obtain compound **1b** (1.7 g) in 63% yield; LCMS: [M+H]⁺ =648.3.

### Step 3: Synthesis of compound 1c

Compound **1b** (900 mg, 1.39 mmol) was added into a 25 mL single-necked bottle, 15 mL DMF was used to dissolve it, then 900 mg of 5% Pd/C was added, and hydrogenation reaction was carried out for 2 h. After the reaction was completed, it was filtered to obtain the filtrate, which was used for the next step of the reaction directly without purification.

### Step 4: Synthesis of compound 1

The DMF solution of compound **1c** obtained in the previous step was placed in an ice-waterbath, DIPEA (235 uL, 1.39 mmol) was added, and compound **M3** (784 mg, 1.39 mmol) was added, and the reaction was brought to room temperature for 1 h. The completion of the reaction was monitored by HPLC, and the reaction solution was purified by high-performance liquid chromatography (HPLC) to obtain the preparative solution, which was lyophilized to obtain compound **1** (604 mg). Yield 54%; LC-MS: [M+H]⁺ =804.4.

### Example 8:

### Synthesis of compound 2:

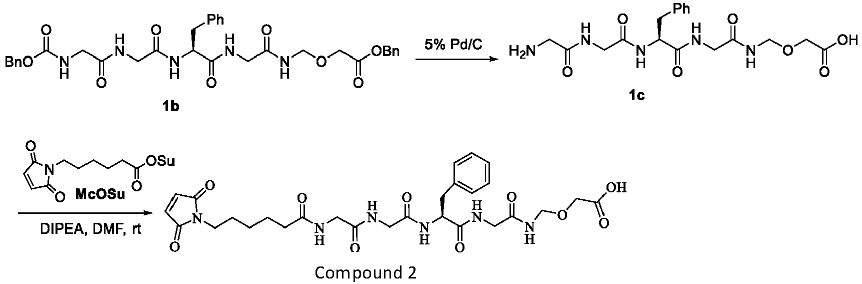

### Step 1: Synthesis of compound 1c

Compound **1b** (900 mg, 1.39 mmol) was added into a 25 mL single-necked bottle, 15 mL DMF was used to dissolve it, then 900 mg of 5% Pd/C was added, and hydrogenation reaction was carried out for 2 h. After the reaction was completed, it was filtered to obtain the filtrate, which was used for the next step of the reaction directly without purification.

### Step 2: Synthesis of compound 2

The DMF solution of compound **1c** obtained in the previous step was placed in an ice-waterbath, DIPEA (235 uL, 1.39 mmol) was added, and then McOSu (428.5 mg, 1.39 mmol) was added, and the reaction was brought to room temperature for 1 h. The completion of the reaction was monitored by HPLC, and the reaction solution was purified by high-performance liquid chromatography to obtain the preparative solution, which was lyophilized to obtain compound **2** (500 mg) in 58% yield; LC-MS: [M+H] =617.3. Compound 2 (500 mg) was lyophilized in 58% yield; LC-MS: [M+H]⁺ =617.3.

### Example 9:

### Synthesis of compounds 3A and 3B:

### 1. Preparation of compound 3A

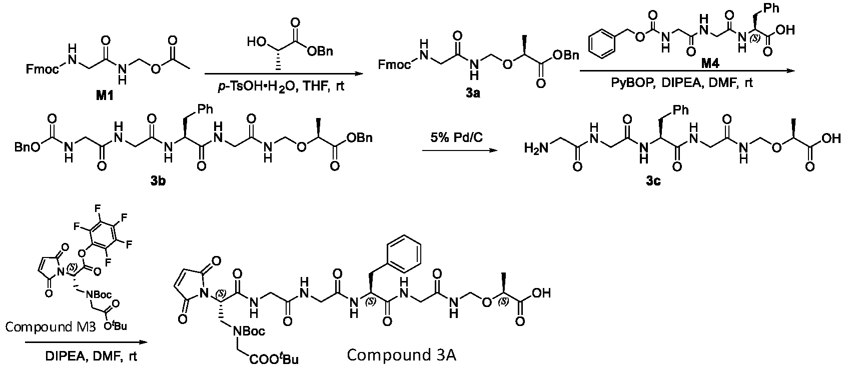

### Step 1: Synthesis of compound 3a

**M1** (1.5 g, 4.0 mmol, 1.0 eq), p-toluenesulfonic acid monohydrate (77 mg, 0.4 mmol, 0.1 eq) and 15 mL of THF were added in a 25 mL bottle, stirred well, and then lowered to 0°C, and then benzyl L-lactate (2.2 g, 12.0 mmol, 3 eq) was slowly added, and then the reaction was raised to room temperature after the addition of benzyl L-lactate.The reaction was monitored by TLC. At the end of the reaction, saturated NaHCO₃ solution was added, extracted with ethyl acetate, dried over anhydrous sodium sulfate, filtered, concentrated, and the residue was purified by reversed-phase column to give compound **3a** (1.03 g) in 52% yield, LC-MS: [M+NH ]₄⁺ =506.2.

### Step 2: Synthesis of compound 3b

Compound **3a** (1 g, 2.04 mmol) and 8 mL of DMF were added in a 25 mL single-necked bottle, stirred well, and then lowered to 0°C, and then DBU (373 mg, 2.45 mmol) was slowly added, and the reaction was brought up to room temperature after the addition of the compound.The reaction was monitored by TLC, and the end of the reaction was recorded as the reaction solution ①;
Another 25 mL single-necked bottle was taken and **M4** (846 mg, 2.04 mmol), PyBOP (1.27 g, 2.45 mmol) and 6 mLofDMF were added, stirred at room temperature for 5 min, and the reaction solution ① was added, the reaction was carried out at room temperature, and monitored by HPLC. The reaction was completed, and the reaction solution was purified by high performance liquid phase purification with HPLC and lyophilized to give compound **3b**(913 mg) in 67.6%yield, LC-MS: [M+NH ]₄⁺=679.2.

### Step 3: Synthesis of compound 3c

Compound **3b** (800 mg, 1.21 mmol, 1.0 eq) was added in a 100 mL single-necked bottle, DMF (15 mL) was dissolved, then 5% Pd/C (800 mg) was added and hydrogenated for 2 h at room temperature (HPLC was used to monitor the progress of the reaction). The Pd/C was filtered and the filtrate was used directly for the next step without concentration.

### Step 4: Synthesis of compound 3A

The DMF solution of compound **3c** obtained in the previous step was placed in an ice-water bath, DIPEA (219 uL, 1.21 mmol) was added, and compound **M3** (683 mg, 1.21 mmol) was added, and the reaction was brought to room temperature for 1 h. The reaction was monitored by HPLC, and the reaction solution was purified by high performance liquid chromatography (HPLC) to obtain the preparative solution, which was lyophilized to obtain compound **3A** (524 mg) in 53% yield, [M-H]-=816.3 by LC-MS. Yield 53%, LC-MS: [M-H]- =816.3.

### 2. Preparation of compound 3B

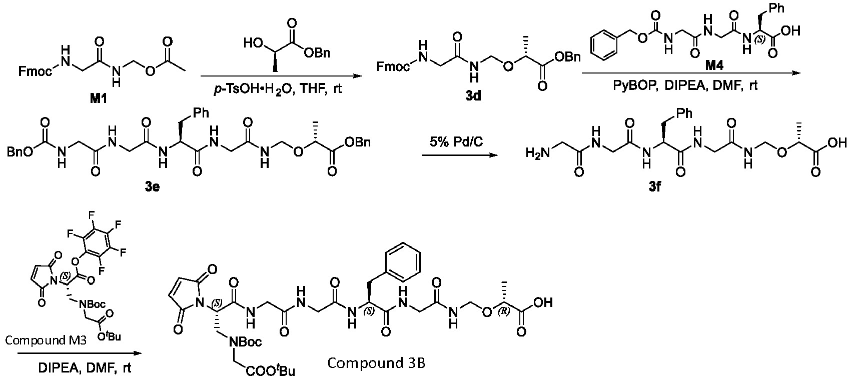

Referring to the synthesis method of compound **3A** above, benzyl L-lactate was changed to benzyl D-lactate in the first step to obtain compound **3B**, LC-MS: [M-H]⁻=816.3.

### Example 10:

### Synthesis of compounds 4A and 4B:

### 1. Preparation of compound 4A

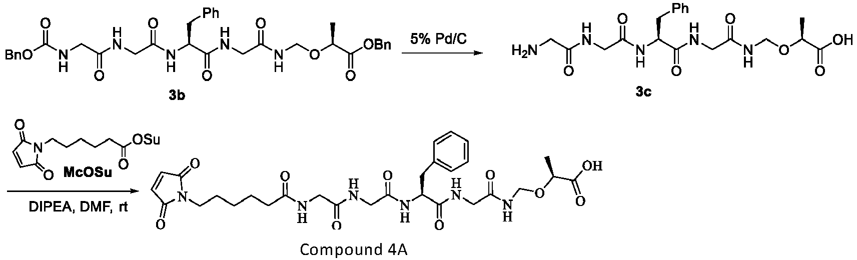

### Step 1: Synthesis of compound 3c

**3b** (800 mg, 1.21 mmol, 1.0 eq) was added to a 100 mL single-necked bottle, dissolved in DMF (15 mL), then 5%Pd/C (800 mg) was added, and the hydrogenation reaction was carried out for 2 h at room temperature (HPLC was used to monitor the progress of the reaction). The Pd/C was filtered and the filtrate was used directly for the next step without concentration.

### Step 2: Synthesis of compound 4A

The DMF solution of compound **3c** obtained in the previous step was placed in an ice-water bath, DIPEA (219 uL, 1.21 mmol) was added, then McOSu (373 mg, 1.21 mmol) was added, and the reaction was brought to room temperature for 1 h. The completion of the reaction was monitored by HPLC, and the reaction solution was purified by high-performance liquid chromatography (HPLC) to obtain the preparation, which was lyophilized to obtain compound **4A** (450 mg), 59% yield; LC-MS. Compound 4A (450 mg) was lyophilized in 59% yield; LC-MS: [M+H]⁺ =631.3.

### 2. Synthesis of compound 4B

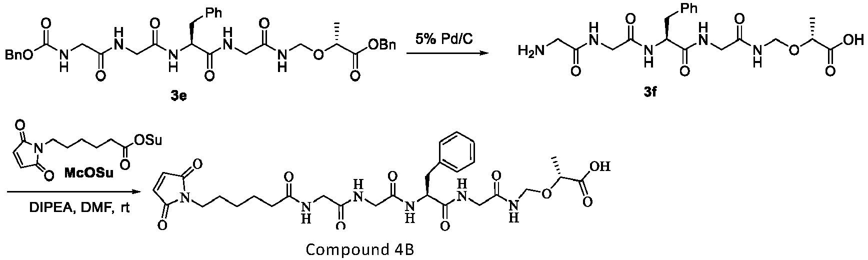

Referring to the synthesis of compound **4A**, compound **4B** was obtained, LC-MS: [M+H]⁺ = 631.3.

### Example 11:

### Synthesis of compound 5:

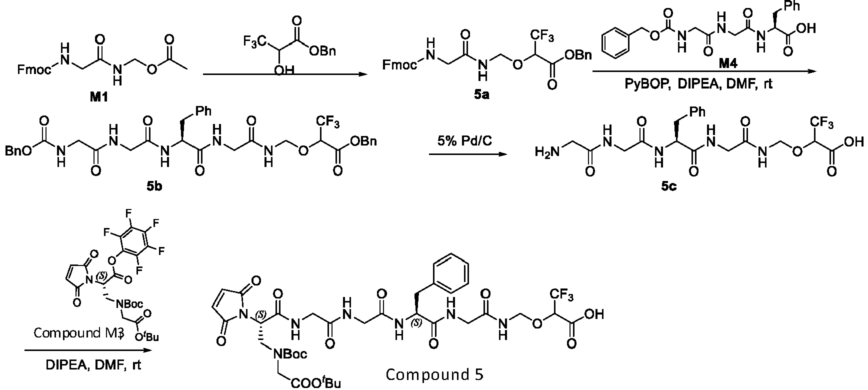

### Step 1: Synthesis of compound 5a

In a 250 mL bottle, add **M1** (10 g, 27.1 mmol), benzyl 3, 3, 3-trifluorolactate (prepared according to the method published in Patent WO2020063673A1) (12.7 g, 54.3 mmol), zinc acetate (9.96 g, 54.3 mmol), and 100 mL of toluene, and react at 100°C for 4 h. The reaction was completed. After the reaction was completed, reduced to room temperature, filtered to remove insoluble material, and the filtrate was concentrated to obtain the crude product. The crude product was purified by silica gel column chromatography (PE:EA=10:1-5:1-2:1) to obtain compound **5a** (5.15)g, yield 35.1%; LC-MS: [M+H]⁺ =543.2.

### Step 2: Synthesis of compound 5b

Compound **5a** (5 g, 9.2 mmol) and 15 mL of DMF were added to a 50 mL single-necked bottle, dissolved and cleared, and then, under an ice-waterbath, DBU (1.68 g, 11 mmol) was added and the reaction was carried out for 1 h, which was recorded as reaction solution ①;
Another 50 mL bottle was taken, **M4** (3.8 g, 9.2 mmol), PyBOP (5.75 g, 11 mmol), HOBt (1.49 g, 11 mmol) and 10 mLDMF were added, and after dissolved, DIPEA(1.82 mL, 11 mmol) was added in an ice-water bath, and the reaction was continued for 30 min, then reaction solution ① was added, and the reaction was brought to room temperature for2 h. The reaction was monitored by HPLC. The reaction was monitored by HPLC for 2 h. After completion of the reaction, the reaction solution was purified by high performance liquid chromatography (HPLC) to obtain the preparative solution. After extraction with dichloromethane, washing with saturated sodium chloride solution, drying with anhydrous sodium sulfate, filtration and concentration, compound **5b** (4.1) g was obtained in 62.3% yield; LC-MS: [M+H]⁺ =716.3.

### Step 3: Synthesis of compound 5

Compound **5b** (900 mg, 1.26 mmol) was added into a 25 mL bottle, 15 mL of DMF was used to dissolve the compound, then 900 mg of 5% Pd/C was added and the reaction was hydrogenated for 2 h. After the reaction was completed, the filtrate was filtered, and the filtrate was placed in an iced-water bath, and DIPEA (228 uL, 1.38 mmol) was added, followed by the addition of compound **M3** (712 mg, 1.26 mmol). The filtrate was placed in an ice water bath, DIPEA (228 uL, 1.38 mmol) was added, and compound M3 (712 mg, 1.26 mmol) was added, and the reaction was brought to room temperature for 1 h. The completion of the reaction was detected by HPLC, and the reaction solution was purified by high performance liquid chromatography (HPLC) to obtain the preparative solution, which was lyophilized to obtain the product Compound **5** (525) mg, with a yield of 47.9%; LC-MS: [M-H]- = 870.3.

### Example 12:

### Synthesis of compound 6:

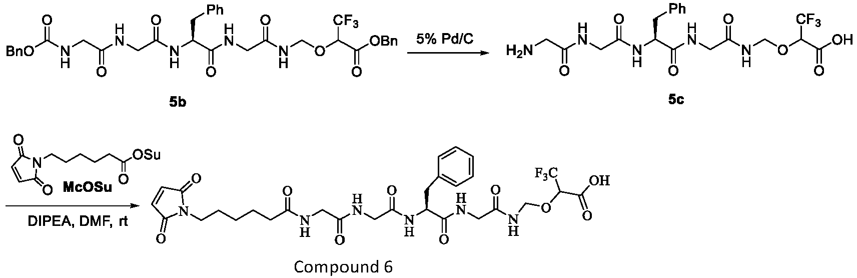

Compound **5b** (900 mg, 1.26 mmol) was added into a 25 mL bottle, 15 mL of DMF was used to dissolve the compound, then 900 mg of 5% Pd/C was added and the hydrogenation reaction was performed for 2 h. After the reaction was completed, the filtrate was filtered, and the filtrate was placed in an ice-water bath, and DIPEA (228 uL, 1.38 mmol) was added, followed by McOSu (388 mg, 1.26 mmol), and the reaction was brought to room temperature for 1 h. The reaction was monitored by HPLC, and the reaction solution was purified by high performance liquid phase purification with HPLC to obtain the preparative solution, which was lyophilized to obtain the product compound 6 (450). The filtrate was placed in an ice-water bath, DIPEA (228 uL, 1.38 mmol) was added, McOSu (388 mg, 1.26 mmol) was added, and the reaction was brought to room temperature for 1 h. The completion of the reaction was detected by HPLC, and the reaction solution was purified by high-performance liquid-phase purification to obtain the preparative solution, which was lyophilized to obtain the product compound **6** (450) mg in 52% yield; LC-MS: [M-H]- =683.3.

### Example 13:

### Synthesis of compound 7:

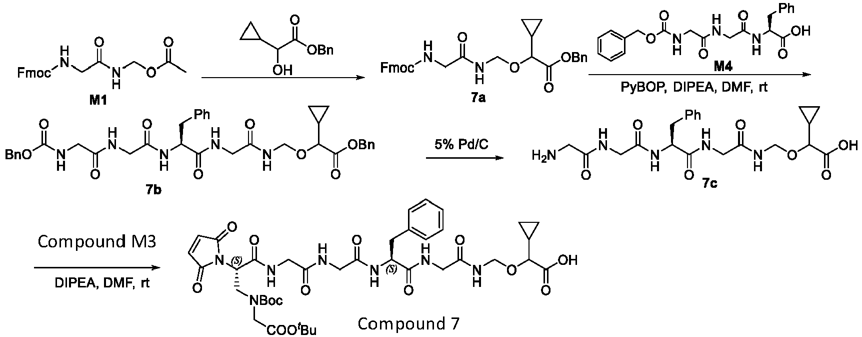

### Step 1: Synthesis of compound 7a

Add **M1** (10 g, 27.1 mmol), benzyl 2-cyclopropyl-2-hydroxyacetate (prepared according to the method published in patent WO2020244657A1) (11.2 g, 54.3 mmol), zinc acetate (9.96 g, 54.3 mmol) and 100 mL of toluene in a 250 mL single-necked bottle, and heat the reaction to 100°C for 4 h. The reaction was completed. The reaction was reduced to room temperature, filtered to remove insoluble material, and the filtrate was concentrated to obtain the crude product. The crude product was purified by silica gel column chromatography (PE:EA=10:1-5 :1-2:1) to obtain compound **7a** (4.97 g) in 36% yield; LC-MS: [M+H]⁺ =515.2.

### Step 2: Synthesis of compound 7b

Compound **7a** (4 g, 7.8 mmol) and 10 mL of DMF were added to a 50 mL bottle. After dissolution, DBU (1.42 g, 9.3 mmol) was added to the bottle in an ice-water bath and reacted for 1 h. The reaction was recorded as reaction solution ①;
Another 50 mL single-necked bottle was taken, and **M4** (3.2 g, 7.8 mmol), PyBOP (4.5 g, 8.6 mmol), HOBt (1.16 g, 8.6 mmol) and 10 mL of DMF were added, and after dissolved, DIPEA (1.65 mL, 10 mmol) was added in an ice-water bath, and the reaction was continued for 30 min, then reaction solution ① was added, and the reaction was brought to room temperature for 2 h. The reaction process was monitored by HPLC, and the preparation was purified by high performance liquid phase purification with HPLC. The reaction was monitored by HPLC for 2 h. After completion of the reaction, the reaction solution was purified by high performance liquid chromatography (HPLC) to obtain the preparative solution. The prepared solution was extracted with dichloromethane, washed with saturated sodium chloride solution, dried with anhydrous sodium sulfate, filtered and concentrated to obtain compound **7b** (4.2 g) in 78% yield; LC-MS: [M+H]⁺ =688.3.

### Step 3: Synthesis of compound 7

Compound **7b** (1000 mg, 1.45 mmol) was added into a 25 mL bottle, 15 mL of DMF was used to dissolve it, then 1000 mg of 5% Pd/C was added, and hydrogenation reaction was carried out for 2 h. After completion of the reaction, filtration was carried out, and the filtrate was placed in an ice-water bath, and DIPEA (248 uL, 1.5 mmol) was added, followed by the addition of **compound M3** (720 mg, 1.45 mmol). The filtrate was placed in an ice water bath, DIPEA (248 uL, 1.5 mmol) was added, and compound M3 (720 mg, 1.45 mmol) was added, and the reaction was brought to room temperature for 1 h. The completion of the reaction was detected by HPLC, and the reaction solution was purified by high performance liquid chromatography (HPLC) to obtain the preparative solution, which was lyophilized to obtain the product **Compound** 7 (503 mg), with a yield of 41%; LC-MS: [M-H]- =842.3.

### Example 14:

### Synthesis of compound 8:

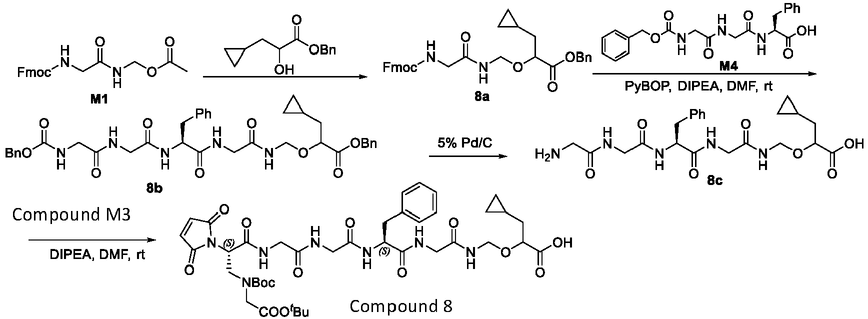

### Step 1: Synthesis of compound 8a

Add **M1** (10 g, 27.1 mmol), benzyl 2-hydroxy-3-cyclopropylpropionate (synthesized by the method published in patent WO2020063676A) (12.0 g, 54.3 mmol), zinc acetate (9.96 g, 54.3 mmol) and 100 mL of toluene in a 250 mL single-necked bottle, and react at 100°C for 4 h. Reaction was completed. The reaction was reduced to room temperature, filtered to remove insoluble material, and the filtrate was concentrated to obtain the crude product. The crude product was purified by silica gel column chromatography (PE:EA=10:1-5:1-2:1) to obtain the target **8a** (5.09 g); LC-MS: [M+H]⁺ =529.2.

### Step 2: Synthesis of compound 8b

Compound **8a** (4 g, 7.6 mmol) and 10 mL of DMF were added to a 50 mL single-necked bottle, and after dissolution, DBU (1.39 g, 9.1 mmol) was added in an ice-water bath and the reaction was carried out for 1 h, which was recorded as reaction solution ①;
Another 50 mL single-necked bottle was taken, and **M4** (3.12 g, 7.6 mmol), PyBOP (4.5 g, 8.6 mmol), HOBt (1.16 g, 8.6 mmol) and 10 mL of DMF were added, and after dissolved, DIPEA (1.65 mL, 10 mmol) was added in an ice-water bath and the reaction was continued for 30 min, and then the reaction solution (1) was added to the reaction solution and the reaction was brought to room temperature for 2 h. HPLC was performed to monitor the reaction progress, and the reaction solution was purified by high performance liquid phase purification to obtain the preparative solution. The reaction was monitored by HPLC, and the reaction solution was purified by high performance liquid chromatography (HPLC) to obtain the preparative solution. The prepared solution was extracted with dichloromethane, washed with saturated sodium chloride solution, dried with anhydrous sodium sulfate, filtered and concentrated to obtain compound **8b** (4.5 g) in 84% yield; LC-MS: [M+H]⁺ =702.3.

### Step 3: Synthesis of compound 8

In a 25 mL bottle, **8b** (1000 mg, 1.42 mmol) was added, 15 mL of DMF was dissolved, then 1000 mg of 5%Pd/C was added, and hydrogenation reaction was carried out for 2 h. After completion of the reaction, filtration was performed, and the filtrate was placed in an ice-water bath, and DIPEA (248 uL, 1.5 mmol) was added, followed by compound **M3** (708 mg, 1.42 mmol), and the reaction was raised to room temperature for 1 h. The reaction was monitored by HPLC, and the reaction solution was purified by high performance liquid phase purification with HPLC to give the preparative solution, which was lyophilizedto give compound 8 (443 mg) in good yield. After the addition, the reaction was brought to room temperature for 1 h. The completion of the reaction was monitored by HPLC, and the reaction solution was purified by high performance liquid phase purification with HPLC to obtain the preparative solution, which was lyophilizedto obtain the product compound **8** (443 mg) in 36% yield; LC-MS: [M-H]- =856.4.

### Example 15:

### Synthesis of compound 9:

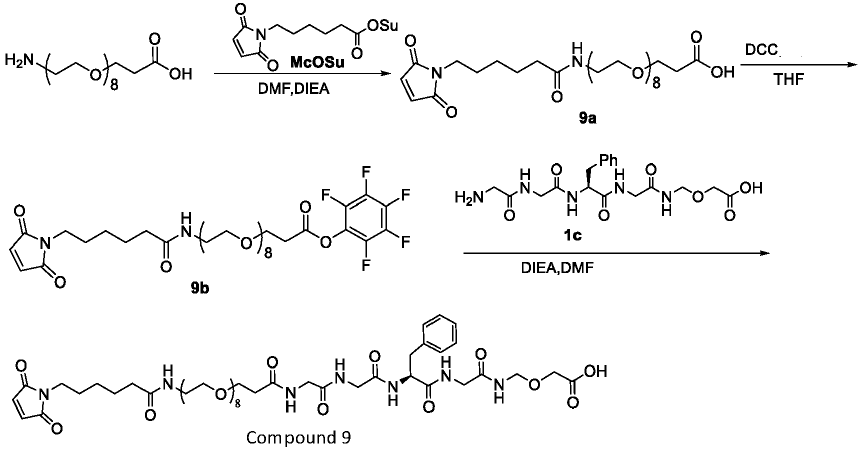

### Step 1: Synthesis of compound 9a

Amino-octa polyethylene glycol-carboxylic acid (10 g, 22.7 mmol) was added to a 500 mL single-necked bottle, 20 mLD MF was dissolved and cleared, McOSu (8.38 g, 27.2 mmol) was added, DIEA(5.6 mL,3.4 mmol) was added, the reaction was carried out at room temperature for 2h, and the reaction was monitored by TLC. At the end of the reaction, the reaction solution was poured into 100 mL of water, extracted with dichloromethane three times, the organic phases were combined, washed twice with saturated sodium chloride solution and dried with anhydrous sodium sulfate. It was filtered and concentrated under reduced pressure at 45°C to obtain the crude product. The crude product was purified by silica gel column chromatography (DCM:MeOH=30:1-10:1), the product eluate was collected and concentrated to obtain compound **9a** (12.1 g) in 84.5% yield, LCMS: [M-H]⁺ =633.3.

### Step 2: Synthesis of compound 9b

In a 500 mL single-necked bottle, compound **9a** (12.0 g, 18.9 mmol), pentafluorophenol (3.83 g, 20.8 mmol), DCC (4.28 g, 20.8 mmol), and THF (50 mL) were added, and the reaction was carried out at room temperature for 1h and monitored by TLC. At the end of the reaction, the insoluble material was filtered. The filtrate was concentrated at45 °Cunderreducedpressureto remove the solventto obtain the residue. The residue was purified by silica gel column chromatography (PE:EA=5:1-2:1), and the product eluate was collected and concentrated to obtain comp ound **9b** (13.8 g) in 91.5% yield, LCMS: [M+H]⁺ =801.3.

### Step 3: Synthesis of compound 9

To compound **1c** (220 mg, 0.5 mmol), 10 mL of DMF was added, and compound **9b** (500 mg, 0.6 mmol) was added in an ice-water bath and cooled to 0°C. Compound DIPEA (124 uL, 0.75 mmol) was added, and the reaction was carried out at room temperature for 1 h and monitored by HPLC. After the reaction was completed, the reaction solution was purified by preparative grade HPLC to obtain the product preparation solution, which was lyophilized after removing acetonitrile under reduced pressure to obtain compound **9** (430 mg) in 83% yield, LCMS:[M-H]⁺ =1038.5.

### Example 16:

### Synthesis of compound 10:

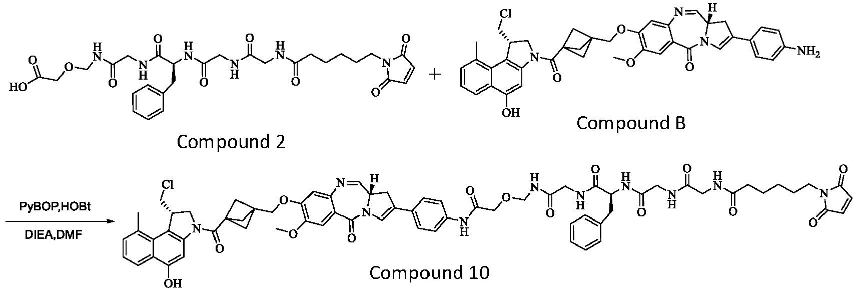

**Compound 2** (50 mg, 0.081 mmol), **Compound B** (55.8 mg, 0.081 mmol), PyBOP (50.4 mg, 0.097 mmol), HOBt (13.1 mg, 0.097 mmol), and 5 mL of DMF were added to a 25 mL bottle, and DIPEA (15.5 mg, 0.12 mmol) was added to the bottle in an ice-water bath. 0.12 mmol) in an ice water bath, and the reaction was brought to room temperature for 2 h. After the completion of the reaction was monitoredby HPLC, the reaction solution was purified by high performance liquid chromatography (HPLC) to obtain the preparative solution, which was lyophilized to give Compound **10** (80 mg) in 76.7% yield, LC-MS: [M+H]⁺ =1287.5.

### Example 17:

### Synthesis of compound 11:

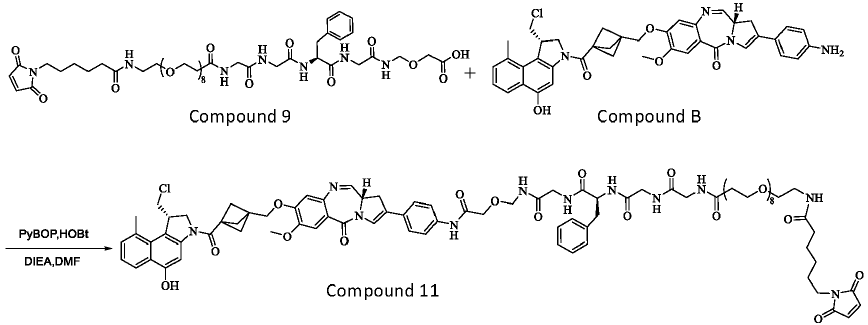

**Compound 9** (84.2 mg, 0.081 mmol), **Compound B** (55.8 mg, 0.081 mmol), PyBOP (50.4 mg, 0.097 mmol), HOBt (13.1 mg, 0.097 mmol), and 5 mL of DMF were added to a 25 mL bottle, and DIPEA (15.5 mg, 0.12 mmol) was added to the bottle in an ice water bath. 0.12 mmol) in an ice water bath, and the reaction was brought to room temperature for 2 h. After the completion of the reaction was monitoredby HPLC, the reaction solution was subjected to high performance liquid phase purification to obtain the product preparation, and the preparation was lyophilized to give compound **11** (100 mg) in 70.9% yield, LC-MS: [M/2+H]⁺ =855.86.

### Example 18:

### Synthesis of compounds 12A and 12B:

### 1. Preparation of compound 12A

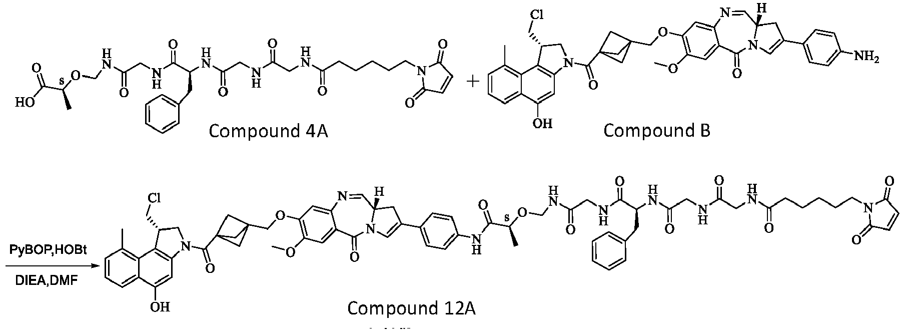

**Compound 4A** (51 mg, 0.081 mmol), **Compound B** (55.8 mg, 0.081 mmol), PyBOP (50.4 mg, 0.097 mmol), HOBt(13.1 mg, 0.097 mmol), and 5 mL of DMF were added to a 25 mL bottle, and DIPEA (15.5 mg, 0.12 mmol) was added to the bottle in an ice water bath. 0.12 mmol) in an ice water bath, and the reaction was brought to room temperature for 2 h. After the completion of the reaction was monitoredby HPLC, the reaction solution was purified by high performance liquid chromatography (HPLC) to obtain the preparative solution, which was lyophilized to obtain Compound **12A** (80 mg) in 76.2% yield, LC-MS: [M+H]⁺ =1301.5.

### 2. Preparation of compound 12B

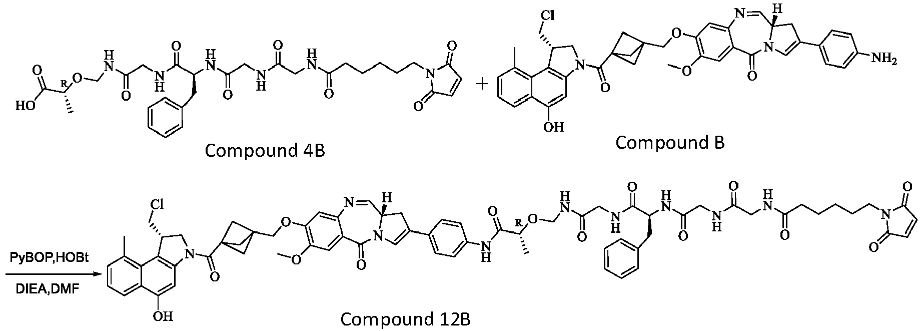

Referring to the synthesis of compound **12A**, compound **12B** was obtained, LC-MS: [M+H]⁺ =1301.5.

### Example 19:

### Synthesis of compounds 13A and 13B:

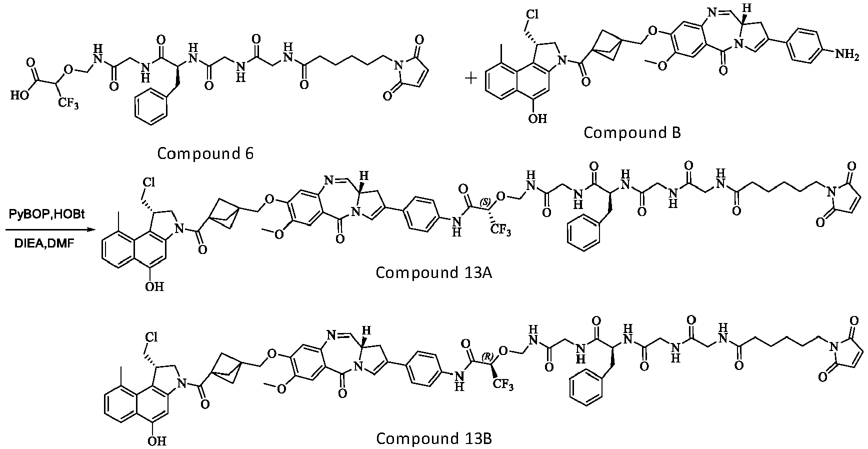

**Compound 6** (99.3 mg, 0.145 mmol), **Compound B** (100 mg, 0.145 mmol), PyBOP (90.5 mg, 0.174 mmol), HOBt (23.5 mg, 0.174 mmol), and 10 mL of DMF were added to a 50 mL bottle, and DIPEA (28.2 mg, 0.218 mmol) was added to the bottle in an ice-water bath. 0.218 mmol) under ice water bath, and the reaction was raised to room temperature for 2 h. After the completion of the reaction was monitored by HPLC, the reaction solution was purified by high performance liquid chromatography (HPLC) to obtain the preparative solution of compound **13A** and compound **13B**, and the preparative solution was lyophilized separately to obtain compound **13A** (90 mg), compound **13B** (80 mg), LC-MS: [M+H]⁺ =1355.5.

### Example 20:

### Synthesis of compound 14:

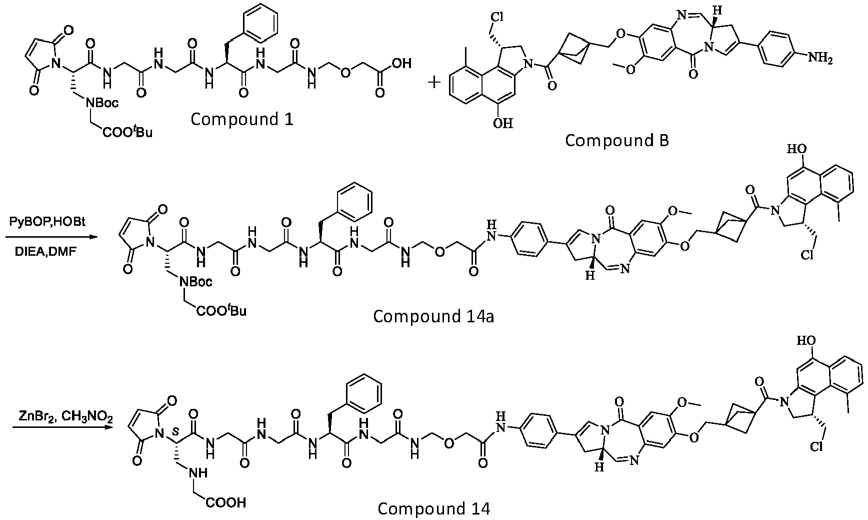

### Step 1: Synthesis of compound 14a

**Compound 1** (500 mg, 0.62 mmol), **Compound B** (427.3 mg, 0.62 mmol), PyBOP (385 mg, 0.74 mmol), HOBt (100 mg, 0.74 mmol) and 15 mL of DMF were added to a 50 mL bottle and DIPEA (120 mg, 0.93 mmol) was added to the bottle under an ice water bath, and the reaction was broughtto room temperature for 2 h. After the reaction was completed as monitored by HPLC, the reaction solution was purified by high performance liquid chromatography (HPLC) to obtain the preparative solution of compound 14a, which was lyophilized to obtain the solid compound 14a (550 mg) in 60% yield, LC-MS: [M+H]⁺ =1474.6.

### Step 2: Synthesis of compound 14

Compound 14a (200 mg, 0.136 mmol), zinc bromide (612 mg, 2.72 mmol) and 6 mL of nitromethane were added to a 25 mL bottle and reacted at room temperature for 1 h. After the reaction was completed as monitored by HPLC, the solvent was concentrated under reduced pressure to remove the solvent, and the crude product was obtained. The crude product was purified by high performance liquid phase purification with HPLC to obtain the product preparation solution, and the preparation solution was lyophilized to obtain solid compound **14** (100 mg) in 56% yield, LC-MS: [M+H]⁺ =1318.5.

### Example 21:

### Synthesis of compounds 15A and 15B:

### 1. Preparation of compound 15A

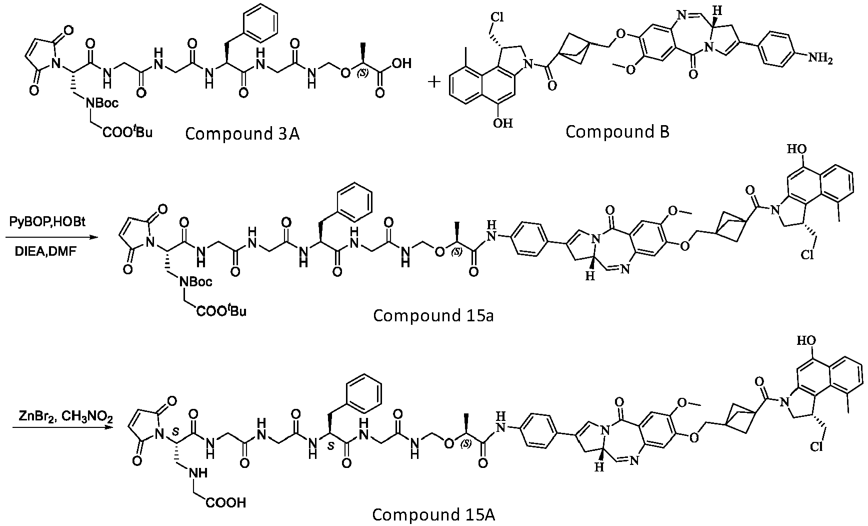

### Step 1: Synthesis of compound 15a

**Compound 3A** (507 mg, 0.62 mmol), **Compound B** (427.3 mg, 0.62 mmol), PyBOP (385 mg, 0.74 mmol), HOBt (100 mg, 0.74 mmol) and 15 mL of DMF were added to a 50 mL bottle, and DIPEA (120 mg, 0.93 mmol) was added to the bottle in an ice-water bath. The reaction was brought to room temperature for 2 h. After the reaction was completed as monitored by HPLC, the reaction solution was subjected to high performance liquid phase purification to obtain the preparative solution of compound **15a,** which was lyophilized to obtain the solid compound **15a** (650 mg) in 70% yield, LC-MS: [M+H]⁺ =1488.6.

### Step 2: Synthesis of compound 15A

Compound **15A** (200 mg, 0.134 mmol), zinc bromide (603.5 mg, 2.68 mmol) and 6 mL of nitromethane were added to a 25 mL bottle and reacted at room temperature for 1 h. After the reaction was completed as monitored by HPLC, the solvent was concentrated under reduced pressure to remove the solvent, and the crude product was obtained. The crude product was purified by high performance liquid phase purification with HPLC to obtain the product preparation solution, and the preparation solution was lyophilized to obtain solid compound **15A**(110 mg) in 62% yield, LC-MS: [M+H]⁺ =1332.5.

### 2. Preparation of compound 15B

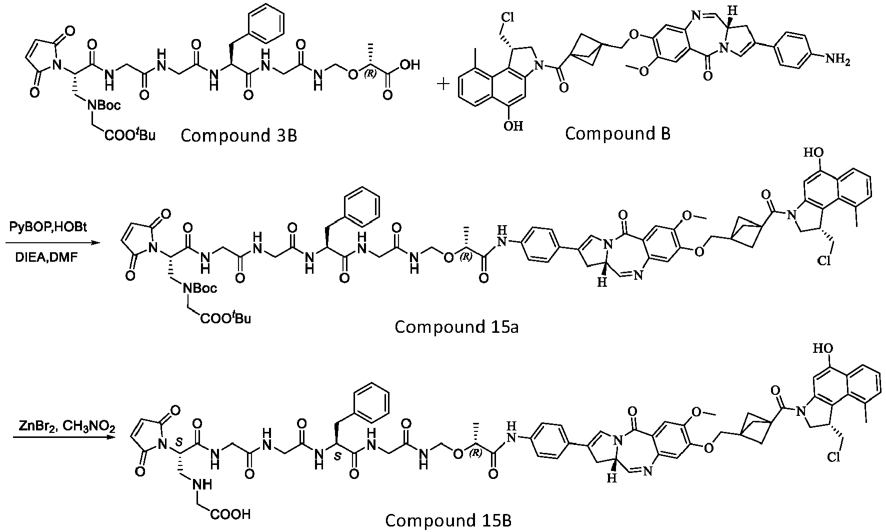

Referring to the synthesis of compound 15A, compound 15B was obtained, LC-MS: [M+H]⁺ =1332.5.

### Example 22:

### Synthesis of compounds 16A and 16B:

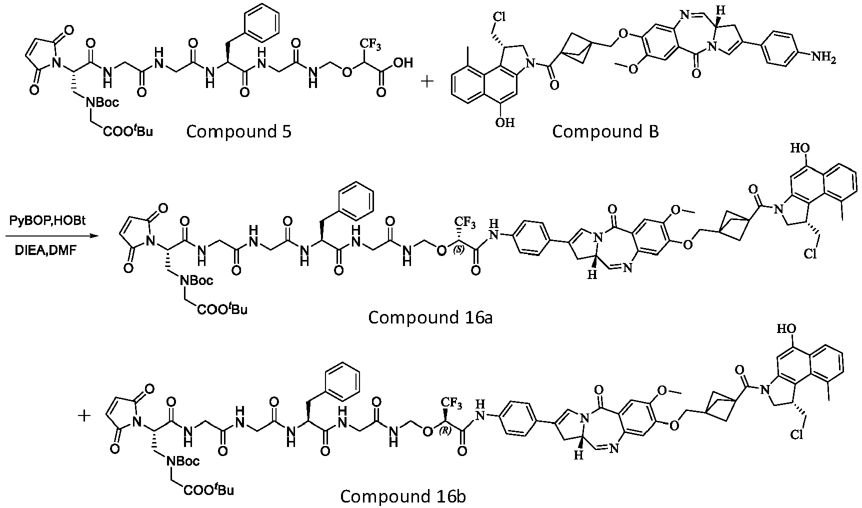

### Step 1: Synthesis of compounds 16a and 16b

**Compound 5** (540 mg, 0.62 mmol), **Compound B** (427.3 mg, 0.62 mmol), PyBOP (385 mg, 0.74 mmol), HOBt (100 mg, 0.74 mmol) and 15 mL of DMF were added to a 50 mL bottle and DIPEA (120 mg, 0.93 mmol) was added to the bottle in an ice-waterbath. DIPEA (120 mg, 0.93 mmol) was added in an ice-water bath, and the reaction was brought to room temperature for 2 h. After the reaction was completed by HPLC, the reaction solution was purified by high performance liquid phase purification with HPLC to obtain the preparative solutions of compound **16a** and compound **16b,** which were lyophilized to obtain compound **16a** (350 mg), compound **16b** (300 mg), respectively, and LC-MS: [M/2+H]⁺ =771.8.

### Step 2: Synthesis of compound 16A

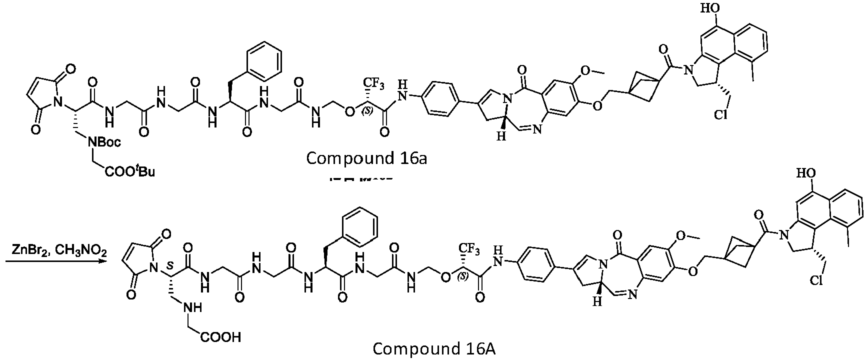

Compound **16A(200mg,** 0.13 mmol), zinc bromide (585.5 mg, 2.6 mmol) and 6 mL of nitromethane were added to a 25 mL bottle and reacted at room temperature for 1 h. After the reaction was completed as monitored by HPLC, the solvent was concentrated under reduced pressure to remove the solvent, and the crude product was obtained. The crude product was purified by high performance liquid phase purification with HPLC to obtain the product preparation solution, and the preparation solution was lyophilized to obtain solid compound **16A**(120 mg) in 66% yield, LC-MS: [M+H]⁺ =1386.4.

### Step 3: Synthesis of compound 16B

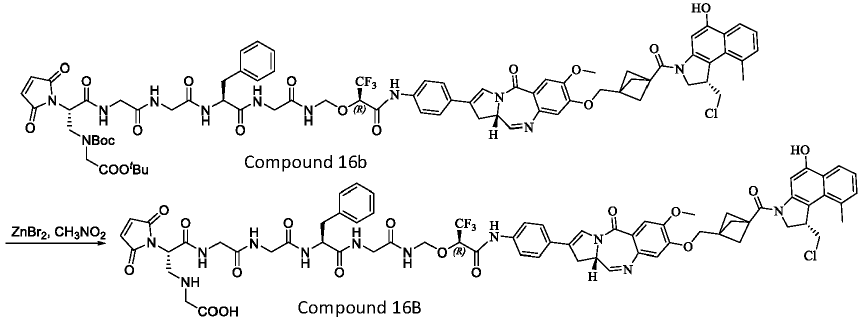

Compound **16B** (200 mg, 0.13 mmol), zinc bromide (585.5 mg, 2.6 mmol) and 6 mL of nitromethane were added to a 25 mL bottle and reacted at room temperature for 1 h. After the reaction was completed as monitored by HPLC, the solvent was concentrated under reduced pressure to remove the solvent, and the crude product was obtained. The crude product was purified by high performance liquid phase purification with HPLC to obtain the product preparation solution, and the preparation solution was lyophilized to obtain solid compound **16B** (115 mg) in 64% yield, LC-MS: [M+H]⁺ =1386.4.

### Example 23 :

### Synthesis of compounds 17A and 17B:

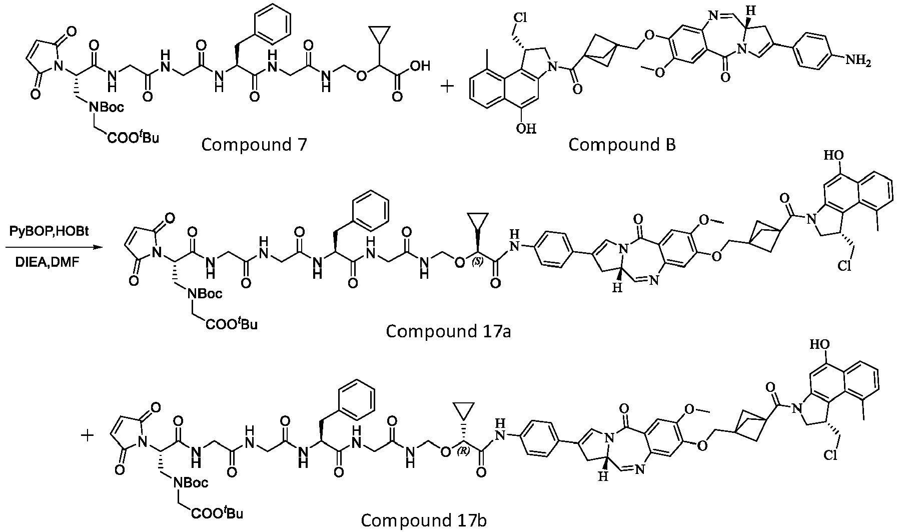

### Step 1: Synthesis of compounds 17a and 17b

**Compound 7** (523 mg, 0.62 mmol), **Compound B** (427.3 mg, 0.62 mmol), PyBOP (385 mg, 0.74 mmol), HOBt (100 mg, 0.74 mmol) and 15 mL of DMF were added to a 50 mL bottle and DIPEA (120 mg, 0.93 mmol) was added to the bottle in an ice bath. DIPEA(120 mg, 0.93 mmol) was added in an ice-water bath, and the reaction was brought to room temperature for 2 h. After the reaction was completed by HPLC, the reaction solution was purified by high performance liquid phase purification with HPLC to obtain the preparative solutions of compound **17a** and compound **17b,** which were lyophilized to obtain compound **17a** (320 mg), compound **17b** (280 mg), respectively, and LC-MS: [M/2+H]⁺ =757.8.

### Step 2: Synthesis of compound 17A

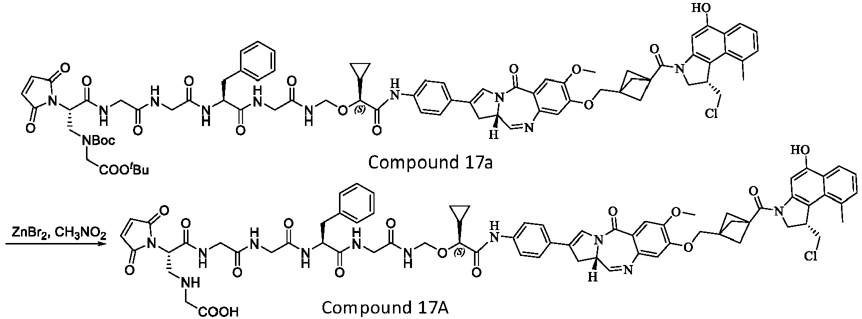

Compound **17A** (200 mg, 0.132 mmol), zinc bromide (594.5 mg, 2.64 mmol) and 6 mL of nitromethane were added to a 25 mL bottle and reacted at room temperature for 1 h. After the reaction was completed as monitored by HPLC, the solvent was concentrated under reduced pressure to remove the solvent, and the crude product was obtained. The crude product was purified by high performance liquid phase purification with HPLC to obtain the product preparation solution, and the preparation solution was lyophilized to obtain solid compound **17A** (120 mg) in 67% yield, LC-MS: [M+H]⁺ =1358.5.

### Step 3: Synthesis of compound 17B

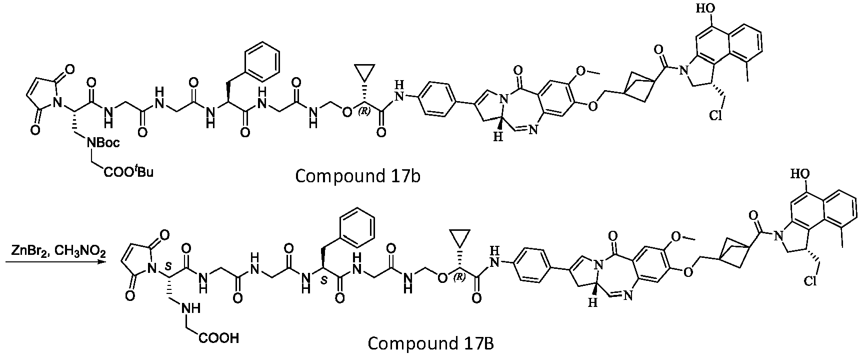

Compound **17B** (200 mg, 0.132 mmol), zinc bromide (594.5 mg, 2.64 mmol) and 6 mL of nitromethane were added to a 25 mL bottle and reacted at room temperature for 1 h. After the reaction was completed as monitored by HPLC, the solvent was concentrated under reduced pressure to remove the solvent, and the crude product was obtained. The crude product was purified by high performance liquid phase purification with HPLC to obtain the product preparation solution, and the preparation solution was lyophilized to obtain solid compound **17B** (116 mg) in 65% yield, LC-MS: [M+H]⁺ =1358.5.

### Example 24:

### Synthesis of compounds 18A and 18B:

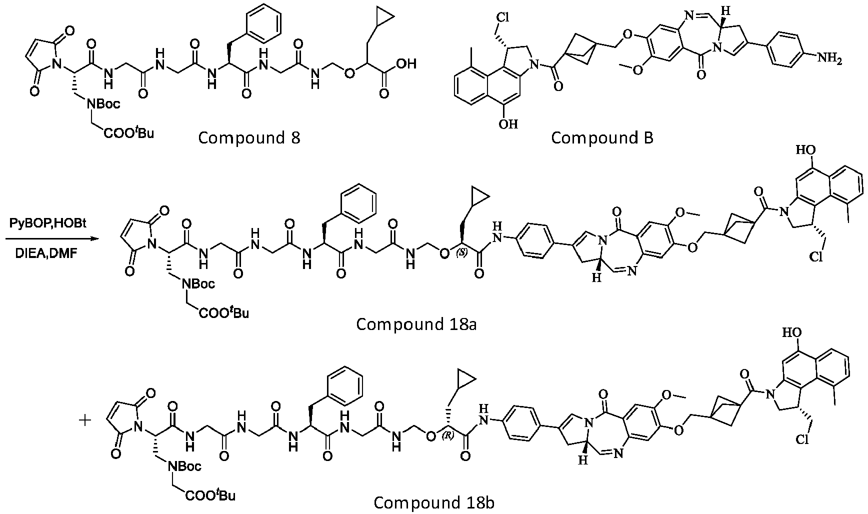

### Step 1: Synthesis of compounds 18a and 18b

**Compound 8** (532 mg, 0.62 mmol), **Compound B** (427.3 mg, 0.62 mmol), PyBOP (385 mg, 0.74 mmol), HOBt (100 mg, 0.74 mmol) and 15 mL of DMF were added to a 50 mL bottle and DIPEA (120 mg, 0.93 mmol) was added to the bottle in an ice bath. DIPEA(120 mg, 0.93 mmol) was added in an ice-water bath, and the reaction was brought to room temperature for 2 h. After the reaction was completed by HPLC, the reaction solution was purified by high performance liquid chromatography (HPLC) to obtain the preparative solutions of compound **18a** and compound **18b,** which were lyophilized to obtain compound **18a** (300 mg), compound **18b** (280 mg), respectively, and the LC-MS: [M/2+H]⁺ =764.8.

### Step 2: Synthesis of compound 18A

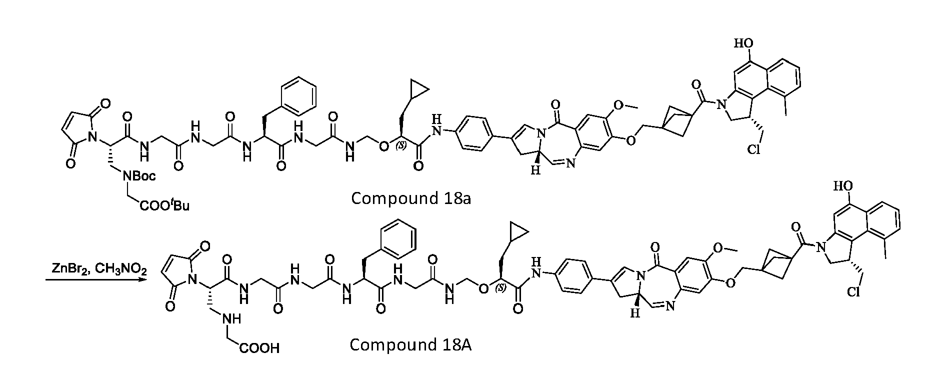

Compound **18a** (200 mg, 0.131 mmol), zinc bromide (590.0 mg, 2.62 mmol), and 6 mL of nitromethane were added to a 25 mL single-necked bottle, and the reaction was carried out at room temperature for 1 h. After the reaction was completed as monitored by HPLC, the solvent was concentrated under reduced pressure to remove the solvent, and the crude product was obtained. The crude product was purified by high performance liquid phase purification with HPLC to obtain the product preparation solution, and the preparation solution was lyophilized to obtain solid compound **18A** (125 mg) in 70% yield, LC-MS: [M+H]⁺ =1372.5.

### Step 3: Synthesis of compound 18B

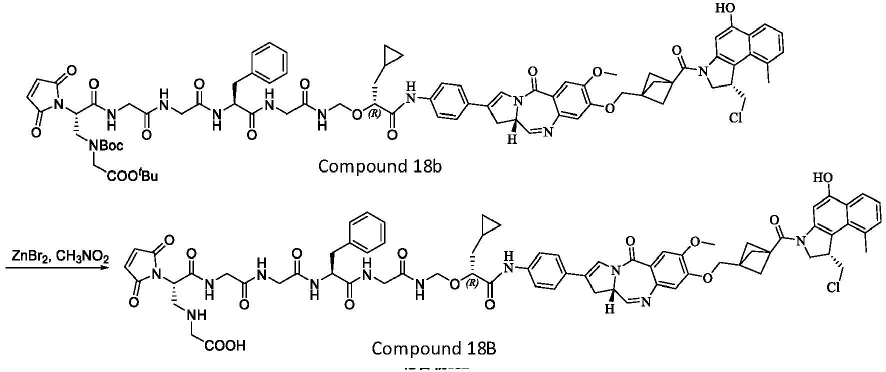

Compound **18B** (200 mg, 0.131 mmol), zinc bromide (590.0 mg, 2.62 mmol) and 6 mL of nitromethane were added to a 25 mL bottle and reacted at room temperature for 1 h. After the reaction was completed as monitored by HPLC, the solvent was concentrated under reduced pressure to remove the solvent, and the crude product was obtained. The crude product was purified by high performance liquid phase purification with HPLC to obtain the product preparation solution, and the preparation solution was lyophilized to obtain the solid compound **18B** (120 mg) in 67% yield, LC-MS: [M+H]⁺ =1372.5.

### Example 25:

### Synthesis of compound 19:

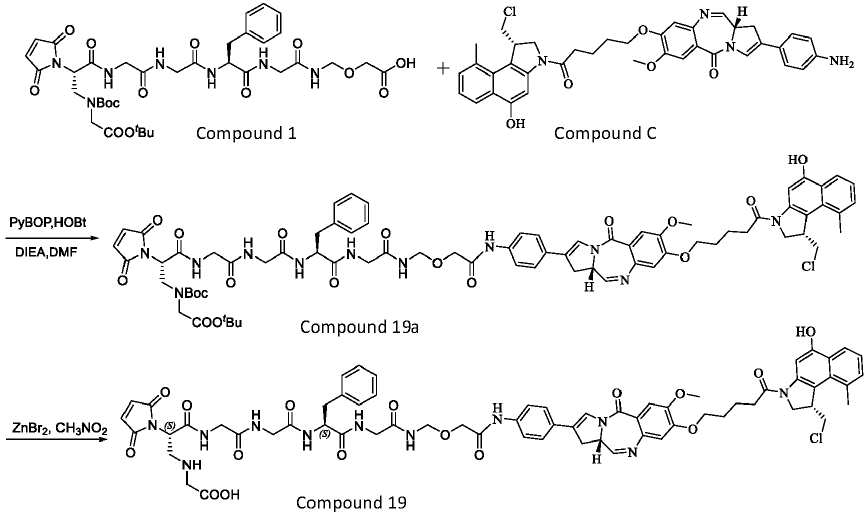

### Step 1: Synthesis of compound 19a

**Compound 1** (500 mg, 0.62 mmol), **Compound C** (412.4 mg, 0.62 mmol), PyBOP (385 mg, 0.74 mmol), HOBt (100 mg, 0.74 mmol) and 15 mL of DMF were added to a 50 mL bottle and DIPEA (120 mg, 0.93 mmol) was added to the bottle under an ice bath. The reaction was brought to room temperature for 2 h. After the reaction was completed as monitored by HPLC, the reaction solution was subjected to high performance liquid phase purification to obtain the preparative solution of compound **19a,** which was lyophilized to obtain the solid compound **19a** (600 mg) in 66% yield, LC-MS: [M+H]⁺ =1450.6.

### Step 2: Synthesis of compound 19

Compound **19a** (200 mg, 0.138 mmol), zinc bromide (621 mg, 2.76 mmol) and 6 mL of nitromethane were added to a 25 mL bottle and reacted at room temperature for 1 h. After the reaction was completed as monitored by HPLC, the solvent was removed by concentration under reduced pressure to obtain the crude product. The crude product was purified by high performance liquid phase purification with HPLC to obtain the product preparation solution, and the preparation solution was lyophilized to obtain solid compound **19** (120 mg) in 67% yield, LC-MS: [M+H]⁺ =1294.5.

### Example 26:

### Synthesis of Compound 20A and Compound 20B:

### 1. Preparation of compound 20A

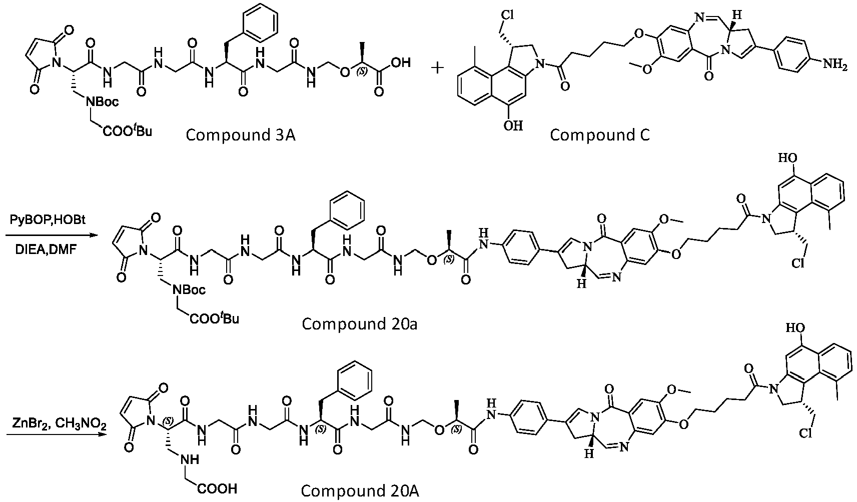

### Step 1: Synthesis of compound 20a

**Compound 3A** (507 mg, 0.62 mmol), **Compound C** (412.4 mg, 0.62 mmol), PyBOP (385 mg, 0.74 mmol), HOBt (100 mg, 0.74 mmol), and 15 mL of DMF were added into a 50 mL bottle, and DIPEA (120 mg, 0.93 mmol) was added into the bottle in an ice water bath. The reaction was broughtto room temperature for 2 h. After the reaction was completed as monitored by HPLC, the reaction solution was subjected to high performance liquid phase purification to obtain the preparative solution of compound**20a**, which was lyophilized to give the solid compound **20a** (560mg)in 62% yield, LC-MS: [M+H]⁺ =1464.6.

### Step 2: Synthesis of compound 20A

Compound **20A** (200 mg, 0.136 mmol), zinc bromide (614.8 mg, 2.73 mmol) and 6 mL of nitromethane were added to a 25 mL bottle and reacted at room temperature for 1 h. After the reaction was completed as monitored by HPLC, the solvent was concentrated under reduced pressure to remove the solvent, and the crude product was obtained. The crude product was purified by high performance liquid phase purification with HPLC to obtain the product preparation solution, and the preparation solution was lyophilized to obtain the solid compound **20A** (120 mg) in 67% yield, LC-MS: [M+H]⁺ =1308.5.

### 2. Preparation of compound 20B

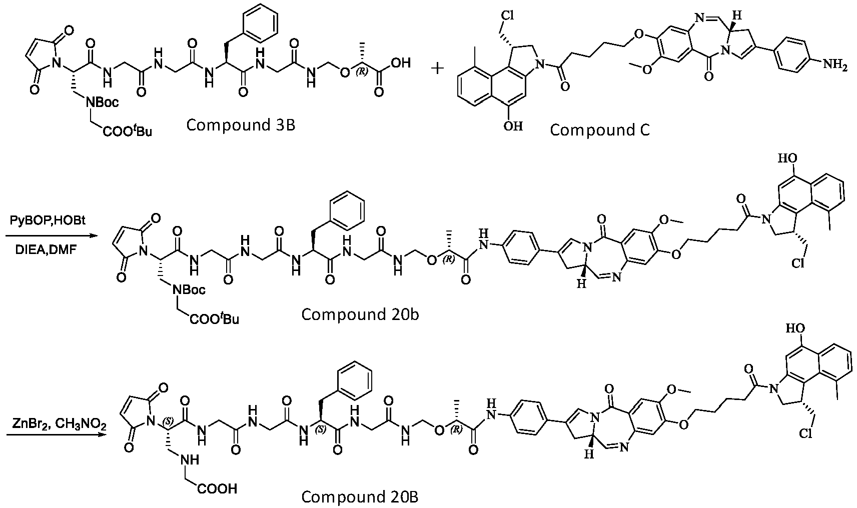

Referring to the synthesis of compound **20A,** compound **20B was obtained,** LC-MS: [M+H]⁺ =1308.5.

### Example 27:

### Synthesis of compounds 21A and 21B:

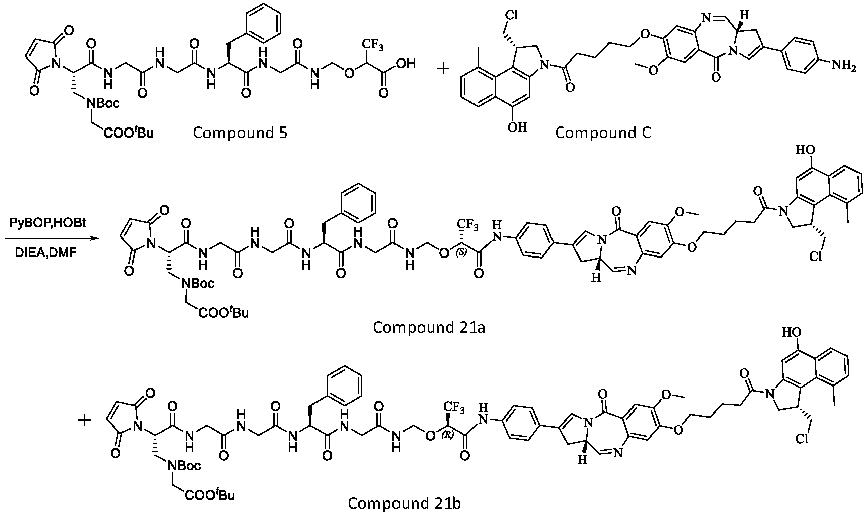

### Step 1: Synthesis of compounds 21a and 21b

**Compound 5** (540 mg, 0.62 mmol), **Compound C** (412.4 mg, 0.62 mmol), PyBOP (385 mg, 0.74 mmol), HOBt (100 mg, 0.74 mmol) and 15 mL of DMF were added to a 50 mL bottle and DIPEA (120 mg, 0.93 mmol) was added to the bottle in an ice-waterbath. The reaction was broughtto room temperature for2 h. After the reaction was completed by HPLC, the reaction solution was purified by high performance liquid phase purification with HPLC to obtain the preparative solutions of compound **21a** and compound **21b,** which were lyophilized to obtain compound **21a** (330 mg), compound **21b** (300 mg), respectively, and LC-MS: [M/2+H]⁺ =759.8.

### Step 2: Synthesis of compound 21A

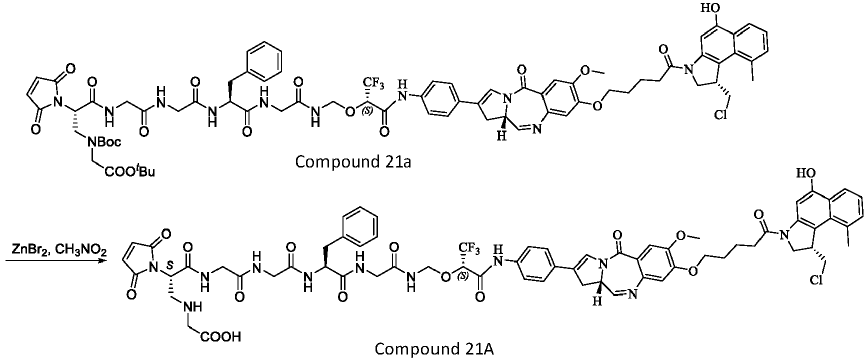

Compound **21A(200** mg, 0.132 mmol), zinc bromide (594.5 mg, 2.64 mmol) and 6 mL of nitromethane were added to a 25 mL bottle and reacted at room temperature for 1 h. After the reaction was completed as monitored by HPLC, the solvent was concentrated under reduced pressure to remove the solvent, and the crude product was obtained. The crude product was purified by high performance liquid phase purification with HPLC to obtain the product preparation solution, and the preparation solution was lyophilized to obtain solid compound **21A** (125 mg) in 69% yield, LC-MS: [M+H]⁺ =1362.4.

### Step 3: Synthesis of compound 21B

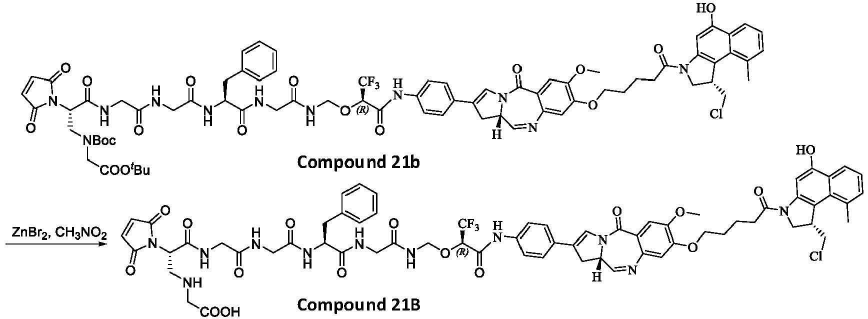

Compound **21B** (200 mg, 0.132 mmol), zinc bromide (594.5 mg, 2.64 mmol) and 6 mL of nitromethane were added to a 25 mL bottle and reacted at room temperature for 1 h. After the reaction was completed as monitored by HPLC, the solvent was concentrated under reduced pressure to remove the solvent, and the crude product was obtained. The crude product was purified by high performance liquid phase purification with HPLC to obtain the product preparation solution, and the preparation solution was lyophilized to obtain solid compound **21B** (112 mg) in 62% yield, LC-MS: [M+H]⁺ =1362.4.

### Example 28:

### Synthesis of compounds 22A and 22B:

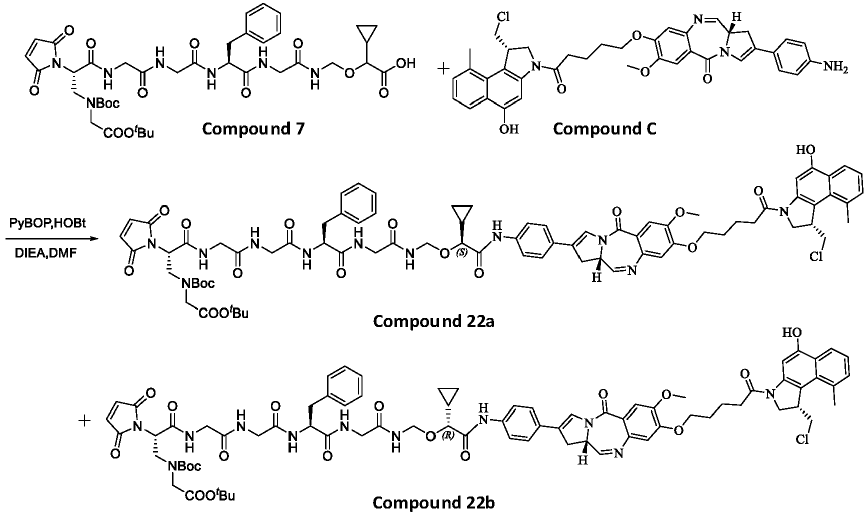

### Step 1: Synthesis of compounds 22a and 22b

**Compound 7** (523 mg, 0.62 mmol), **Compound C** (412.4 mg, 0.62 mmol), PyBOP (385 mg, 0.74 mmol), HOBt (100 mg, 0.74 mmol) and 15 mL of DMF were added to a 50 mL bottle and DIPEA (120 mg, 0.93 mmol) was added to the bottle in an ice-waterbath. The reaction was broughtto room temperature for2 h. After the reaction was completed by HPLC, the reaction solution was purified by high performance liquid phase purification with HPLC to obtain the preparative solutions of compound **22a** and compound **22b,** which were lyophilized to obtain compound **22a** (330 mg), compound **22b** (300 mg), respectively, and LC-MS: [M+H]⁺ =1490.6.

### Step 2: Synthesis of compound 22A

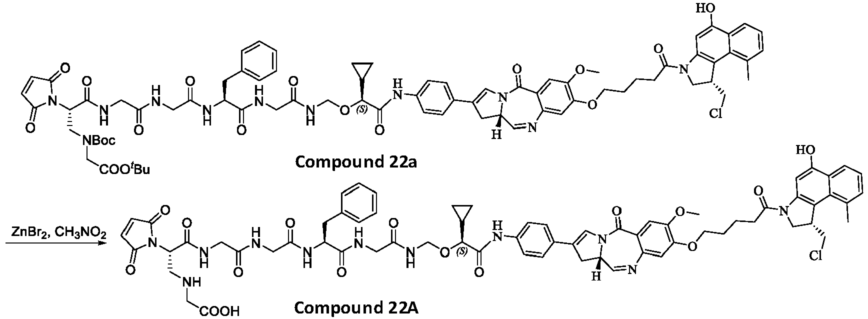

Compound **22A** (200 mg, 0.134 mmol), zinc bromide (603.5 mg, 2.68 mmol) and 6 mL of nitromethane were added to a 25 mL bottle and reacted at room temperature for 1 h. After the reaction was completed as monitored by HPLC, the solvent was concentrated under reduced pressure to remove the solvent, and the crude product was obtained. The crude product was purified by high performance liquid phase purification with HPLC to obtain the product preparation solution, and the preparation solution was lyophilized to obtain the solid compound **22A** (120 mg) in 67% yield, LC-MS: [M+H]⁺ =1334.5.

### Step 3: Synthesis of compound 22B

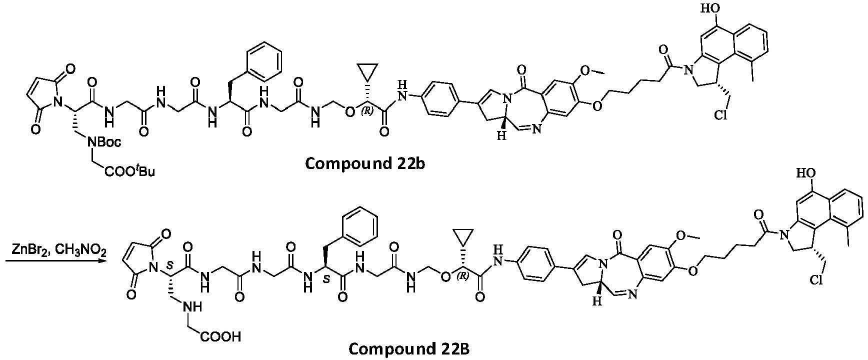

Compound **22B** (200 mg, 0.134 mmol), zinc bromide (603.5 mg, 2.68 mmol) and 6 mL of nitromethane were added to a 25 mL bottle and reacted at room temperature for 1 h. After the reaction was completed as monitored by HPLC, the solvent was concentrated under reduced pressure to remove the solvent, and the crude product was obtained. The crude product was purified by high performance liquid phase purification with HPLC to obtain the product preparation solution, and the preparation solution was lyophilized to obtain the solid compound **22B** (115 mg) in 65% yield, LC-MS: [M+H]+=1334.5.

### Example 29:

### Synthesis of compounds 23A and 23B:

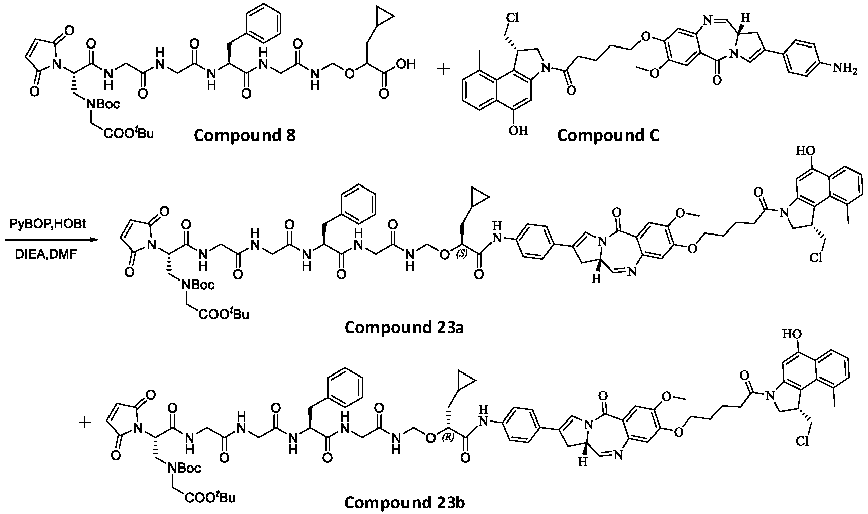

### Step 1: Synthesis of compounds 23a and 23b

**Compound 8** (532 mg, 0.62 mmol), **Compound C** (412.4 mg, 0.62 mmol), PyBOP (385 mg, 0.74 mmol), HOBt (100 mg, 0.74 mmol) and 15 mL of DMF were added to a 50 mL bottle and DIPEA (120 mg, 0.93 mmol) was added to the bottle in an ice-waterbath. The reaction was broughtto room temperature for2 h. After the reaction was completed by HPLC, the reaction solution was purified by high performance liquid phase purification with HPLC to obtain the preparative solutions of compound **23a** and compound **23b,** which were lyophilized to obtain compound **23a** (320 mg), compound **23b** (300 mg), respectively, and LC-MS: [M/2+H]⁺ =752.8.

### Step 2: Synthesis of compound 23A

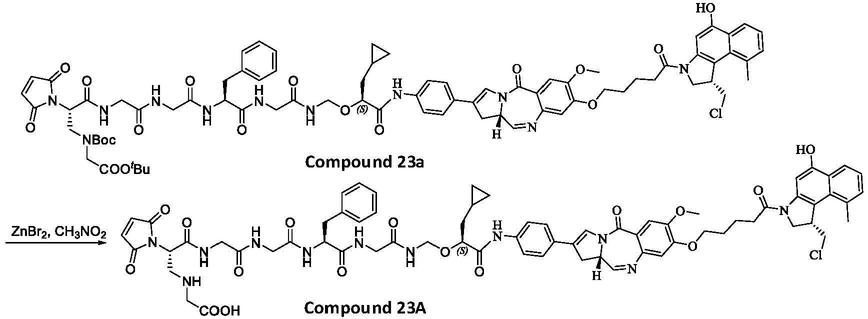

Compound **23A** (200 mg, 0.133 mmol), zinc bromide (598.5 mg, 2.66 mmol) and 6 mL of nitromethane were added to a 25 mL bottle and the reaction was carried out at room temperature for 1 h. After the reaction was completed as monitored by HPLC, the solvent was concentrated under reduced pressure to remove the solvent, and the crude product was obtained. The crude product was purified by high performance liquid phase purification with HPLC to obtain the product preparation solution, and the preparation solution was lyophilized to obtain solid compound **23A** (122 mg) in 68%yield, LC-MS: [M+H]⁺ =1348.5.

### Step 3: Synthesis of compound 23B

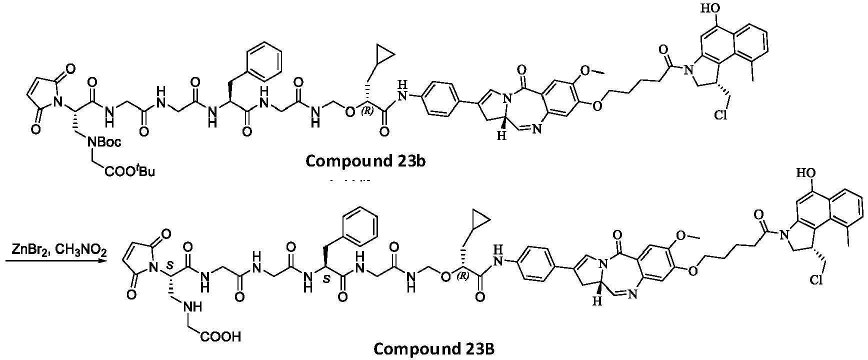

Compound **23B** (200 mg, 0.133 mmol), zinc bromide (598.5 mg, 2.66 mmol) and 6 mL of nitromethane were added to a 25 mL bottle and reacted at room temperature for 1 h. After the reaction was completed as monitored by HPLC, the solvent was concentrated under reduced pressure to remove the solvent, and the crude product was obtained. The crude product was purified by high performance liquid phase purification with HPLC to obtain the product preparation solution, and the preparation solution was lyophilized to obtain solid compound **23B** (115 mg) in 64% yield, LC-MS: [M+H]⁺ =1348.5.

### Example 30:

### Synthesis of compound 24:

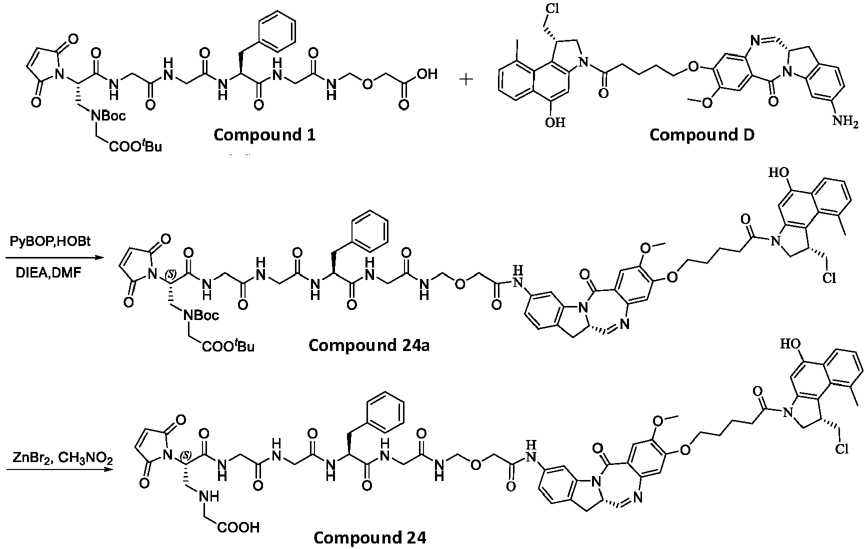

### Step 1: Synthesis of compound 24a

**Compound 1** (500 mg, 0.62 mmol), **Compound D** (396.3 mg, 0.62 mmol), PyBOP (385 mg, 0.74 mmol), HOBt (100 mg, 0.74 mmol) and 15 mL of DMF were added to a 50 mL bottle and DIPEA (120 mg, 0.93 mmol) was added to the bottle in an ice waterbath. The reaction was broughtto room temperature for 2 h. After the reaction was completed as monitored by HPLC, the reaction solution was subjected to high performance liquid phase purification to obtain the preparative solution of compound **24a,** which was lyophilized to obtain the solid compound **24a** (600 mg) in 68% yield, LC-MS: [M+H]⁺ =1424.6.

### Step 2: Synthesis of compound 24

Compound **24a** (200 mg, 0.14 mmol), zinc bromide (631 mg, 2.8 mmol) and 6 mL of nitromethane were added to a 25 mL bottle and reacted at room temperature for 1 h. After the reaction was completed as monitored by HPLC, the solvent was removed by concentration under reduced pressure to obtain the crude product. The crude product was purified by high performance liquid phase purification with HPLC to obtain the product preparation solution, and the preparation solution was lyophilized to obtain solid compound **24** (120 mg) in 68% yield, LC-MS: [M+H]⁺ =1268.4.

### Example 31:

### Synthesis of compound 25A and compound 25B:

### 1. Preparation of compound 25A

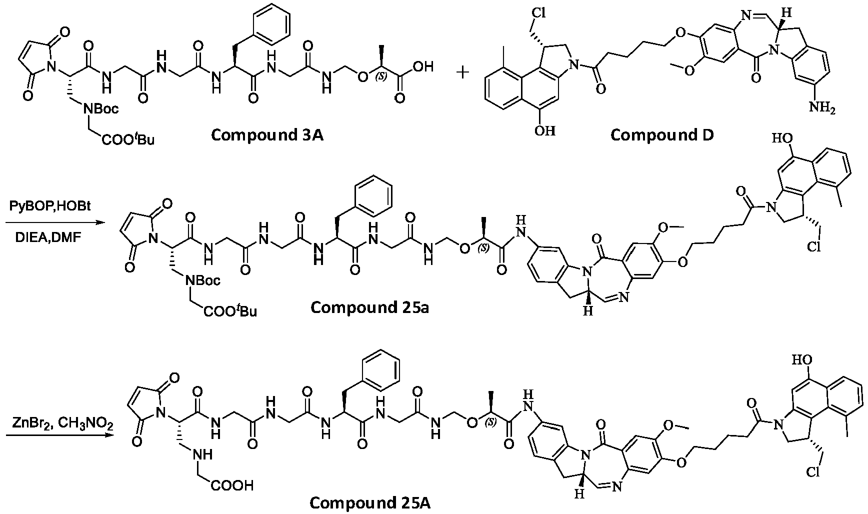

### Step 1: Synthesis of compound 25a

**Compound 3A** (507 mg, 0.62 mmol), **Compound D** (396.3 mg, 0.62 mmol), PyBOP (385 mg, 0.74 mmol), HOBt (100 mg, 0.74 mmol) and 15 mL of DMF were added to a 50 mL bottle and DIPEA (120 mg, 0.93 mmol) was added to the bottle in an ice-waterbath. The reaction was broughtto room temperature for2 h. After the reaction was completed as monitored by HPLC, the reaction solution was purified by high performance liquid chromatography (HPLC) to obtain the preparative solution of compound **25a,** which was lyophilized to obtain compound **25a** (560 mg) as a solid compound in 63% yield, LC-MS: [M+H]⁺ =1438.6.

### Step 2: Synthesis of compound 25A

Compound **25A** (200 mg, 0.139 mmol), zinc bromide (626 mg, 2.78 mmol) and 6 mL of nitromethane were added to a 25 mL bottle and reacted at room temperature for 1 h. After the reaction was completed as monitored by HPLC, the solvent was concentrated under reduced pressure to remove the solvent, and the crude product was obtained. The crude product was purified by high performance liquid phase purification with HPLC to obtain the product preparation solution, and the preparation solution was lyophilized to obtain the solid compound **25A** (115 mg) in 65% yield, LC-MS: [M+H]+=1282.5.

### 2. Preparation of compound 25B

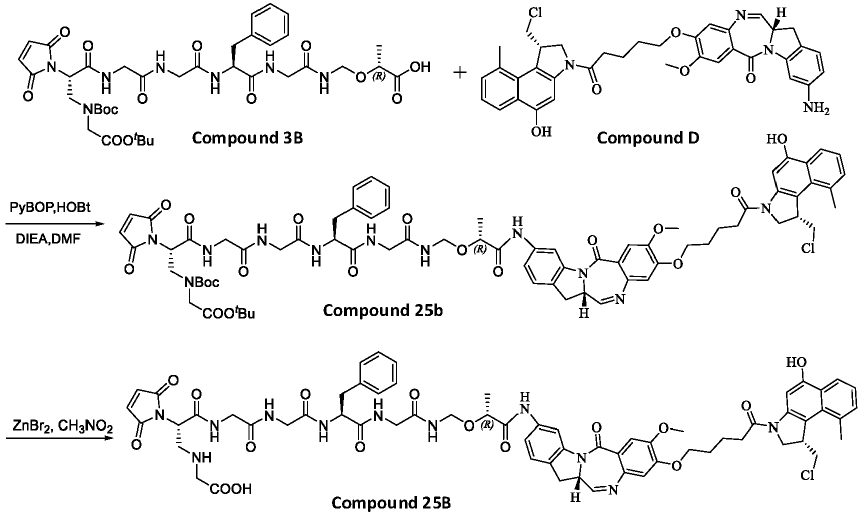

Referring to the synthesis of compound **25A,** compound **26B was obtained,** LC-MS: [M+H]+=1282.5.

### Example 32:

### Synthesis of compounds 26A and 26B:

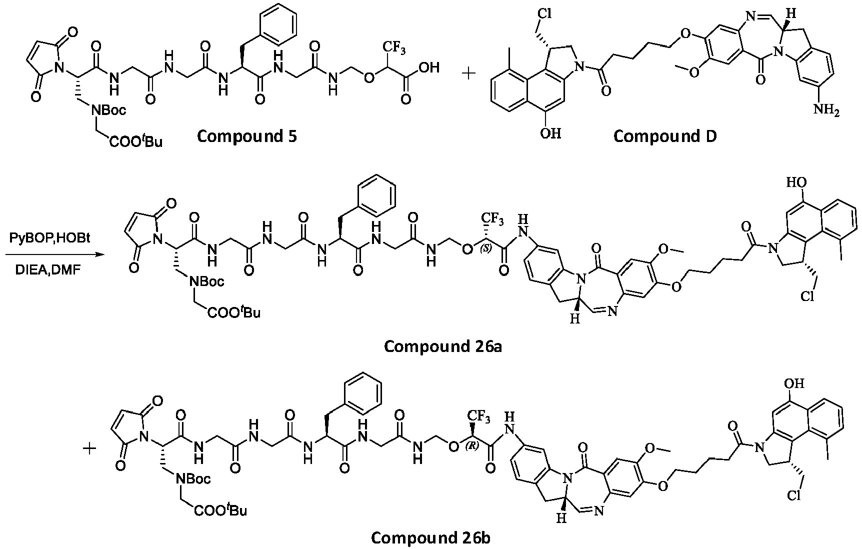

### Step 1: Synthesis of compounds 26a and 26b

**Compound 5** (540 mg, 0.62 mmol), **Compound D** (396.3 mg, 0.62 mmol), PyBOP (385 mg, 0.74 mmol), HOBt (100 mg, 0.74 mmol) and 15 mL of DMF were added to a 50 mL bottle and DIPEA (120 mg, 0.93 mmol) was added to the bottle in an ice-waterbath. The reaction was broughtto room temperature for2 h. After the reaction was completed by HPLC, the reaction solution was purified by high performance liquid phase purification with HPLC to obtain the preparative solutions of compound **26a** and compound **26b,** which were lyophilized to obtain compound **26a** (330 mg), compound **26b** (300 mg), respectively, and LC-MS: [M+H]⁺ =1492.5.

### Step 2: Synthesis of compound 26A

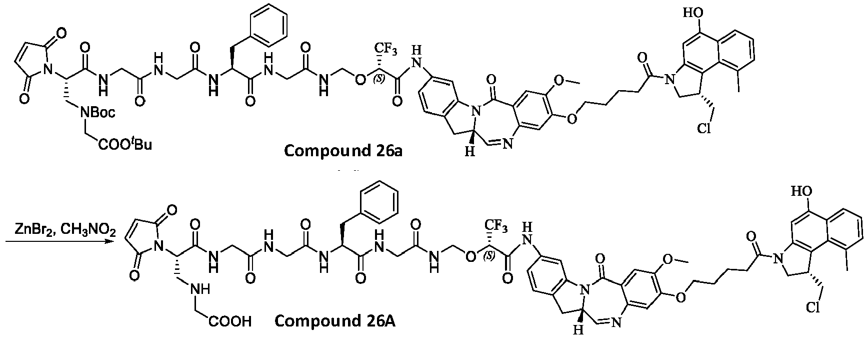

Compound **26A** (200 mg, 0.134 mmol), zinc bromide (603.5 mg, 2.68 mmol) and 6 mL of nitromethane were added to a 25 mL bottle and reacted at room temperature for 1 h. After the reaction was completed as monitored by HPLC, the solvent was concentrated under reduced pressure to remove the solvent, and the crude product was obtained. The crude product was purified by high performance liquid phase purification with HPLC to obtain the product preparation solution, and the preparation solution was lyophilized to obtain the solid compound **26A** (125 mg) in 70% yield, LC-MS: [M+H]⁺ =1336.4.

### Step 3: Synthesis of compound 26B

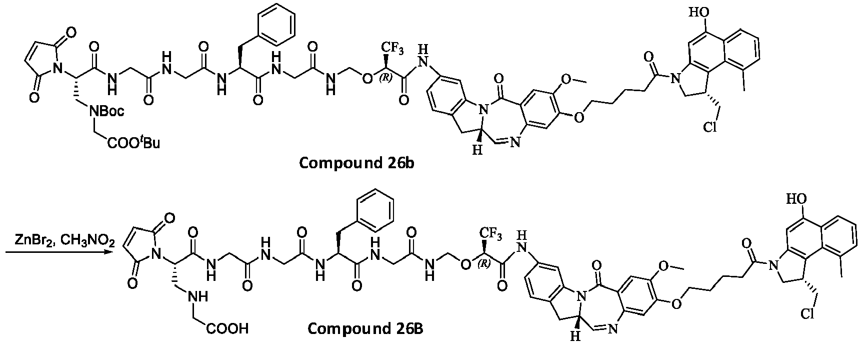

Compound **26B** (200 mg, 0.134 mmol), zinc bromide (603.5 mg, 2.68 mmol) and 6 mL of nitromethane were added to a 25 mL bottle and reacted at room temperature for 1 h. After the reaction was completed as monitored by HPLC, the solvent was concentrated under reduced pressure to remove the solvent, and the crude product was obtained. The crude product was purified by high performance liquid phase purification with HPLC to obtain the product preparation solution, and the preparation solution was lyophilized to obtain solid compound **26B** (112 mg) in 63% yield, LC-MS: [M+H]⁺ =1336.4.

### Example 33:

### Synthesis of compounds 27A and 27B:

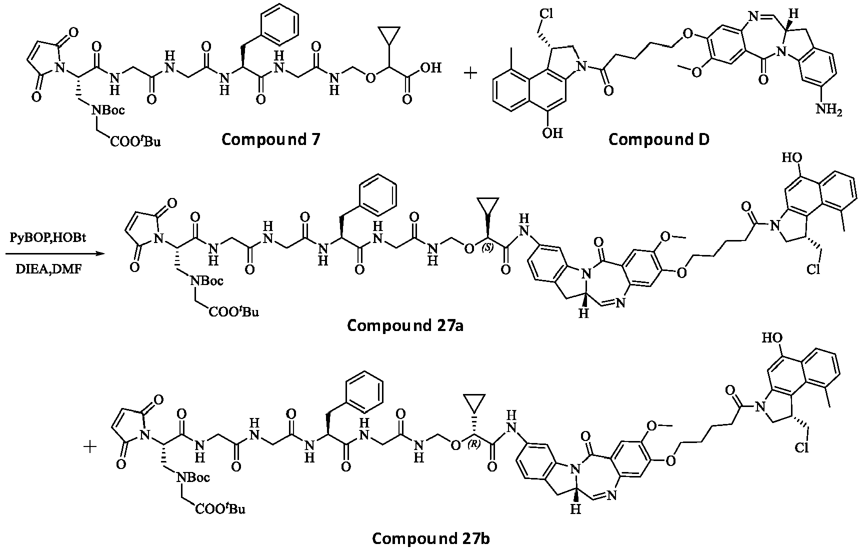

### Step 1: Synthesis of compounds 27a and 27b

**Compound 7** (523 mg, 0.62 mmol), **Compound D** (396.3 mg, 0.62 mmol), PyBOP (385 mg, 0.74 mmol), HOBt (100 mg, 0.74 mmol) and 15 mL of DMF were added to a 50 mL bottle and DIPEA (120 mg, 0.93 mmol) was added to the bottle in an ice-waterbath. The reaction was broughtto room temperature for2 h. After the reaction was completed by HPLC, the reaction solution was purified by high performance liquid phase purification with HPLC to obtain the preparative solutions of compound **27a** and compound **27b,** which were lyophilized to obtain compound **27a** (330 mg), compound **27b** (300 mg), respectively, and LC-MS: [M+H]⁺ =1464.6.

### Step 2: Synthesis of compound 27A

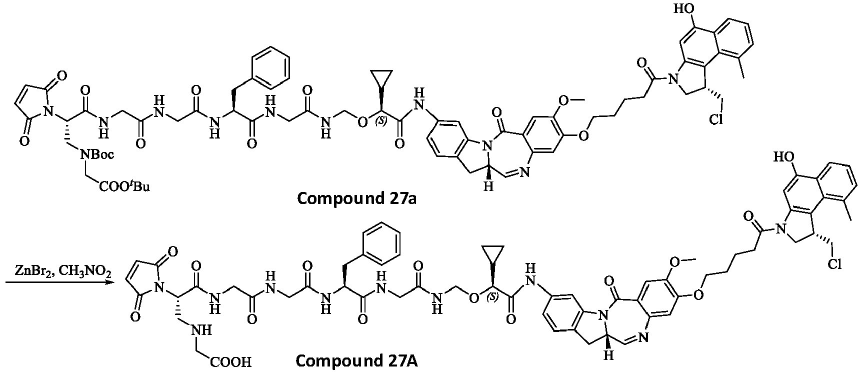

Compound **27A** (200mg, 0.136 mmol), zinc bromide (614.8 mg, 2.73 mmol) and 6 mL of nitromethane were added to a 25 mL bottle and reacted at room temperature for 1 h. After the reaction was completed as monitored by HPLC, the solvent was concentrated under reduced pressure to remove the solvent, and the crude product was obtained. The crude product was purified by high performance liquid phase purification with HPLC to obtain the product preparation solution, and the preparation solution was lyophilized to obtain the solid compound **27A** (125 mg) in 70% yield, LC-MS: [M+H]⁺ =1308.5.

### Step 3: Synthesis of compound 27B

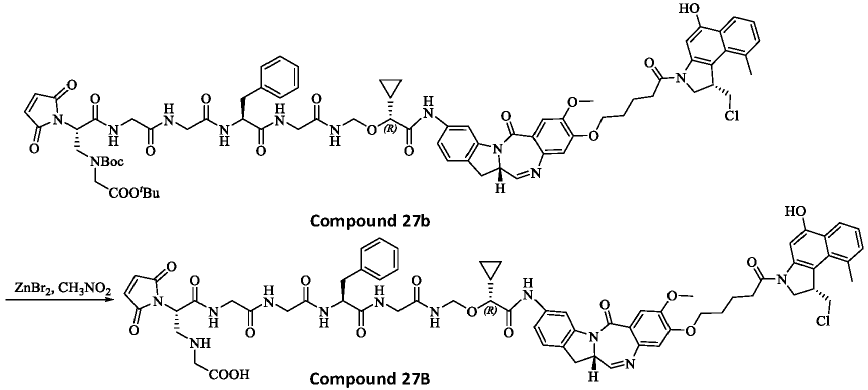

Compound **27B** (200 mg, 0.136 mmol), zinc bromide (614.8 mg, 2.73 mmol) and 6 mL of nitromethane were added to a 25 mL bottle and reacted at room temperature for 1 h. After the reaction was completed as monitored by HPLC, the solvent was concentrated under reduced pressure to remove the solvent, and the crude product was obtained. The crude product was purified by high performance liquid phase purification with HPLC to obtain the product preparation solution, and the preparation solution was lyophilized to obtain solid compound **27B** (115 mg) in 64% yield, LC-MS: [M+H]⁺ = 1308.5.

### Example 34:

### Synthesis of compounds 28A and 28B:

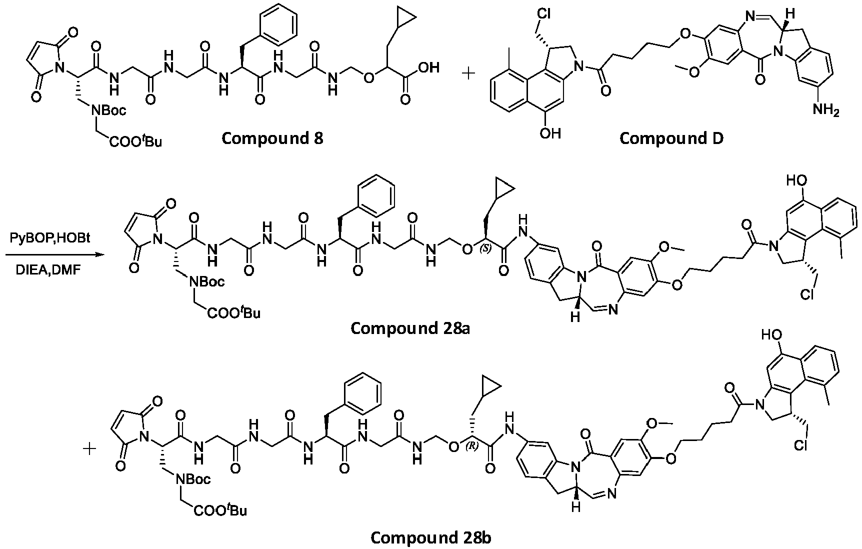

### Step 1: Synthesis of compounds 28a and 28b

**Compound 8** (532 mg, 0.62 mmol), **Compound D** (396.3 mg, 0.62 mmol), PyBOP (385 mg, 0.74 mmol), HOBt (100 mg, 0.74 mmol) and 15 mL of DMF were added to a 50 mL bottle and DIPEA (120 mg, 0.93 mmol) was added to the bottle in an ice-waterbath. The reaction was broughtto room temperature for2 h. After the reaction was completed by HPLC, the reaction solution was purified by high performance liquid chromatography (HPLC) to obtain the preparative solutions of compound **28a** and compound **28b,** which were lyophilized to obtain compound **28a** (320 mg), compound **28b** (300 mg), respectively, and the LC-MS: [M+H]⁺ =1478.6.

### Step 2: Synthesis of compound 28A

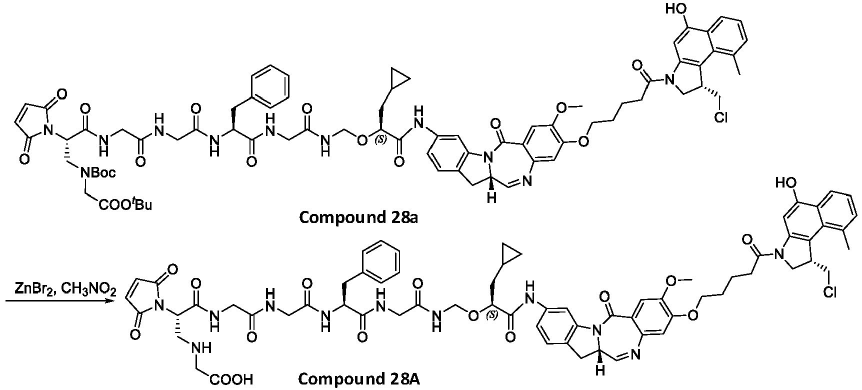

Compound **28A** (200 mg, 0.135 mmol), zinc bromide (608 mg, 2.7 mmol) and 6 mL of nitromethane were added to a 25 mL bottle and reacted at room temperature for 1 h. After the reaction was completed as monitored by HPLC, the solvent was removed by concentration under reduced pressure to obtain the crude product. The crude product was purified by high performance liquid phase purification with HPLC to obtain the product preparation solution, and the preparation solution was lyophilized to obtain the solid compound **28A** (121 mg) in 68% yield, LC-MS: [M+H]⁺ =1322.5.

### Step 3: Synthesis of compound 28B

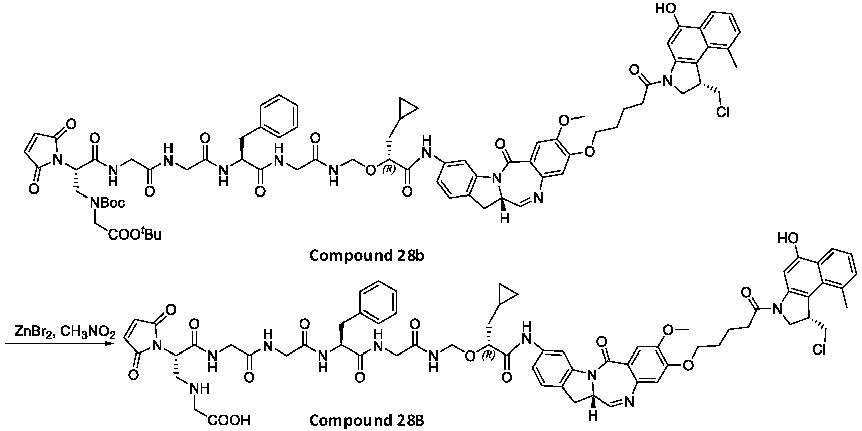

Compound **28B** (200 mg, 0.135 mmol), zinc bromide (608 mg, 2.7 mmol) and 6 mL of nitromethane were added to a 25 mL bottle and reacted at room temperature for 1 h. After the reaction was completed as monitored by HPLC, the solvent was removed by concentration under reduced pressure to obtain the crude product. The crude product was purified by high performance liquid phase purification with HPLC to obtain the product preparation solution, and the preparation solution was lyophilized to obtain solid compound **28B** (115 mg) in 64% yield, LC-MS: [M+H]⁺ =1322.5.

### Example 34:

### Compound 29 synthesized:

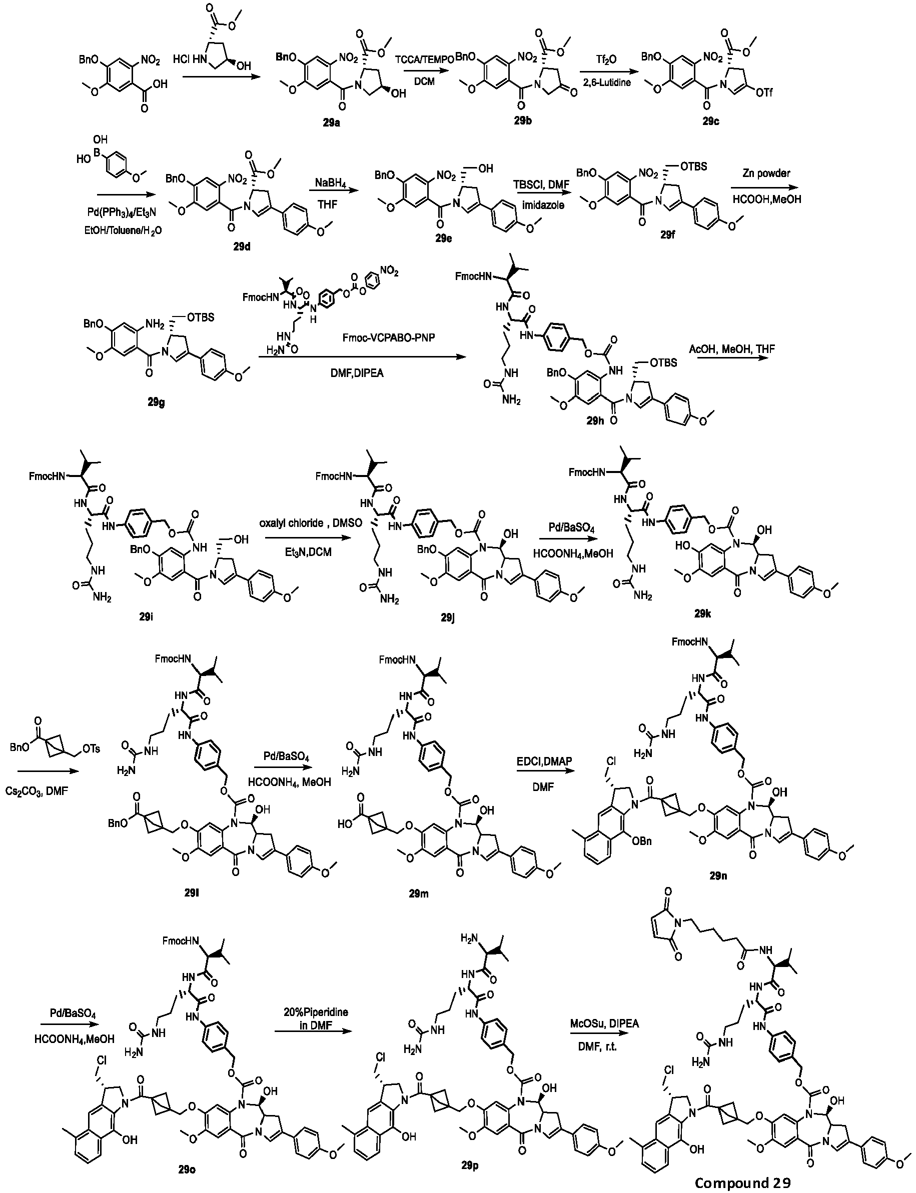

### Step 1: Synthesis of compounds 29a to 29d

Compound **29d** was obtained by the synthesis of "Compound **26"** in Patent CN102933236A.

### Step 2: Synthesis of compound 29e

In a 250 mL triple-necked flask, Compound **29d** (3.0 g, 5.8 mmol), 50 mL of anhydrous THF under nitrogen protection and an ice-waterbath maintained at 0 °C was added, sodium borohydride solid (329 mg, 8.7 mmol) was added, the reaction was carried out at 0 °C for 30 min, and then the reaction was raised to room temperature for 2 h. The end point of the reaction was monitored by TLC. At the end of the reaction, the reaction solution was transferred to ice water, the reaction was quenched by slowly adding 50 mL of water, then 1N dilute HCl was added until no bubbles were formed, ethyl acetate was added to extract (60 mL*3), the organic phases were combined, washed twice with saturated sodium chloride solution, dried with anhydrous sodium sulfate, filtered, and concentrated to obtain the crude product. The crude product was purified by column chromatography (DCM/MeOH=20:1-5:1) to yield compound **29e** (2.1 g, yellow solid) in 73.8% yield, LCMS: [M+H]⁺ =491.2.

### Step 3: Synthesis of compound 29f

Compound **29e** (2.1g, 4.3mmol), TBSCl (1.3g, 8.6mmol), imidazole (580mg, 8.6mmol) were added to a 100mL bottle and dissolved in 15mL of anhydrous DMF and the reaction was carried out for 3h at room temperature and the endpointwas monitored by TLC. At the end of the reaction, the reaction was poured into 30 mL of water and extracted with DCM (25 mL*3), the organic phases were combined, washed with water and brine, dried over anhydrous sodium sulfate and evaporated under reduced pressure to obtain the crude product. The crude product was subjected to column chromatography (PE/EA=5 :1-1:2) to yield compound**29f** (2.4 g, yellow solid) in 89.3% yield, LCMS: [M+H]⁺ =605.3.

### Step 4: Synthesis of compound 29g

Compound **29f** (2.4g, 4.0mmol), 5% formic acid/methanol solution 30mL was added in a 100mL single-necked bottle, cooled to below 5°C in an ice bath, and zinc powder (5.2g, 80.0mmol) was added slowly with stirring, and then the reaction was raised to room temperature for 1h, and the endpoint of the reaction was monitored by TLC. At the end of the reaction, filtered while hot, the filter cake was washed with a small amount of methanol, the filtrate was adjusted to pH 7 with saturated sodium bicarbonate solution, and then concentrated under reduced pressure at 45°C to remove the solvent to obtain a brownish-yellow oily material, add 60mL DCM, separate the organic layer, washed with saturated brine once, anhydrous sodium sulfate drying, filtration, and concentrated under reduced pressure at 45°C to obtain the crude product. The crude product was purified by column chromatography (PE:EA=1:1-1:3) to obtain the compound **29g** (1.9g, light yellow solid) in 82.5% yield, LCMS: [M+H]⁺ = 575.3.

### Step 5: Synthesis of compound 29h

Compound **29g** (1.9g, 3.3mmol), Fmoc-VCPABO-PNP (2.5g, 3.3mmol),15mL DMF solubilized in a 50mL single-necked bottle, then DIPEA(827uL, 5.0mmol) was added, and reaction was carried out at room temperature for 2h, and the end point was monitored by HPLC. At the end of the reaction, the reaction solution was purified by high performance liquid phase preparation to obtain the product preparation, and then lyophilized to obtain compound **29h** (3.3g, light yellow solid) in 83.1% yield, LCMS: [M+H]⁺ =1202.5.

### Step 6: Synthesis of compound 29i

Compound **29h** (3.3 g, 2.7 mmol) was added to a 50 mL single-necked bottle, dissolved in 8 mL of THF and 8 mL of water, and 20 mL of glacial acetic acid was added to the reaction at room temperature, and the endpoint of the reaction was monitored by HPLC. At the end of the reaction, the reaction solution was slowly dripped into 400 mL of saturated sodium bicarbonate solution, extracted with ethyl acetate (100 mL*3), the organic phases were combined, washed with water and brine, dried with anhydrous sodium sulfate and evaporated under reduced pressure to obtain the crude product. The crude product was subjected to column chromatography (DCM/MeOH=10:1-5:1) to obtain compound **29i** (2.1 g, light yellow solid) in 71.1% yield, LCMS: [M+H]⁺ =1088.5.

### Step 7: Synthesis of compound 29j

Compound **29i** (2.1 g, 1.9 mmol) was added to a 100 mL three-necked bottle, 40 mL of anhydrous DCM was dissolved, and Dess-Martin High Iodine Reagent (0.88 g, 2.1 mmol) was added under the protection of nitrogen, the reaction was carried out at room temperature for 4h, and the end point was monitored by HPLC. After the reaction, the filtrate was filtered, and the filtrate was washed by saturated sodium bicarbonate solution, water, saturated sodium chloride solution, dried with anhydrous sodium sulfate, and concentrated to obtain the crude product. The crude product was purified by high performance liquid phase purification with HPLC, and the product preparation was lyophilized to yield compound **29j** (1.6 g, white solid) in 77.6% yield, LCMS: [M+H]⁺ =1086.5.

### Step 8: Synthesis of compound 29k

Add compound **29j** (1.6g, 1.5mmol), 5% palladium barium sulfate (0.29g), ammonium formate (1.9g, 30mmol) in a 50mL triple-necked bottle, dissolve in 20mL methanol, andreactat45 ° C for 2h under nitrogen protection, andmonitorthe endpoint of the reaction by HPLC. After the end of the reaction, filtration, the filtrate was concentrated, 20mLDCM, 20mL water was added and stirred well and partitioned, the aqueous layer was extracted again with DCM, the organic layers were combined, washed once with saturated aqueous sodium chloride, partitioned, dried with anhydrous sodium sulfate, and concentrated to obtain the crude product. The crude product was purified by high performance liquid phase purification with HPLC to obtain the product preparation solution, lyophilized to obtain compound **29k** (1.3g, off-white solid), yield 93.3%, LCMS: [M+H]⁺ =996.4.

### Step 9: Synthesis of compound 291

Add benzyl 3-((p-toluenesulfonyloxy)methyl)bicyclo[1 .1 .1]pentane-1-carboxylate (0.61g,1.2mmol), compound **29k** (1.3g, 1.3mmol) and 20mL of DMF in a 100mL single-necked bottle, stir to dissolve, then add cesium carbonate (0.78g,2.4mmol), add, and raise the temperature under the protection of nitrogen to 60°C. The reaction was carried out for 4h, and the end point was monitored by TLC. Add 40mL of water to the reaction solution, extract with 30mL EA for 2 times, combine the organic layers, wash with water and saturated saline once each in turn, separate the organic layer, anhydrous sodium sulfate drying, filtration, and concentration under reduced pressure at 45 °C to obtain the crude product, which was purified by high performance liquid chromatography to obtain the product preparation, lyophilized to obtain the compound **291** (1.2g, off-white solid), the yield of 84.0%, LCMS: [M+H]⁺ =1210.5.

### Step 10: Synthesis of compound 29m

Add compound **291** (1.2g, 1.0mmol), 5% palladium barium sulfate (0.24g), ammonium formate (1.3g, 20mmol) in a 50mL triple-necked bottle, dissolve in 20mL methanol, and react at 45°C for 2h under nitrogen protection, and monitor the endpoint of the reaction by HPLC. After the end of the reaction, filtration, the filtrate was concentrated, 20mLDCM, 20mL water was added and stirred well and partitioned, the aqueous layer was extracted again with DCM, the organic layers were combined, washed once with saturated aqueous sodium chloride, partitioned, dried with anhydrous sodium sulfate, and concentrated to obtain the crude product. The crude product was purified by high performance liquid phase purification with HPLC to obtain the product preparation solution, lyophilized to obtain compound **29m** (0.92g, off-white solid), yield 82.5%, LCMS: [M+H]⁺ =1120.5.

### Step 11: Synthesis of compound 29n

Compound **29m** (920mg,0.82mmol) and 15mL DMF were added into a 100mL single-necked bottle, 1-ethyl-(3-dimethylaminopropyl)carbodiimide hydrochloride (496mg, 2.6mmol) and DMAP (313mg,2.6mmol) were added sequentially under room temperature stirring, and then compound CBI (refer to Patent CN109180681A) (376mg, 1.0mmol) was added, the reaction was protected by N2 at room temperature overnight, HPLC showed that the raw material was basically reacted. Compound CBI (refer to Patent CN109180681A) (376mg, 1.0mmol), N2 protection at room temperature overnight reaction, HPLC showed that the raw materials are basically reacted completely. Add 40 mL of water to the reaction solution, extract with 30 mL of DCM for 2 times, combine the organic layers, wash with water and saturated saline once each in turn, separate the organic layer, dry with anhydrous sodium sulfate, filter, and concentrate under reduced pressure at 40°C to obtain the crude product. The crude product was purified by high performance liquid chromatography (HPLC) to obtain the product preparation solution, and lyophilized to obtain compound **29n** (874 mg, off-white solid), yield 74.5%, LCMS: [M+H]⁺ =1439.5.

### Step 12: Synthesis of compound 29o

Add compound **29n** (874 mg, 0.60mmol), 5%palladium barium sulfate (174 mg), ammonium formate (390 mg, 6 mmol) in a 50 mL three-necked bottle, dissolve in 20 mL of methanol, and the reaction was carried out at 45°C for 2 h under nitrogen protection, the endpoint of the reaction was monitored by HPLC. At the end of the reaction, the filtrate was filtered, the filtrate was concentrated, 20mL DCM, 20mL water was added and stirred well and partitioned, the aqueous layer was extracted again with DCM, the organic layers were combined, washed with saturated aqueous sodium chloride once, partitioned, dried with anhydrous sodium sulfate and concentrated to obtain the crude product. The crude product was purified by high performance liquid phase purification with HPLC to obtain the product preparation solution, lyophilized to obtain compound **29o** (667 mg, off-white solid), yield 82.4%, LCMS: [M+H]⁺ =1349.5.

### Step 13: Synthesis of compound 29p

Compound **29o** (667 mg, 0.49 mmol), 20% hexahydropyridine DMF solution (15 mL) were added in a 50 mL single-necked bottle and reacted at room temperature for 2 h. The end point of the reaction was monitored by HPLC. At the end of the reaction, the filtrate was filtered and the filtrate was purified by high performance liquid phase purification with HPLC to give the product preparation, lyophilized to give compound **29p** (428 mg, off-white solid) in 77.5% yield, LCMS: [M+H]⁺ =1127.5.

### Step 14: Synthesis of compound 29

Compound **29p** (428 mg, 0.38 mmol), DIPEA (156 uL, 0.95 mmol), McOSu (166 mg, 0.57 mmol) were added in a 50 mL single-necked bottle, dissolved in 20 mLDMF, and reacted for 2 h at room temperature, and the end point was monitored by HPLC. At the end of the reaction, the reaction solution was purified by high performance liquid phase purification with HPLC to obtain the product preparation and lyophilizedto yield compound **29** (386 mg, off-white solid) in 77.0% yield, LCMS: [M+H]⁺ =1320.5.

### Example 35:

### Synthesis of Compound 30:

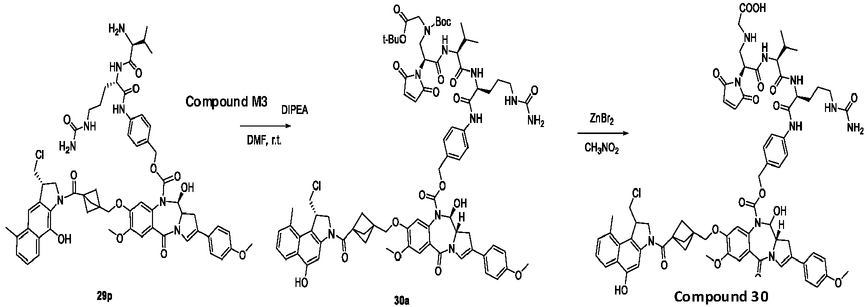

### Step 1: Synthesis of compound 30a

Compound **29p** (560 mg, 0.50 mmol), DIPEA(161 mg, 1.25 mmol), compound M3 (423 mg, 0.75 mmol) were added in a 50 mL single-necked bottle, dissolved in 10 mLDMF, and reacted for 2 h at room temperature, and the end point was monitored by HPLC. At the end of the reaction, the reaction solution was purified by high performance liquid phase purification with HPLC to obtain the product preparation and lyophilized to obtain compound **30a** (622 mg, off-white solid) in 82.6% yield, LCMS: [M+H]⁺ =1507.6.

### Step 2: Synthesis of compound 30

Add compound **30a** (622 mg, 0.41 mmol), zinc bromide (922 mg, 4.1 mmol) in a 50 mL bottle, 10 mL nitromethane was dissolved, the reaction was carried out at 25° C for 45 min, and the endpoint of the reaction was monitored by HPLC, after the end of the reaction, the solvent was removed from the concentrate by spinning it off under reduced pressure at 40 ° C, and then it was purified by high performance liquid chromatography (HPLC) to obtain the product preparation, and then lyophilized to obtain compound 30 (260 mg, white-like solid), yield 48%. **30** (260 mg, off-white solid), yield 48%, LCMS: [M+H]⁺ =1322.5.

### Example 36:

### Synthesis of Compound 31:

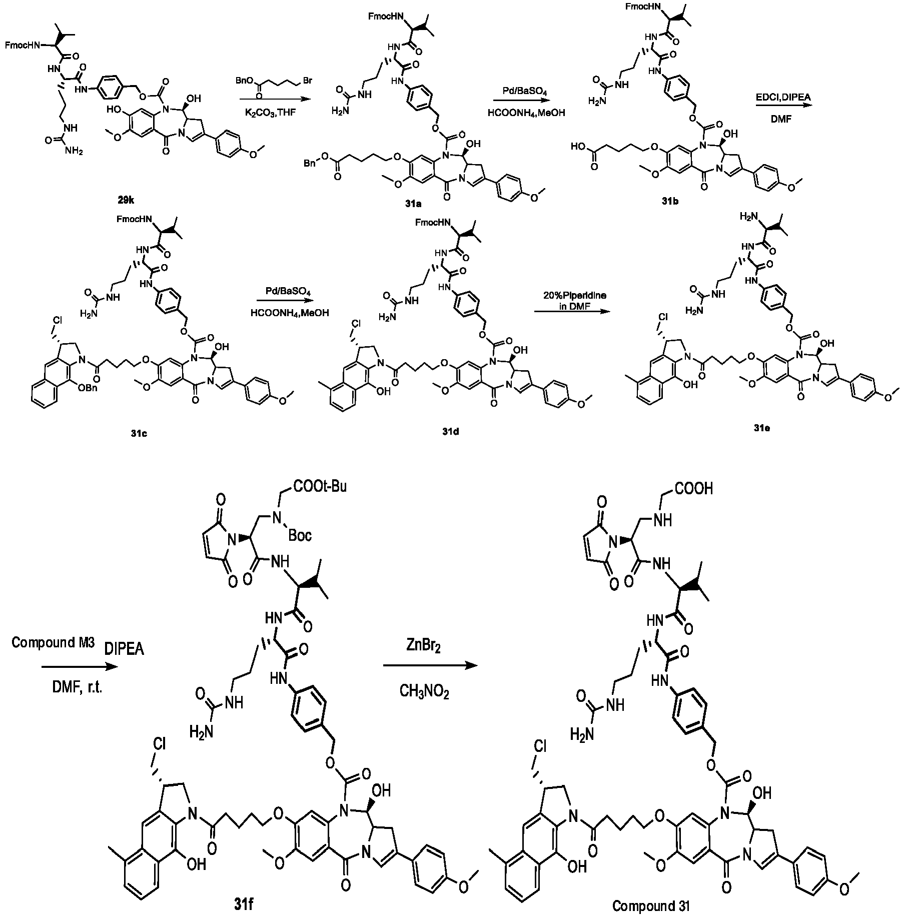

### Step 1: Synthesis of compound 31a

Add benzyl 5-bromovalerate (488 mg, 1.8 mmol), compound **29k** (1.8 g, 1.8 mmol) and 20 mL of DMF in a 100 mL bottle, dissolve with stirring, then add potassium carbonate (372 mg, 2.7 mmol), after the addition, raise the temperature to 60°C reaction for 4h, and then monitor the endpoint of the reaction by HPLC. Add 40mL of water to the reaction solution, extract with 30mL EA for 2 times, combine the organic layers, wash with water and saturated saline once each in turn, separate the organic layer, anhydrous sodium sulfate drying, filtration, and concentration under reduced pressure at 45°C to obtain the crude product, which was purified by high performance liquid chromatography (HPLC) to obtain the product preparation, lyophilized to obtain the compound **31a** (1.8g, off-white solid), yield 84.0%, LCMS : [M+H]⁺ =1186.5.

### Step 2: Synthesis of compound 31b

Add compound **31a** (1.8g, 1.0mmol), 5% palladium barium sulfate (0.36g), ammonium formate (0.95g, 15mmol) in a 50mL triple-necked bottle, dissolve in 20mL methanol, and react at 45°C for 2h under nitrogen protection, and monitor the endpoint of the reaction by HPLC. After the end of the reaction, filtration, the filtrate was concentrated, 20mL DCM, 20mL water was added and stirred well, the aqueous layer was extracted again with DCM, the organic layers were combined, washed once with saturated aqueous sodium chloride solution, the liquid was separated, dried with anhydrous sodium sulfate, and concentrated to obtain the crude product. The crude product was purified by high performance liquid phase purification with HPLC to obtain the product preparation solution, lyophilized to obtain compound **31b** (0.85g, off-white solid), yield 78%, LCMS: [M+H]⁺ =1096.5.

### Step 4: Synthesis of compound 31c

Compound **31b** (850 mg, 0.85 mmol) and 15 mL of DMF were added to a 100 mL bottle. 1-Ethyl-(3-dimethylaminopropyl)carbodiimide hydrochloride (514 mg, 2.7 mmol) and DMAP (324 mg, 2.7 mmol) were added sequentially under stirring at room temperature, and the reaction was stirred for 1 h at room temperature under N₂ protection. Compound CBI (refer to Patent CN109180681A) (376mg, 1.0mmol), N₂ protection at room temperature overnight reaction, HPLC showed that the raw materials are basically reacted completely. To the reaction solution, 40 mL of water was added, extracted twice with 30 mL of DCM, the organic layers were combined, washed with water and saturated saline, the organic layer was separated, dried with anhydrous sodium sulfate, filtered, and concentrated under reduced pressure at 40°C to obtain the crude product. The crude product was purified by high performance liquid phase purification with HPLC to obtain the product preparation solution, lyophilized to obtain compound **31c** (941 mg, off-white solid), yield 78.2%, LCMS: [M+H]⁺ =1415.6.

### Step 5: Synthesis of compound 31d

Add compound **31c** (941 mg, 0.66 mmol), 5% palladium barium sulfate (188 mg), ammonium formate (415 mg, 6.6 mmol) in a 50 mL triple-necked bottle, dissolve in 20 mL of methanol, and react at 45°C for 2 h under nitrogen protection, and monitor the endpoint of the reaction by HPLC. After the end of the reaction, filtration, the filtrate was concentrated, 20mLDCM, 20mL water was added and stirred well and partitioned, the aqueous layer was extracted again with DCM, the organic layers were combined, washed once with saturated aqueous sodium chloride, partitioned, dried with anhydrous sodium sulfate, and concentrated to obtain the crude product. The crude product was purified by high performance liquid phase purification with HPLC to obtain the product preparation solution, lyophilized to obtain compound **31d** (735 mg, off-white solid), yield 84.0%, LCMS: [M+H]⁺ =1325.5.

### Step 6: Synthesis of compound 31e

Compound **31d** (735 mg, 0.55 mmol) was added to 20% hexahydropyridine DMF solution (15 mL) in a 50 mL single-necked bottle and reacted at room temperature for 2 h. The endpoint of the reaction was monitored by HPLC. At the end of the reaction, the filtrate was filtered and the filtrate was purified by high performance liquid phase purification with HPLC to give the product preparation, lyophilized to give compound **31e** (428 mg, off-white solid) in 86.5% yield, LCMS: [M+H]⁺ =1103.5.

### Step 7: Synthesis of compound 31f

Compound **31e** (524 mg, 0.48 mmol), DIPEA (168 mg, 1.30 mmol), compound M3 (441 mg, 0.78 mmol) were added in a 50 mL single-necked bottle, dissolved in 10 mLDMF, and reacted for 2 h at room temperature, and the end point was monitored by HPLC. At the end of the reaction, the reaction solution was purified by high performance liquid phase purification with HPLC to obtain the product preparation and lyophilized to obtain compound **31f** (549 mg, off-white solid) in 77.2% yield, LCMS: [M+H]⁺ =1483.6.

### Step 8: Synthesis of compound 31

Add compound **31f** (549mg, 0.37mmol), zinc bromide (832mg, 3.7mmol) in a 50mL bottle, 10mL nitromethane was dissolved, the reaction was carried out at 25°C for 45min, and the endpoint of the reaction was monitored by HPLC, after the end of the reaction, the solvent was removed from the concentrate by spinning it off under reduced pressure at 40°C, and then the product preparation was obtained by high performance liquid chromatography (HPLC), and compound 31 (213mg, white-like solid) was lyophilized to obtain 43.5% yield, LCMS: [M+H] =1326.5. **31** (213 mg, off-white solid), yield 43.5%, LCMS: [M+H]⁺ =1326.5.

### Example 37:

### Synthesis of Compound 32:

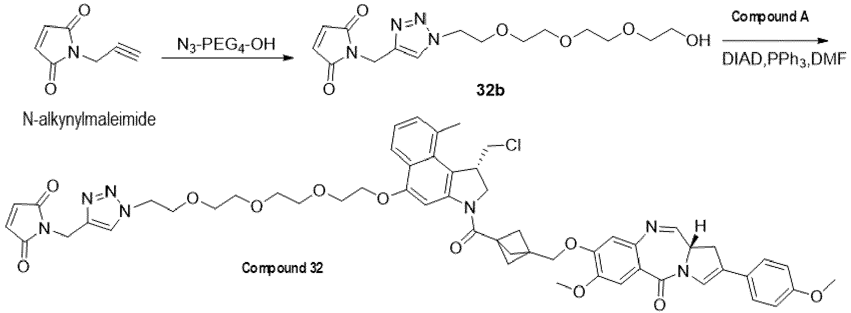

### Step 1: Synthesis of compound 32a

In a 50 mL single-necked bottle, compound N-alkynylmaleimide (500 mg, 3.7 mmol), N₃-PEG₄-OH (811 mg, 3.7 mmol), 10 mL of DMF and 5 mL of water were added to dissolve, afterwards CuSO₄-5H₂ O (1.01 g, 4.1 mmol), sodium ascorbate (806 mg, 4.1 mmol) were added., 4.1 mmol), the reaction was carried out at room temperature for 1 h. The end point of the reaction was monitored by HPLC. At the end of the reaction, the reaction solution was purified by high performance liquid phase preparation to obtain the product preparation, the preparation was lyophilized to give compound **32a** (1.06g) in 81% yield, LCMS: [M+H]⁺ =355.1.

### Step 2: Synthesis of compound 32

**Compound 32a** (20 mg, 0.056 mmol), Compound **A** (19.8 mg, 0.028 mmol), triphenylphosphine (29.6 mg, 0.11 mmol) and 8 mL of DMF were added to a 50 mL triple-necked bottle protected by nitrogen, and the reaction was carried outby dropwise addition of diisopropyl azodicarboxylate (28 uL, 0.11 mmol) to an ice-water bath and raised to room temperature. HPLC monitoring. At the end of the reaction, the preparation was purified and lyophilized to give compound **32** (18 mg) in 63% yield, LC-MS: [M+H]⁺ =1040.4.

### Example 38:

### Synthesis of Compound 33:

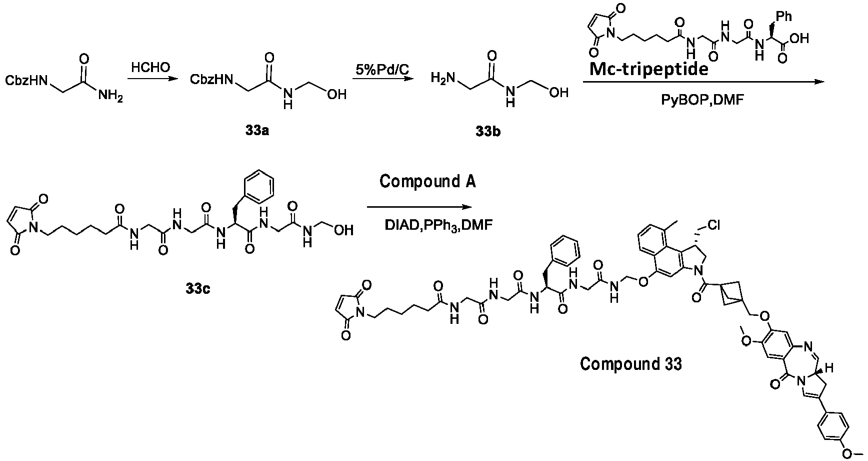

### Step 1: Synthesis of compound 33a

Prepared according to the synthesis method of "Compound 22" in Patent CN111686259A.

### Step 2: Synthesis of compound 33c

Compound **33a** (500 mg, 2.0 mmol) was added in a 50 mL single-necked bottle, dissolved in 8 mLDMF, 300 mg of 5% Pd/C was added, and hydrogenation reaction was carried out for 2 h. The end point of the reaction was monitored by TLC. At the end of the reaction, it was filtered to obtain a DMF solution of compound **32b** and set aside.

To the above DMF solution was added compound **Mc-tripeptide** (992 mg, 2.0 mmol), PyBop (1.3 g, 2.5 mmol), HOBt (339 mg, 2.5 mmol) and DIEA (415 uL, 2.5 mmol), the reaction was carried out at room temperature for 2h, and the end point was monitored by HPLC. The reaction solution was purified by high performance liquid phase purification with HPLC preparation to obtain the product preparation, lyophilized to obtain compound **33c** (580 mg) in 52% yield, LC-MS: [M+H]⁺ = 559.2.

### Step 3: Synthesis of compound 33

**Compound 33c** (20 mg, 0.035 mmol), Compound **A** (12.6 mg, 0.018 mmol), triphenylphosphine (18.4 mg, 0.07 mmol) and 5 mL of DMF were added to a 50 mL triple-necked bottle protected by nitrogen, and diisopropyl azodicarboxylate (14.2 uL, 0.07 mmol) was added dropwise in an ice-water bath; the solution was allowed to come to room temperature. The reaction was monitored by HPLC. At the end of the reaction, purification was prepared and lyophilized to give compound **33** (15 mg) in 68% yield, LC-MS: [M+H]⁺ =1244.5.

### Example 39 (control example):

### Synthesis of compounds 34 and 35:

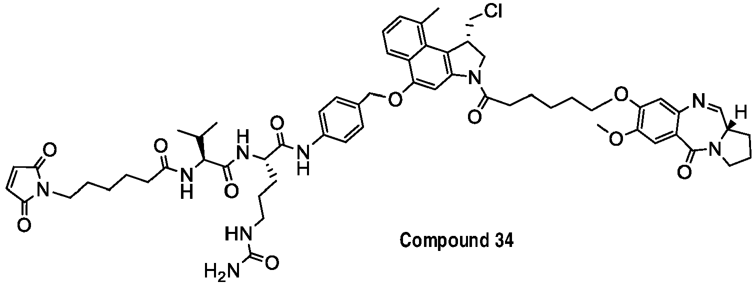

Compound **34** was synthesized accordingto the synthesis method of "Compound 56" in Patent CN105636612 A.

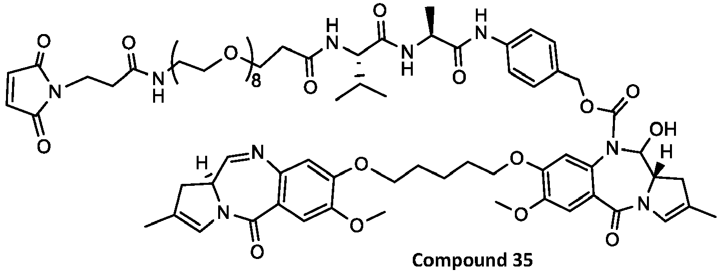

Compound 35 was synthesized according to the synthetic method of "SG3249, tesirine" in the literature "ACS Med Chem Lett. 2016;7:983-987".

### Example 40:

Below is the sequence for Trastuzumab:
Light Chain
   MDMRVPAQLLGLLLLWLRGARC 



 


*
Heavy Chain
   MDMRVPAQLLGLLLLWLRGARC





ASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKT
HTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNK
ALPAPIEKTISKAKGQPREPQVYTLPPSREEMTKNQVSLTCLVKGFYPSDIAVE
WESNGQPENNYKTTPPVLDSDGSFFLYSKLTVDKSRWQQGNVFSCSVMHEAL
HNHYTQKSLSLSPG

### Preparation of ligand-drug conjugates:

### 1) Generalized conjugation methods

Antibody molecules with >95% monomerization after initial purification were exchangedinto phosphate buffer using ultrafiltration centrifugetubes ata concentration of 10 mg/mL. 20 times the molar number of antibody molecules of TCEP was added and the reaction was carried out at room temperature for 4 h to open the disulfide bonds between the antibody chains. Add the linker-drug compound (payload) at the amount of 20 times molar ratio over the antibody and react at room temperature for 2 h. At the end of the reaction, use an ultrafiltration centrifuge tube with a molecular weight cutoff of 30 KDa to exchange the solution into PBS and remove the uncoupled payload. the ADC sample after exchange was filtered through a 0.22 µm bacteriostatic filter and then prepared for use.

### 2) Determination of DAR values of ligand-drug conjugates

### Monomer rate detection conditions:

The samples were centrifuged at 14000 rpm for 5 minutes and the supernatant was taken into the sample for analysis;
Instrument: Waters e2695 (2489UV/Vis);
Chromatographic column: TSKgel G3000SWXL (7.8×300 mm, 5 µm);
Mobile phase: A: 50 mM PB, 300 mMNaCl, 200 mMArg, 5% IPA, pH 6.5;
The mobile phase A was isocratic eluted for 3 0 min at a flow rate of 0.714 mL/min, column temperature 25°C, detection wavelength: 280 nm.

### DAR detection conditions:

The samples were centrifuged at 14000 rpm for 5 minutes and the supernatant was taken into the sample for analysis;
Instrument: Waters H-class (TUV);
Chromatographic column: Proteomix HIC Butyl-NP5 (4.6×35 mm, 5 µm);
Mobile phases: A: 1.5 M ammonium sulfate, 0.025 M anhydrous sodium phosphate, pH 7.0, B: 0.025 M anhydrous sodium phosphate, 25% IPA, pH 7.0;
The separation was carried out on a mobile phase A balanced column with gradient elution of mobile phases A and B at a flow rate of 0.8 mL/min; the column temperature was 25°C and the detection wavelength was 214 nm.

### Example 41 :

ADC-1 was prepared according to the general conjugation method of Example 40:

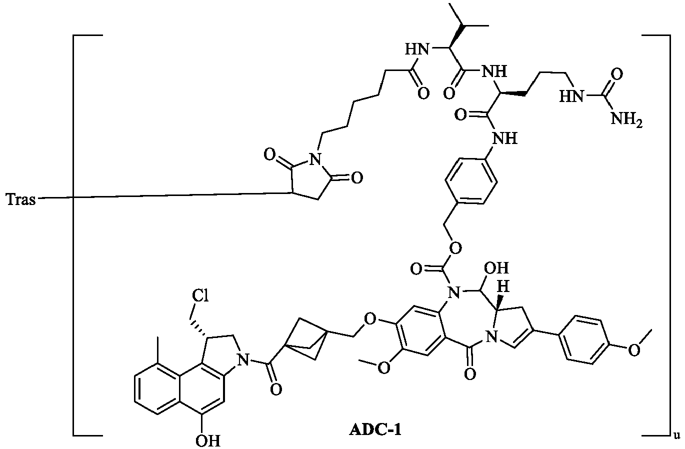

### Example 42:

ADC-2 was prepared according to the general conjugation method of Example 40:

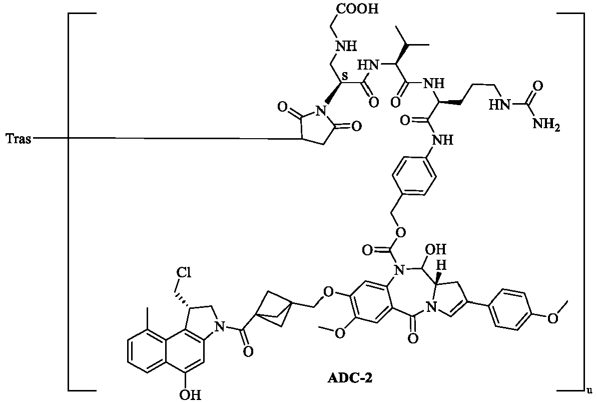

### Example 43 :

**ADC-3 was** prepared according to the general conjugation method of Example 40:

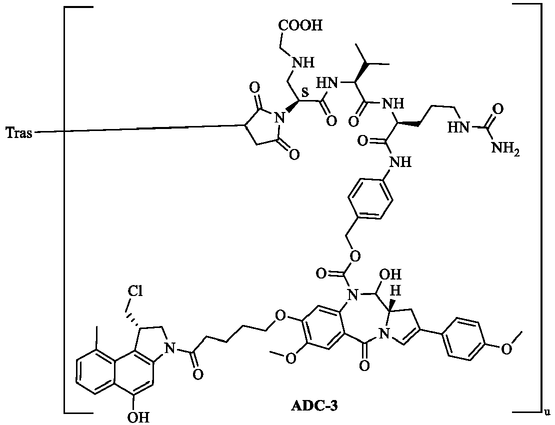

### Example 44:

**ADC-4** was prepared according to the general conjugation method of Example 40:

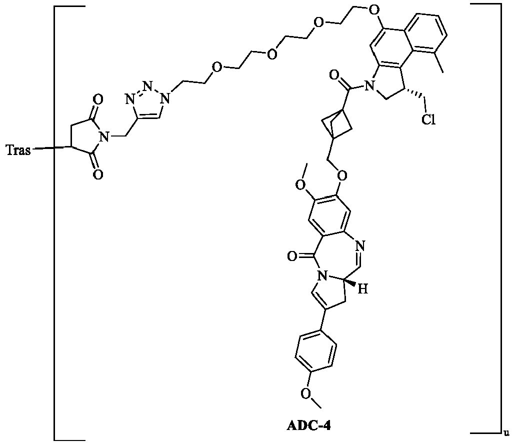

### Example 45:

**ADC-5** was prepared according to the general conjugation method of Example 40:

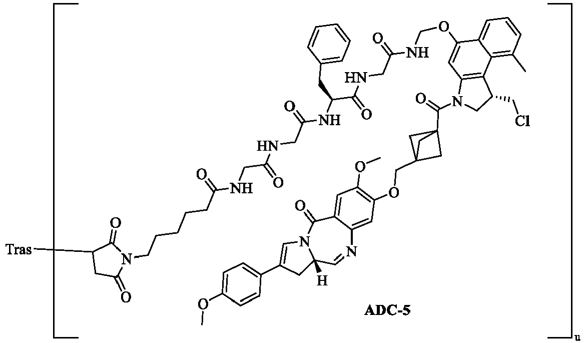

### Example 46:

**ADC-6** was prepared according to the general conjugation method of Example 40:

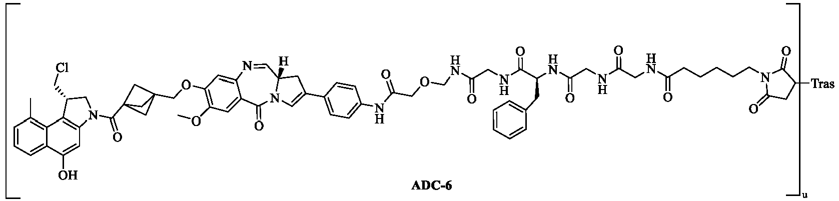

### Example 47:

**ADC-7** was prepared according to the general conjugation method of Example 40:

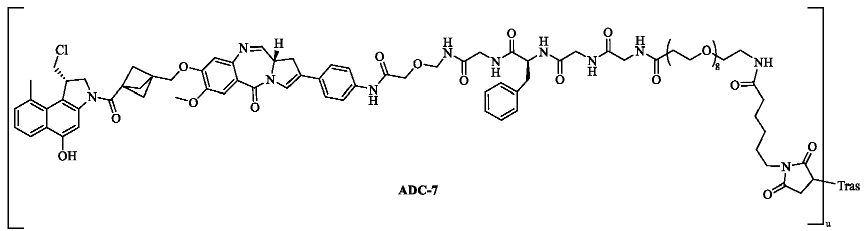

### Example 48:

**ADC-8** was prepared according to the general conjugation method of Example 40:

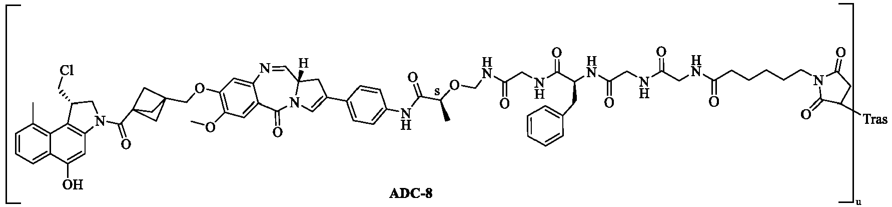

### Example 49:

**ADC-9** was prepared according to the general conjugation method of Example 40:

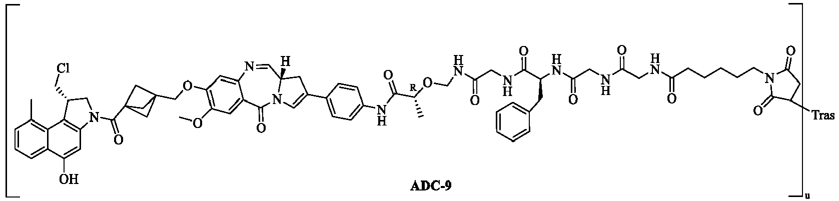

### Example 50:

**ADC-10** was prepared according to the general conjugation method of Example 40:

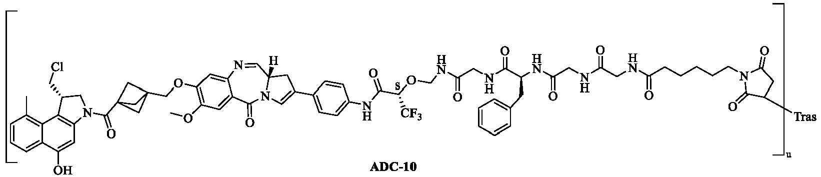

### Example 51 :

**ADC-11** was prepared according to the general conjugation method of Example 40:

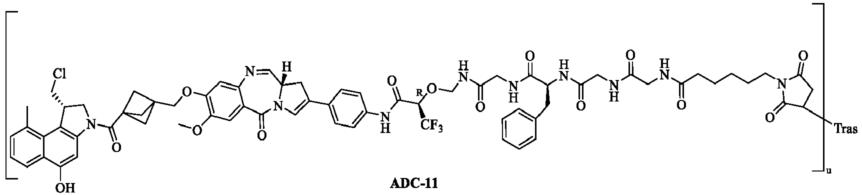

### Example 52:

**ADC-12** was prepared according to the general conjugation method of Example 40:

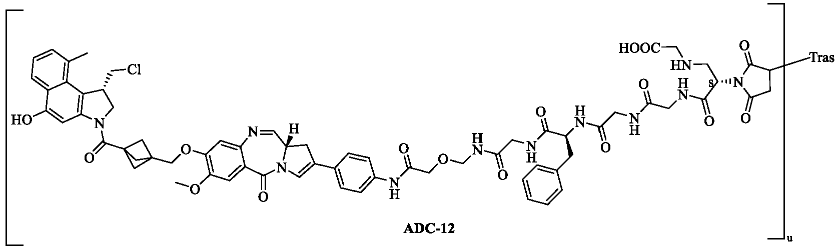

### Example 53:

**ADC-13** was prepared according to the general conjugation method of Example 40:

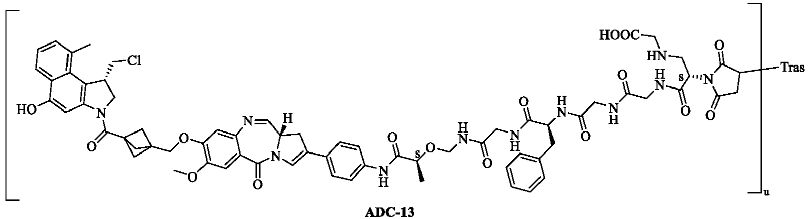

### Example 54:

**ADC-14** was prepared according to the general conjugation method of Example 40:

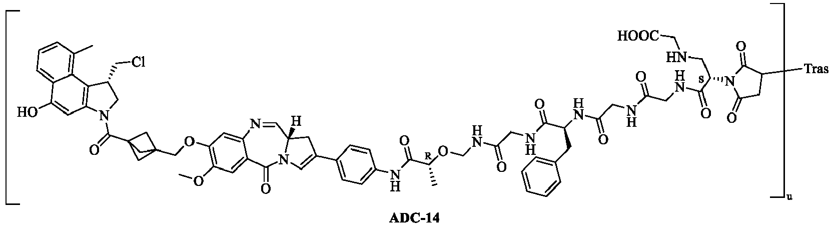

### Example 55:

**ADC-15** was prepared according to the general conjugation method of Example 40:

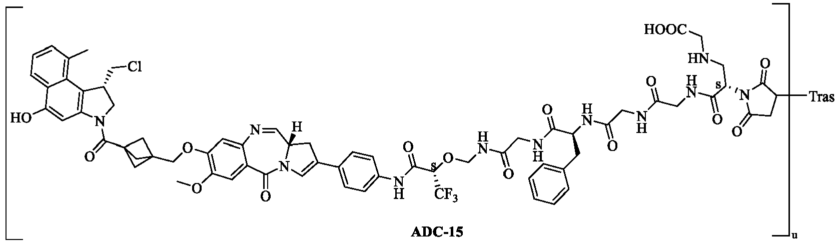

### Example 56:

**ADC-16** was prepared according to the general conjugation method of Example 40:

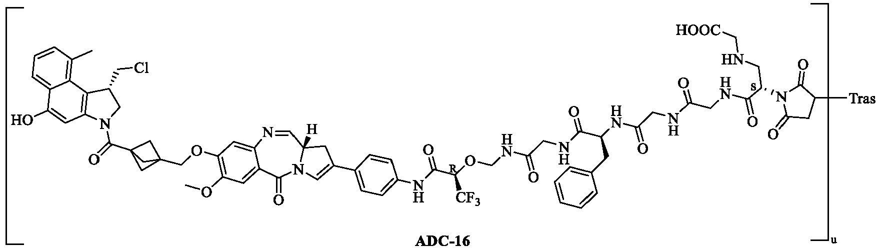

### Example 57:

**ADC-17** was prepared according to the general conjugation method of Example 40:

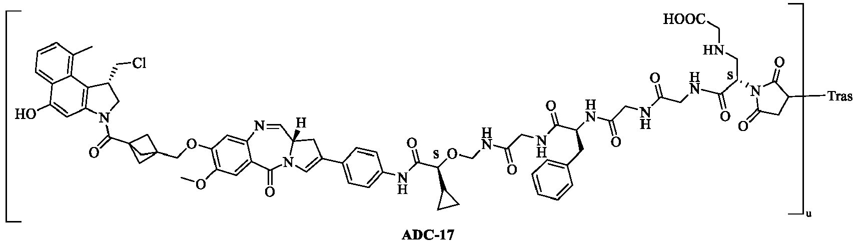

### Example 58:

**ADC-18** was prepared according to the general conjugation method of Example 40:

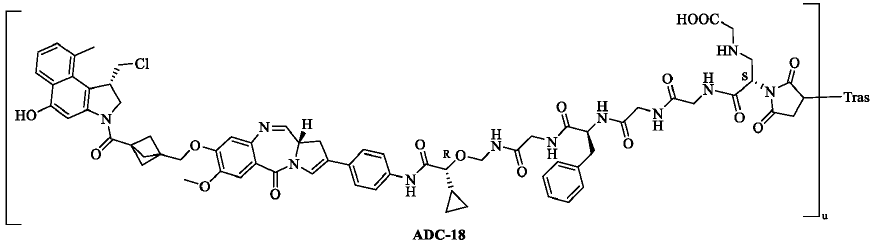

### Example 59:

**ADC-19** was prepared accordingto the generalized conjugation method of Example 40:

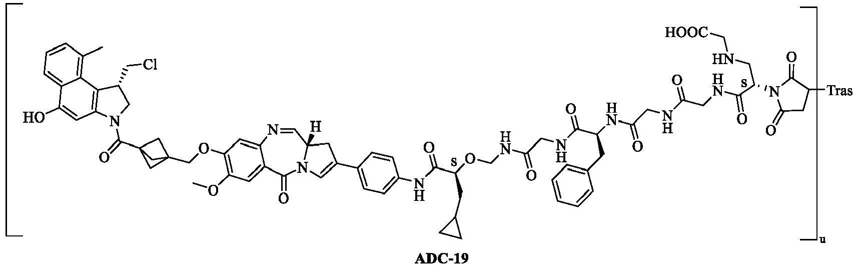

### Example 60:

**ADC-20** was prepared according to the general conjugation method of Example 40:

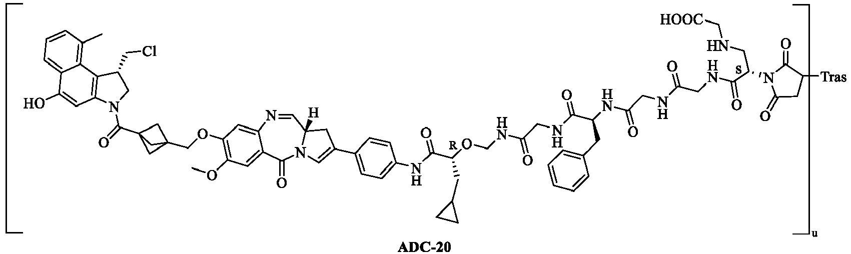

### Example 61 :

**ADC-21** was prepared according to the general conjugation method of Example 40:

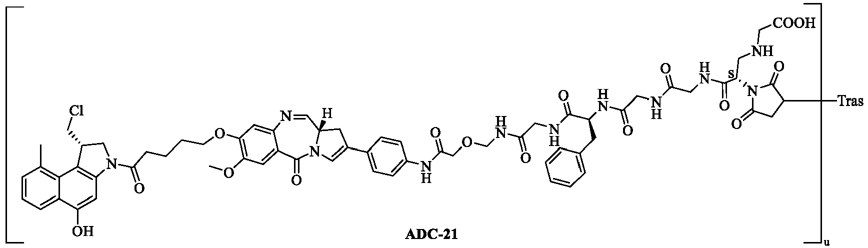

### Example 62:

**ADC-22** was prepared according to the general conjugation method of Example 40:

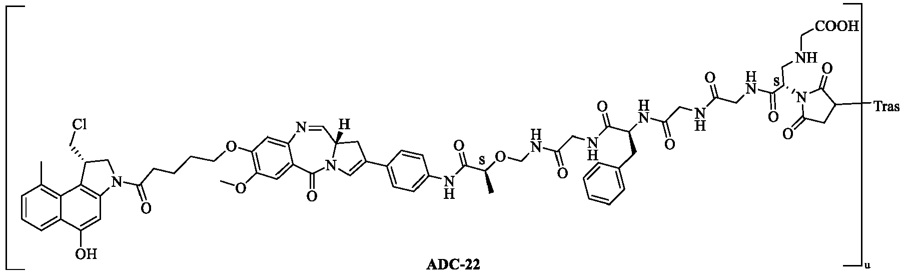

### Example 63:

**ADC-23** was prepared according to the general conjugation method of Example 40:

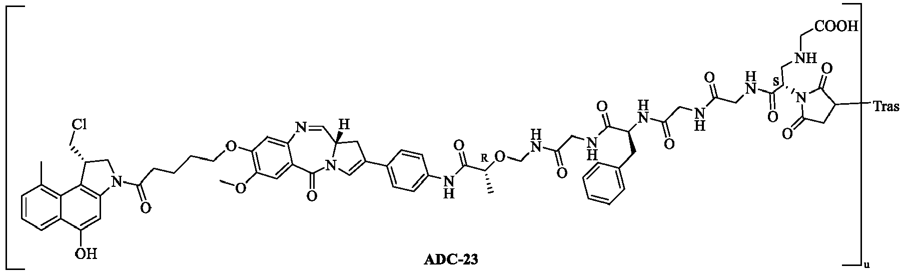

### Example 64:

**ADC-24** was prepared according to the general conjugation method of Example 40:

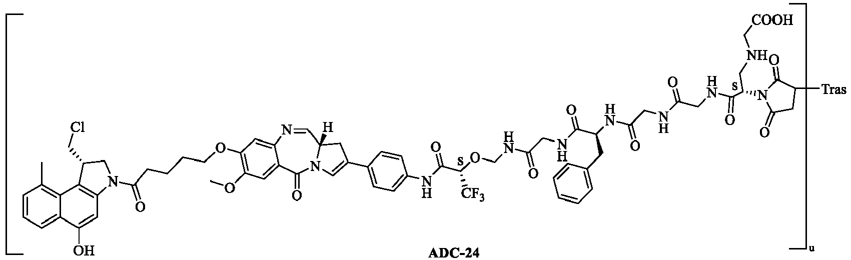

### Example 65:

**ADC-25** was prepared according to the general conjugation method of Example 40:

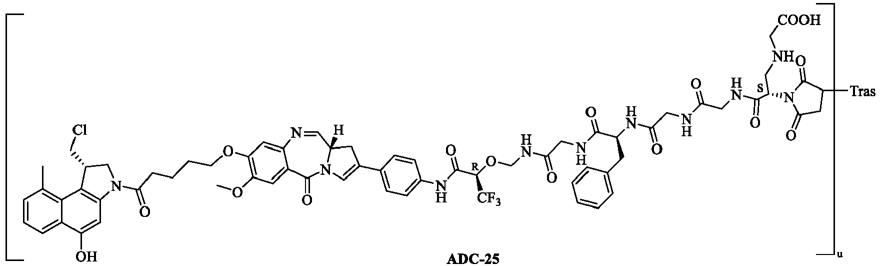

### Example 66:

**ADC-26** was prepared according to the general conjugation method of Example 40:

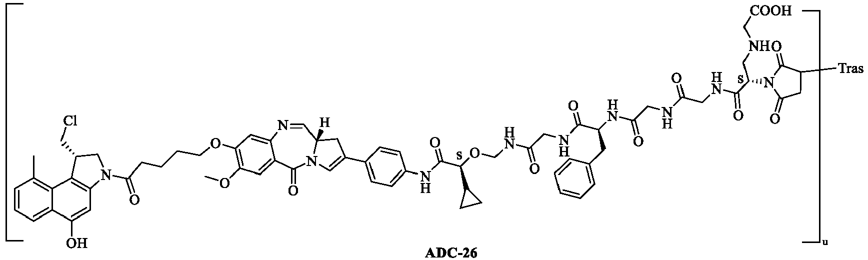

### Example 67:

**ADC-27** was prepared according to the general conjugation method of Example 40:

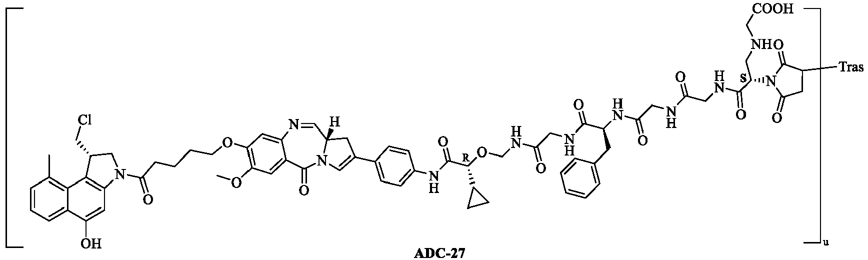

### Example 68:

**ADC-28** was prepared according to the general conjugation method of Example 40:

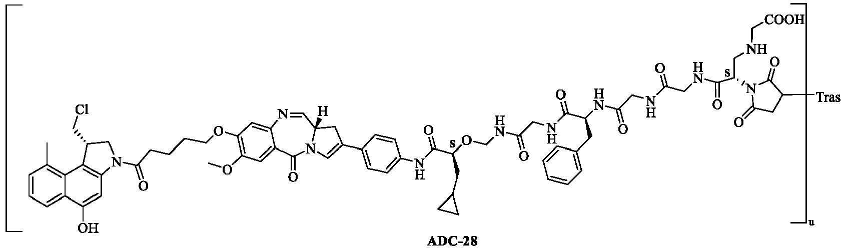

### Example 69:

**ADC-29** was prepared according to the general conjugation method of Example 40:

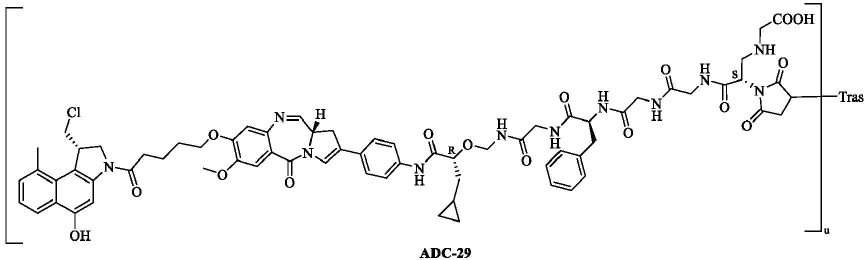

### Example 70:

**ADC-30** was prepared according to the general conjugation method of Example 40:

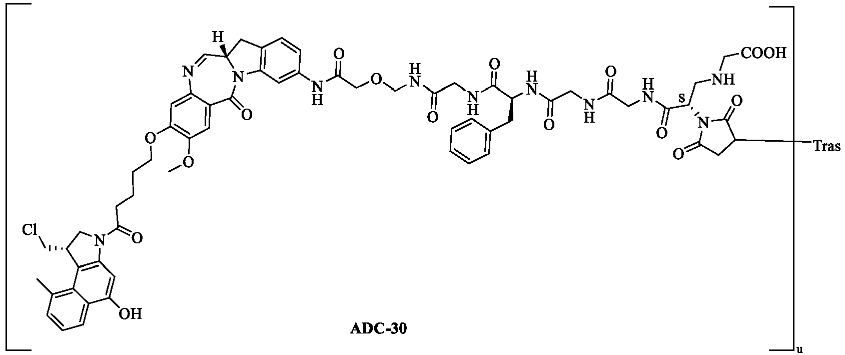

### Example 71 :

ADC-31 was prepared according to the general conjugation method of Example 40:

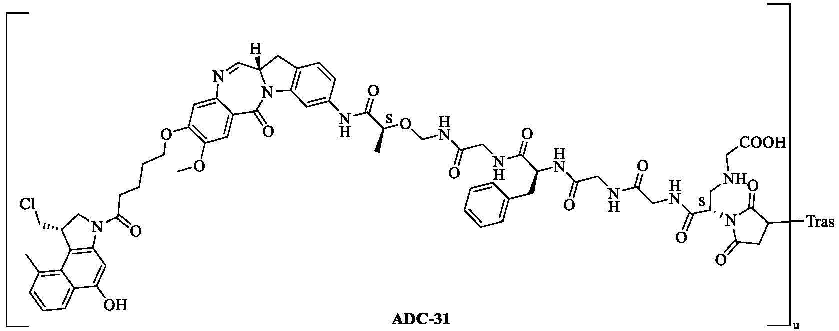

### Example 72:

ADC-32 was prepared according to the general conjugation method of Example 40:

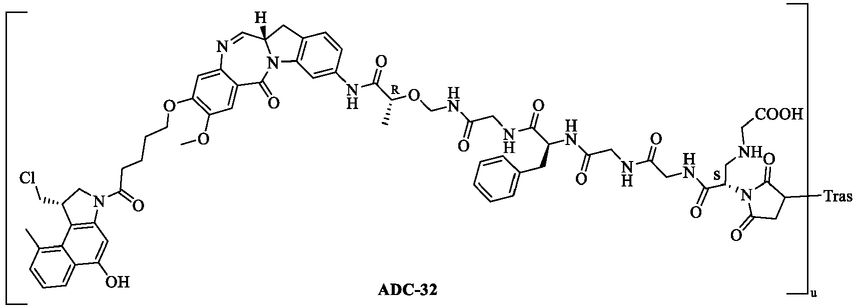

### Example 73:

ADC-33 was prepared according to the general conjugation method of Example 40:

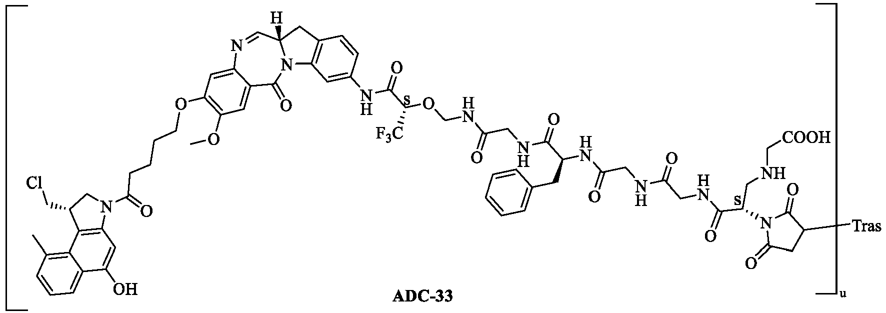

### Example 74:

ADC-34 was prepared according to the general conjugation method of Example 40:

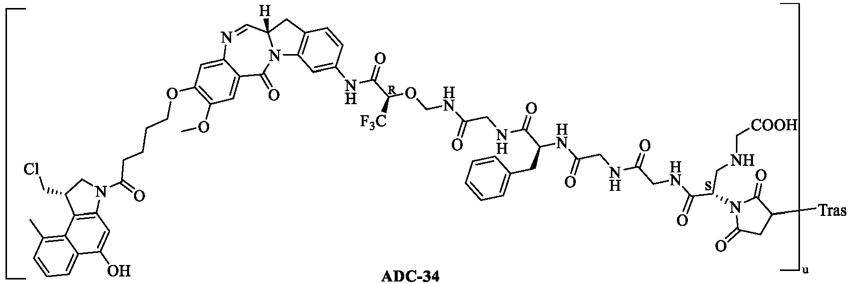

### Example 75:

ADC-35 was prepared according to the general conjugation method of Example 40:

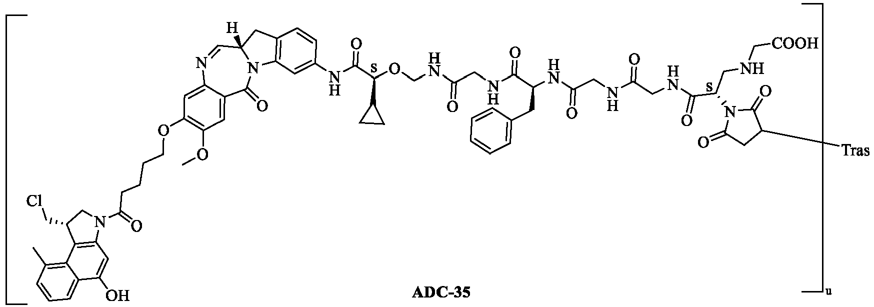

### Example 76:

ADC-36 was prepared according to the general conjugation method of Example 40:

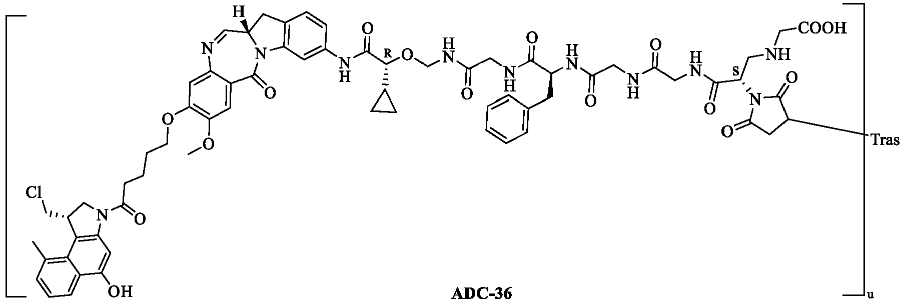

### Example 77:

ADC-37 was prepared according to the general conjugation method of Example 40:

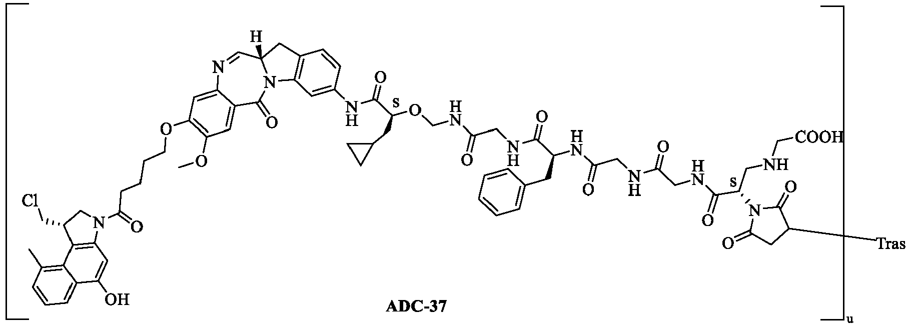

### Example 78:

ADC-38 was prepared according to the general conjugation method of Example 40:

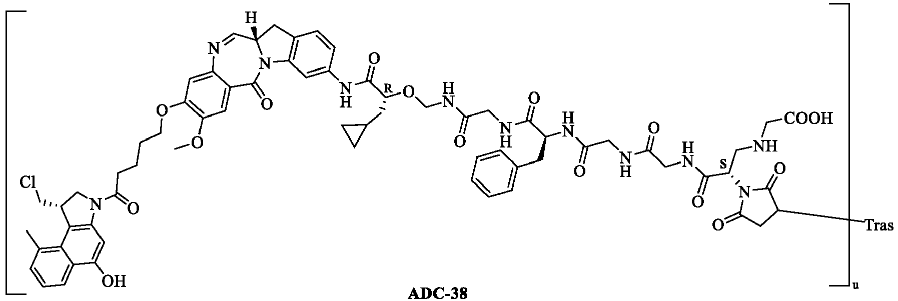

### Example 79 (control):

ADC-39 was prepared according to the general conjugation method of Example 40:

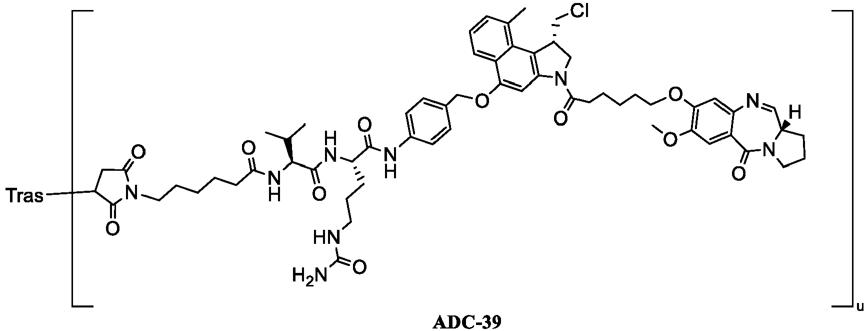

### Example 80 (control):

ADC-40 was prepared according to the general conjugation method of Example 40:

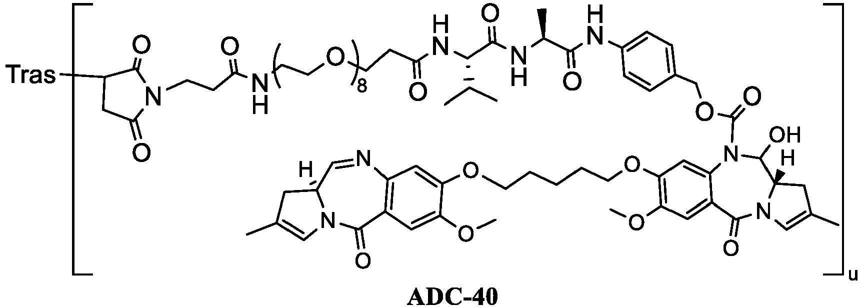

### Example 81: Plasma Stability Assay

### 1. Operation

A certain amount of ADC sample was taken and added to the human plasma from which human IgG had been removed, three tubes of each ADC were repeated and placed in a 37°C water bath for incubation, and after incubation for 72 h and 144 h, respectively, the ADC samples were removed, and 100 uL Protein A resin (MabSelect SuReTM LX Lot: #10221479GE, washed with PBS) was added to each tube, adsorbed by shaking on a vertical mixer for 2 h; after the washing and elution steps, the incubated ADC was obtained. The incubated ADC samples for the specific time periods were subjected to RP-HPLC measurements.

### 2. Results

**Table 1. DAR values and monomer rate data for ligand-drug conjugate (ADCs) of the present application disclosure.**

| Molecule name | DAR | Aggregate % | Mono % |
|---|---|---|---|
| Trastuzumab | NA | 1.61 | 98.39 |
| ADC-3 | 7.87 | 1.43 | 98.57 |
| ADC-5 | 7.65 | 1.71 | 98.29 |
| ADC-7 | 7.56 | 1.31 | 98.69 |
| ADC-12 | 7.54 | 1.81 | 98.19 |
| ADC-21 | 7.60 | 1.42 | 98.58 |
| ADC-30 | 7.64 | 1.46 | 98.54 |

**Table 2. Plasma stability data for ligand-drug conjugate (ADCs) of the present application disclosure.**

| Molecule name | DAR | | | |
|---|---|---|---|---|
| | unincubateed | Incubate 0 days | Incubate for 3 days | Incubate for 7 days |
| ADC-3 | 7.87 | 7.70 | 7.36 | 7.02 |
| ADC-5 | 7.65 | 7.56 | 7.03 | 6.41 |
| ADC-7 | 7.56 | 7.47 | 6.93 | 6.38 |
| ADC-12 | 7.54 | 7.41 | 6.81 | 6.55 |
| ADC-21 | 7.60 | 7.50 | 6.92 | 6.56 |
| ADC-30 | 7.64 | 7.52 | 6.83 | 6.48 |

### 3) Conclusion

As shown in Table 1, the ADCs disclosed in the present application have excellent properties with high DAR values (>7.5) and monomerization rates (>97%).

As shown in Table 2, the DAR values of the ADCs disclosed herein can remain high after 7 days of incubation in plasma, demonstrating that the ADCs of the present application have excellent stability in plasma.

### Example 82: In vitro activity test

### 1) Experimental materials

Cells: derived from the Cell Bank of the Chinese Academy of Sciences;
Tumor cell culture medium: Gibco^{™};
Fetal Bovine Serum (FBS): BIOWEST^{™};

### 2) Preparation of culture medium

Growth medium (with 10% FBS, Penicillin/streptomycin (100 U/mL);
Assay medium (with 1% FBS, Penicillin/streptomycin (100 U/mL);

### 3) Operation

The biosafety cabinet was turned on for UV light irradiation 30 min in advance, and subsequently ventilated for 3 minutes. The growth medium, assay medium, D-PBS and trypsin were preheated in a 37°C thermostatic water bath, after which the surface was sterilized with alcohol and placed in the biosafety cabinet. Cells with a confluence of ~80% (logarithmic growth phase) were selected and placed in a biosafety cabinet, old medium was aspirated off, rinsed with D-PBS, aspirated and discarded, digested with trypsin for 2-3 minutes, after which growth medium was added to terminate the trypsin, and centrifuged at 500 × g for 5 minutes. The centrifugation supernatant was aspirated off, and the cells were mixed with 4 mL of assay medium, and 100 uL of the cells were taken for counting (of which 50 uL of cytosol was taken out, and 50 uL of 0.4% cell solution was added to 50 µL of 0.4% Trypan Blue Stain and mix, count after mixing). Spread the plate according to the previously set cell number, 80 uL/well in 96-well plate, wells E11, F11, G11 only received 80 uL of detection medium, the wells on the edge received 200 uL of DPBS to seal the edge. After the cells are completely attached to the wall (usually at least 4 hours), the test samples were configured and diluted: 1.0 mL of 2.5 µM (5 ×Top Dose) test samples were configured with the assay medium and dispensed into the first column of the V-type 96-well plate, with 200 µL in each well; 180 µL of assay medium was added to the second through eighth columns, and 30 µL was added to the second column from the first column, and mixed up and down with a row gun for 10 minutes. The test medium was mixed up and down 10 times with a row gun, discard the gun tip, and the remaining test concentration points were operated sequentially to perform 7-fold gradient concentration dilution. The gradient concentration of the test sample was added to the cells at 20 uL per well, while only 20 uL of assay medium was added to the 11th column, and three replicate wells were set up for each concentration, followed by placing the 96-well plate into a 5% CO₂, 37°C cell culture incubator and incubating for 5 days.

### 4) Detection

The MTS reagent was removed from the test samples after 5 days of action, thawed at room temperature and protected from light, vortexed and mixed thoroughly, and then 20 µL of Cell Titer One Solution Reagen MTS reagent was added along the side wall of the wells for every 100 µL of cell culture volume in a biosafety cabinet. Gently tapped the plate surface, so that the MTS solution mixed uniformly, placed in a cell culture box with 5% CO₂, 37°C light-avoiding static incubation for 2 h. After the end of the reaction, took out the 96-well plate, placed in the enzyme labeling instrument to detect the OD490 nm absorbance value, and performed the data recording, organization and storage.

### 5) Results

**Table 3: IC50 values of the antibody-drug conjugates and toxins for inhibition ofN87 tumor cells growth in vitro.**

| Sample | IC50 (nM) |
|---|---|
| Trastuzumab | >500 |
| Compound A | 0.82 |
| Compound B1 | 5.03 |
| Compound C1 | 9.68 |
| Compound D1 | 12.33 |
| ADC-3 | 0.61 |
| ADC-5 | 0.74 |
| ADC-7 | 1.55 |
| ADC-12 | 1.82 |
| ADC-21 | 1.11 |
| ADC-30 | 1.05 |
| ADC-39 (control) | 22.86 |

**Table 4: IC50 values of the antibody-drug conjugates and toxins for inhibition of SK-BR-3 tumor cells growth in vitro.**

| Sample | IC50 (nM) |
|---|---|
| Compound A | 0.52 |
| Compound B1 | 4.23 |
| Compound C1 | 8.67 |
| Compound D1 | 10.33 |
| ADC-3 | 0.45 |
| ADC-5 | 0.60 |
| ADC-7 | 4.55 |
| ADC-12 | 5.82 |
| ADC-21 | 6.11 |
| ADC-30 | 9.05 |
| ADC-39 (control) | 26.12 |

### 6) Conclusion

As shown in Table 3, the ligand-drug conjugates of the present application against HER2 targets had significant in vitro growth inhibitory activity against HER2-positive cells N87, which was significantly better than that of naked antibody (Trastuzumab), and control ADC-39.

As shown in Table 4, the ADCs and single agents disclosed in the present application also exhibited significant in vitro growth inhibitory activity against HER2-positive cells SK-BR-3, as compared to the naked antibody (Trastuzumab) and control ADCs.

### Example 83: In vivo safety testing

### 1) Experimental materials

Cells: derived from the Cell Bank of the Chinese Academy of Sciences;
Tumor cell culture medium: Gibco^{™};
Balb/c-nu nude mice: female, 5-7 weeks (mouse age at the time of tumor cell inoculation), body weight 18.0-24.0 g, 180 (120 plus 60 surplus mice). Purchased from Beijing Viton Lihua Laboratory Animal Technology Co;
Tests and controls:
   Supplies: ADC-3, ADC-40 were provided by Chengdu Dote Antibody Drug Co.
   Histidine buffer, provided by Chengdu Dote Antibody Drug Co.
   0.9% Sodium chloride Injection: Kelun Pharmaceuticals, LLC.

### 2) Cell culture

NCI-H1975 (human non-small cell lung cancer adenocarcinoma cells) were cultured in RPMI1640 medium. NCI-H1975 cells in the exponential growth phase were collected and RPMI1640 medium was resuspended to a suitable concentration and used for subcutaneous tumor inoculation in mice.

### 3) Animal modeling and randomization

120 female nude mice were subcutaneously inoculated with 5×10⁷ NCI-H1975 cells on the right shoulder side. When the average tumor volume was about 170 mm³, the mice were randomly grouped according to tumor size. One hundred and twenty hormonal mice with appropriate tumor volume were selected, randomly grouped and started to be administered (tail vein injection, administration volume at 0.1 mL/10g). The day of grouping was defined as day 0.

### 4) Preparation of test and control products

**Table 5. Preparation of donor and control solutions for the study of antitumor effects in NCI-H1975 (human non-small cell lung cancer adenocarcinoma cells) transplantation tumor model.**

| **test item /control** | **dosage (mg/kg)** | **concentration (mg/mL)** | **formulation** | **post-formulation state** | **formulation frequency** | **post-formulation storage** |
|---|---|---|---|---|---|---|
| vehicle | - | - | 1200 µl Histidine buffer. | 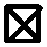 solution □ suspension | formulate on the spot every time | 4 °C |
| ADC-3 | 3.75 | 0.375 | 102 µl of ADC stock solution was mixed with 2298 µl of Histidine buffer, upside down. | 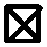 solution □ suspension | formulate on the spot every time | discard after use |
| ADC-3 | 11.25 | 1.125 | 2094 µl of Histidine buffer was added to 306 µl of ADC stock | 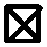 solution □ suspension | formulate on the spot every time | discard after use |
| | | | solution and mixed upside down. | | | |
| ADC-40 (control) | 3.75 | 0.375 | 92 µl of ADC stock solution was mixed with 2308 µl of Histidine buffer, upside down. | 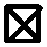 solution □ suspension | formulate on the spot every time | discard after use |
| ADC-40 (control) | 11.25 | 1.125 | To 277 µl of ADC stock solution, 2123 µl of Histidine buffer was added and mixed upside down. | 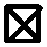 solution □ suspension | formulate on the spot every time | discard after use |
| Trastuzumab | 11.25 | 1.125 | 277 µl of Trastuzumab stock solution was mixed with 2123 µl of Histidine buffer, upside down. | 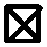 solution □ suspension | formulate on the spot every time | discard after |

| | | | | | | |
|---|---|---|---|---|---|---|
| Note: Mix well before use to ensure that the preparation is homogeneous. | | | | | | |

### 5) Experimental observation and data collection

During this experiment, animal experimental operations were performed according to the requirements of the standard operating procedures for in vivo screening tests of antitumor drugs. After tumor inoculation, routine monitoring included the effects of tumor growth (tumor measured twice a week) and treatment on the normal behavior of the animals, specifically the mobility of the experimental animals, feeding and drinking water, weight gain or loss (body weight measured twice a week), eyes, coat and other abnormalities. Clinical symptoms observed during the experiment were recorded in the raw data. Tumor volume calculation formula: tumor volume (mm³) = 1/2 × (a × b²) (where a indicates the long diameter and b indicates the short diameter). Data were recorded manually in the experiments, including the measurement of the long and short diameters of the tumors and the weighing of the animals.

### 6) Results

**Table 6: Effect of administration of antibody drug conjugation (11.25 mg/kg) on body weight of transplanted tumor-bearing NCI-H1975 mice.**

| **NCI-H1975\ Subgroup information\ Average weight (g)** | **D0** | **D3** | **D7** | **D10** | **D14** | **D17** | **D21** | **D24** | **D28** | **D31** |
|---|---|---|---|---|---|---|---|---|---|---|
| Vehicle | 19.80 | 20.62 | 20.13 | 19.89 | 20.12 | 19.73 | 19.67 | 20.65 | 18.61 | 19.49 |
| ADC-3 (3.75mg/kg) | 19.85 | 20.58 | 19.32 | 20.29 | 20.47 | 19.79 | 20.48 | 19.89 | 20.72 | 20.73 |
| ADC-3 (11.25mg/kg) | 19.85 | 20.50 | 19.71 | 20.50 | 20.35 | 19.88 | 20.25 | 19.99 | 20.12 | 20.61 |
| ADC-40 (3.75mg/kg) control | 19.85 | 20.58 | 19.32 | 20.29 | 20.47 | 19.79 | 20.48 | 19.89 | 20.72* | 20.73* |
| ADC-40 (11.25 mg/kg) control | 19.78 | 20.55 | 19.52 | 19.32 | 19.57* | 19.78* | 19.36* | 19.59* | 20.14* | 18.99* |

| | | | | | | | | | | |
|---|---|---|---|---|---|---|---|---|---|---|
| Note: *Mice deaths were observed in the marker group | | | | | | | | | | |

### 7) Conclusion

As shown in Table 6, the present application discloses that the effects of ADC-3 on the body weight of transplanted tumor-bearing NCI-H1975 mice at low and high doses was significantly less compared to the those of ADC-40, and even at the high dose group, there was no mice death in experimental groups compared to death in the control group, which proves that the ADC drugs described in the present application have a significant advantage in terms of safety.

## Claims

1. A ligand-drug conjugate as shown in Formula I, or a pharmaceutically acceptable salt, deuterated derivative, or solvate thereof:
Ab-L-D (I)
wherein:
Ab is a ligand unit, selected from antibodies, antibody fragments, targeting proteins or
Fc-fusion proteins;
L is a linker unit between D and Ab;
D is a drug unit, selected from the following structures: 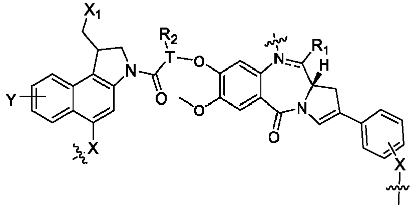 or 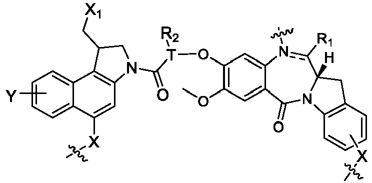
wherein:
the wavy lines indicate sites wherein the drug unit is connected to L, wherein only one of the three sites is connected to L;
R₁ is H, deuterium, OH or an ether represented by OR₃, a sulfite SO₃⁻ or OSO₃⁻, wherein R₃ is selected from a straight, branched or cyclic alkyl, alkenyl or alkynyl groups of C₁-C₁₀;
the double line -̅ -̅ -̅ between N and C represents a single or double bond provided that, when it is a double bond, N is not connected to L at N and R₁ is H; when it is a single bond, N is connected to L at N; R₁ is selected from OH or an ether represented by OR₃, sulfite SO₃⁻ or OSO₃⁻, wherein R₃ is selected from a straight, branched, or cyclic alkyl, alkenyl, or alkynyl group of C ₁-C₁₀;
R₂ is an H or alkyl;
T is selected from C₂-C₁₂ alkyl, Z, (C₁-C₆ alkylidene)-Z-(C₁-C₆ alkylidene), (C₁-C₆ alkylidene)-Z-(C₁-C₆ alkylidene)-Z-(C₁-C₆ alkylidene), (C₁-C₆ alkylidene)-Z-(C₁-C₆ alkylidene), or (C₁-C₆ alkylidene)-Z-(C₁-C₆ alkylidene);
wherein:
Z is selected from O, S, NR₄, aryl or heteroaryl; wherein R₄ is selected from H, P(O)₃H₂ or C(O)NR₅R₆; wherein R₅ and R₆ are selected from H, C₁-C₆ alkyl, one or more F-substituted C₁-C₆ alkyls, or wherein R₅ and R₆ form a five-membered or six-membered heterocyclic group;
alkylidene, alkenylidene, aryl or heteroaryl are selected from F, OH, O(C₁-C₆ alkyl), NH₂, NHCH₃, N(CH₃)₂ or C₁-C₆ alkyl-substituted, wherein the alkyl group is substituted with one or more F;
Y is selected from one or more H or C₁-C₄ alkyl groups;
X is selected from -O-, -N-, -S-, -OC(O)-CR₇R₈-(CR₉R₁₀)m-O-, -OC(O)-CR₇R₈-(CR₉R₁₀)m-NH-, -OC(O)-CR₇R₈-(CR₉R₁₀)m-S-, -NHC(O)-CR₇R₈-(CR₉R₁₀)m-O-, - NHC(O)-CR₇R₈-(CR₉R₁₀)m-NH- or -NHC(O)-CR₇R₈-(CR₉R₁₀)m-S-;
wherein:
R₇, R₈ are independently a hydrogen atom, a deuterium atom, a halogen, an alkyl group, a deuteroalkyl group, a haloalkyl group, a cycloalkyl group, a cycloalkyl alkyl group, an alkoxyalkyl group, a heterocyclyl group, an aryl group, a substituent aryl group or a heteroaryl group, respectively; alternatively, R₇, R₈ and the carbon atoms connected thereto constitute a C₃-C₆ cycloalkyl group, a cycloalkyl alkyl group or a heterocyclyl group;
R₉, R₁₀ are identical or different and are independently hydrogen, deuterium, halogen, alkyl, haloalkyl, deuteroalkyl, alkoxy, hydroxy, amino, cyano, nitro, hydroxyalkyl, cycloalkyl, or heterocycloalkyl, respectively; alternatively, R₉, R₁₀ and the carbon atoms connected thereto constitute a C₃-C₆ cycloalkyl, cycloalkylalkyl, or heterocycloalkyl;
m is chosen from integers 0-4; and
X₁ is selected from halogen or OSO₂R₁₁, wherein R₁₁ is selected from H, C₁-C₄ hydrocarbyl, phenyl or substituted phenyl.

2. The ligand-drug conjugate or a pharmaceutically acceptable salt, deuterated derivative, solvate thereof according to claim 1, wherein Ab is derived from an antibody, which is configured to form a linkage bond with the linker unit through its heteroatom, said antibody being selected from murine antibodies, chimeric antibodies, humanized antibodies, fully human antibodies, antibody fragments, bispecific antibodies, or multispecific antibodies.

3. The ligand-drug conjugate or a pharmaceutically acceptable salt, deuterated derivative, solvate thereof according to claim 1 or 2, wherein the antibody or antigen-binding fragment comprises: anti-EGFRvIII antibody, anti-DLL-3 antibody, anti-PSMA antibody, anti-CD70 antibody, anti-MUC16 antibody, anti-ENPP3 antibody, anti-TDGF1 antibody, anti-ETBRantibody, anti-MSLN antibody, anti-TIM-1 antibody, anti-LRRC15 antibody, anti-LIV-1 antibody, anti-CanAg/AFP antibody, anti-cladin 18.2 antibody, anti-Mesothelin antibody, anti-HER2 (ErbB2) antibody, anti-EGFR antibody, anti-c-MET antibody, anti-SLITRK6 antibody, anti-KIT/CD117 antibody, anti-STEAP1 antibody, Anti-SLAMF7/CS1 antibody, anti-NaPi2B/SLC34A2 antibody, anti-GPNMB antibody, anti-HER3(ErbB3) antibody, anti-MUC1/CD227 antibody, anti-AXL antibody, anti-CD166 antibody, anti-B7-H3(CD276) antibody, anti-PTK7/CCK4 Antibodies, anti-PRLR antibodies, anti-EFNA4 antibodies, anti-5T4 antibodies, anti-NOTCH3 antibodies, anti-Nectin 4 antibodies, anti-TROP-2 antibodies, anti-CD142 antibodies, anti-CA6 antibodies, anti-GPR20 antibodies, anti-CD174 antibodies, anti-CD71 antibodies, anti- EphA2 antibody, anti-LYPD3 antibody, anti-FGFR2 antibody, anti-FGFR3 antibody, anti-FRα antibody, anti-CEACAMs antibody, anti-GCC antibody, anti-Integrin Av antibody, anti-CAIX antibody, anti-P-cadherin antibody, anti-GD3 antibody, anti-Cadherin 6 antibody, anti-LAMP1 antibody, anti-FLT3 antibody, anti-BCMAantibody, anti-CD79b antibody, anti-CD19 antibody, anti-CD33 antibody, anti-CD56 antibody, anti-CD74 antibody, anti-CD22 antibody, anti-CD30 antibody, anti-CD37 antibody, anti-CD47 antibody, anti-CD138 antibody, anti-One or more of CD352 antibody, anti-CD25 antibody, or anti-CD123 antibody.

4. The ligand-drug conjugate or a pharmaceutically acceptable salt, deuterated derivative, solvate thereof according to claim 1 or 2, wherein L is cleavable or non-cleavable.

5. The ligand-drug conjugate or a pharmaceutically acceptable salt, deuterated derivative, solvate thereof as claimed in claim 1 or 2, wherein the pharmaceutically acceptable salt comprises a sodium, potassium, calcium, or magnesium salt formed with an acidic functional group in the structure; or an acetate, trifluoroacetate, citrate, oxalate, tartrate, malate, nitrate, chloride, bromide, iodide, sulfate, bisulfate, phosphate, lactate, oleate, ascorbate, salicylate, formate, glutamate, methanesulfonate, ethane sulfonate, benzenesulfonate, or p-toluenesulfonate formed with a basic functional group in the structure.

6. The ligand-drug conjugate compound or a pharmaceutically acceptable salt, deuterated derivative, or solvate thereof as claimed in any one of claims 1-5, prepared for use as a therapeutic or prophylactic tumor drug.

7. The use as claimed in claim 6, wherein: the tumor is a solid tumor or a hematoma.

8. The use as claimed in claim 7, wherein: the tumor is breast cancer, ovarian cancer, cervical cancer, uterine cancer, prostate cancer, kidney cancer, urethral cancer, bladder cancer, liver cancer, stomach cancer, endometrial cancer, salivary gland cancer, esophageal cancer, lung cancer, colon cancer, rectum cancer, colorectal cancer, bone cancer, skin cancer, thyroid cancer, pancreatic cancer, melanoma, glioma, neuroblastoma, Glioblastoma multiforme, sarcoma, lymphoma, or leukemia.

9. A compound of formula II or III, or a pharmaceutically acceptable salt or solvate thereof, linked to the Ab as claimed in claim 1: 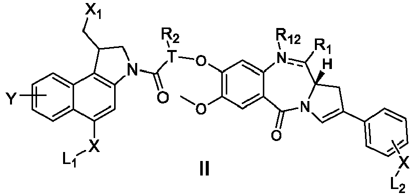 or 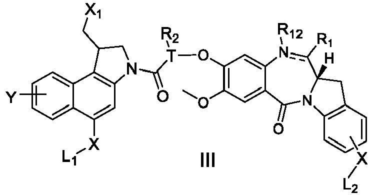 wherein:
R₁ is H, OH or an ether represented by OR₃, a sulfite SO₃⁻ or OSO₃⁻, wherein R₃ is selected from the straight, branched or cyclic alkyl, alkenyl or alkynyl groups of C₁-C₁₀;
The double line -̅ -̅ -̅ between N and C represents a single or double bond provided that, when it is a double bond, R₁₂ is absent and R₁ is H; when it is a single bond, R₁₂ is -C(O)O-L₃, wherein L₃ is the linking unit; and R₁ is selected from OH, an ether represented by OR₃, a sulphite SO₃⁻, or an OSO₃⁻, wherein R₃ is selected from a straight-chained, branched, or cyclic alkyl, alkenyl, or alkynyl group of C₁-C₁₀;
R₂ is an H or alkyl;
T is selected from C₂-C₁₂ alkyl, Z, (C₁-C₆ alkylidene)-Z-(C₁-C₆ alkylidene), (C₁-C₆ alkylidene)-Z-(C₁-C₆ alkylidene)-Z-(C₁-C₆ alkylidene), (C₁-C₆ alkylidene)-Z- (C₁-C₆ alkylidene), or (C₁-C₆ alkylidene)-Z-(C₁-C₆ alkylidene);
wherein:
Z is selected from O, S, NR₄, aryl and heteroaryl; wherein R₄ is selected from H, P(O)₃H₂, C(O)NR₅R₆, wherein R₅ and R₆ are selected from H, C₁-C₆ alkyl, one or more F-substituted C₁-C₆ alkyls or wherein R₅ and R₆ form a five-membered or six-membered heterocyclic group;
Alkylidene, alkenylidene, arylidene and heteroarylidene are selected from F, OH, O(C₁-C₆ alkylidene), NH₂, NHCH₃, N(CH₃) ₂ and C₁-C₆ alkylidene-substituted, wherein the alkylidene is substituted with one or more F;
Y is selected from one or more H or C₁-C₄ alkyl groups;
X selected from -O-, -N-, -S-, -OC(O)-CR₇R₈-(CR₉R₁₀)m-O-, -OC(O)-CR₇R₈-(CR₉R₁₀)m-NH-, -OC(O)-CR₇R₈-(CR₉R₁₀)m-S-, -NHC(O)-CR₇R₈-(CR₉R₁₀)m-O-, - NHC(O)-CR₇R₈-(CR₉R₁₀)m-NH- or -NHC(O)-CR₇R₈-(CR₉R₁₀)m-S-;
wherein:
R₇, R₈ are independently a hydrogen atom, a deuterium atom, a halogen, an alkyl group, a deuteroalkyl group, a haloalkyl group, a cycloalkyl group, a cycloalkyl alkyl group, an alkoxyalkyl group, a heterocyclyl group, an aryl group, a substituted aryl group or a heteroaryl group, respectively; alternatively, R₇, R₈ and the carbon atoms connected thereto constitute a C₃-C₆ cycloalkyl group, a cycloalkyl alkyl group or a heterocyclyl group;
R₉, R₁₀ are identical or different and are independently hydrogen, deuterium, halogen, alkyl, haloalkyl, deuteroalkyl, alkoxy, hydroxy, amino, cyano, nitro, hydroxyalkyl, cycloalkyl, or heterocycloalkyl, respectively; alternatively, R₉, R₁₀ and the carbon atoms connected thereto constitute a C₃-C₆ cycloalkyl, cycloalkylalkyl, or heterocycloalkyl;
m is selected from integers 0-4;
X₁ is selected from halogen or OSO₂R₁₁, wherein R₁₁ is selected from the hydrocarbon group of H, C₁-C₄, phenyl or substituted phenyl;
L₁, L₂ are linker units or substituents.

10. The compounds II, III or pharmaceutically acceptable salts or solvates thereof according to claim 9, wherein T is selected from the alkylene group of C₂-C₁₂ .

11. The compound of formula II, III or pharmaceutically acceptable salts or solvates thereof according to claim 9 or 10, wherein T is

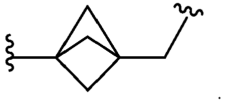

12. The compound of formula II, III or pharmaceutically acceptable salts or solvates thereof according to claim 9 or 10, wherein X is -O-, -N- or -NHC(O)-CR₇R₈-(CR₉R₁₀)m-O-.

13. The compound of formula II, III or pharmaceutically acceptable salts or solvates thereof according to claim 9 or 10, wherein L₃ is: 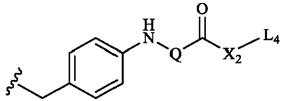 wherein: a connection to -C(O)O- occurs at the wavy line; L₄ is a linker unit, connected to the ligand unit.

14. The compound of formula II, III, or pharmaceutically acceptable salts or solvates thereof according to claim 9 or 10, wherein L₄ comprises: 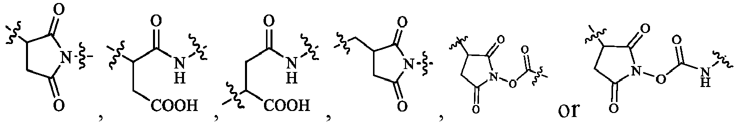 wherein: at the left wavy line, a carbon is connected to the ligand unit, and at the right wavy line, a nitrogen or ester carbonyl is connected to X₂ .

15. The compound of formula II, III, or pharmaceutically acceptable salts or solvates thereof according to claim 9 or 10, wherein Q is: 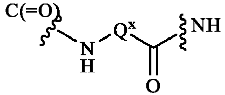 wherein Q^{x} is an amino acid residue or a peptide residue comprising an amino acid

16. The compound of formula II, III, or pharmaceutically acceptable salts or solvates thereof according to claim 9 or 10, wherein X₂ is: 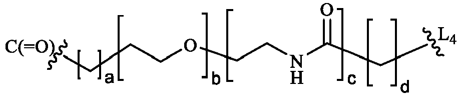 where a is selected from integers 0-5, b is selected from integers 0-16, c is selected from integers 0-1, and d is selected from integers 0-5.

17. The compound of formula II, III, or pharmaceutically acceptable salts or solvates thereof according to claim 9 or 10, wherein L₃ comprises: 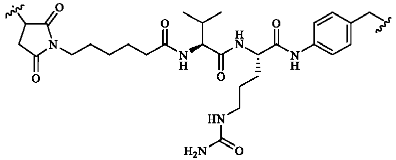 or 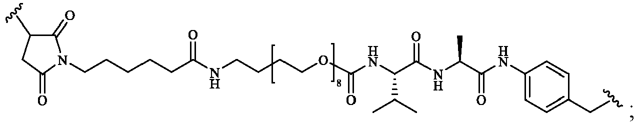 wherein: at the left wavy line, the butanediimide is connected to the ligand unit, and at the right wavy line, a connection to -C(O)O- occurs.

18. The compound of formula II, III, or pharmaceutically acceptable salts or solvates thereof according to claim 9 or 10, wherein L₁ and L₂ are independently selected from:
structure A: a hydrogen atom, C(O)NR'R", wherein R' and R" are selected from H, C₁-C₆ alkyl, one or more F-substituted C₁-C₆ alkyls, respectively; or wherein R' and R" form a five-membered or six-membered heterocyclic group; and
structure B: L₄-L₅-, L₄-L₆- or L₄-L₇-L₈-L₉-, wherein L₄, L₅, L₆, L₇, L₈, and L₉ are linker units, L₄ is connected to the ligand unit, and L₅, L₆, and L₉ are connected to X.

19. The compound of formula II, III, or pharmaceutically acceptable salts or solvates thereof according to claim 9 or 10, wherein:
when there is a single bond between N and C, and R₁₂ is present, L₁, L₂ are independently selected from structure A; and
when there is a double bondbetweenN and C, and R₁₂ is not present, L₁ is structure A or B, then L₂ is structure B or A.

20. The compound of formula II, III, or pharmaceutically acceptable salts or solvates thereof according to claim 18, wherein:
L₅ is -((CH₂)sO)r(CH₂)sX₃L₁₀- or -((CH₂)sO)r(CH₂)sX₄L₁₀-;
L₆ is -((CH₂)sO)r(CH₂)s-;
L₁₀ is -(CH₂)s- or -((CH₂)sNHC(=O)X₅X₆C(=O)(CH₂)s-;
wherein:
X₃ comprises the following group: 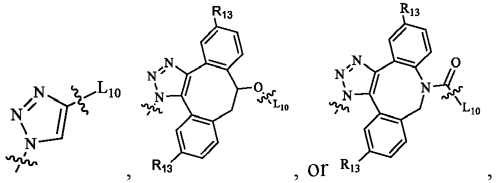
wherein R₁₃ is independently selected from a hydrogen atom, a C₁-C₆ hydrocarbon group, a halogen atom or a hydroxyl group;
X₄ comprises the following group: 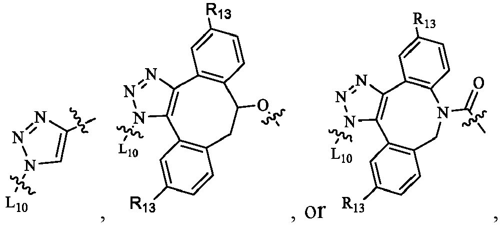
wherein R₁₃ is independently selected from a hydrogen atom, C₁-C₆ a hydrocarbon group, a halogen atom or a hydroxyl group;
X₅ comprises the following group: 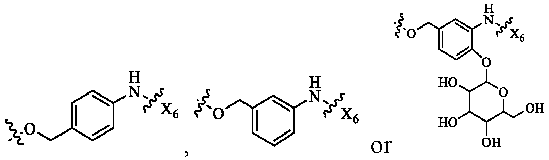
X₆ is selected from peptide residues consisting of amino acids comprising: 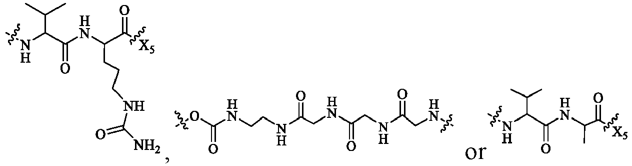
s is an integer from 1-10 and r is an integer from 1-14.

21. The compound of formula II, III, or pharmaceutically acceptable salts or solvates thereof according to claim 18, wherein:
L₇ is -NC(R₁₄R₁₅)C(O), -NR₁₆(CH₂)ₒC(O)-, -NR₁₆(CH₂CH₂O)ₒCH₂C(O)-, - S(CH₂)ₚC(O)-, or a chemical bond, wherein o is selected from an integer from 0-20; p is an integer from 0-20; R₁₄ is identical or different from R₁₅, and each is independently selected from a hydrogen atom, a deuterium atom, an alkyl, a substituted alkyl, a deuterioalkyl, a heteroalkyl carboxyl, amino, substituted amino; R₁₆ is selected from a hydrogen atom, a deuterium atom, a halogen, an alkyl, a substituted alkyl, a deuteroalkyl, a cycloalkylalkyl, an alkoxyalkyl, an aryl, a substituted aryl or a heteroaryl;
L₈ is selected from peptide residues derived from free amino acids;
L₉ is -NR₁₇(CR₁₈R₁₉)_{q}-, -C(O)NR₁₇-, -C(O)NR₁₇(CH₂)_{q}-, or a chemical bond, wherein q is selected from an integer from 0 to 6; R₁₇, R₁₈ and R₁₉ are identical or different and are each independently selected from a hydrogen atom, a deuterium atom, a halogen, an alkyl group, a substituted alkyl group, a deuterated alkyl group, a cycloalkyl group, a cycloalkyl alkyl group, an alkoxyalkyl group, a heterocyclic group, an aryl group, a substituted aryl group, or a heteroaryl group.

22. The compound of formula II, III, or pharmaceutically acceptable salts or solvates thereof according to claim 21, wherein said L₈ is a peptide residue derived from one or more amino acids comprising phenylalanine, glycine, valine, lysine, citrulline, serine, glutamate, or aspartic acid.

23. The compound of formula II, III, or pharmaceutically acceptable salts or solvates thereof according to claim 19, wherein L₁, L₂ are independently selected from structure B comprising: 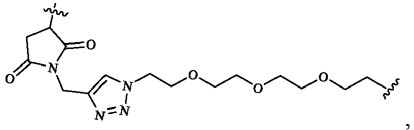 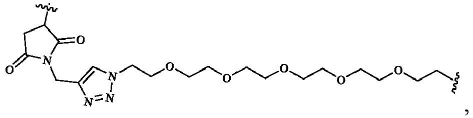 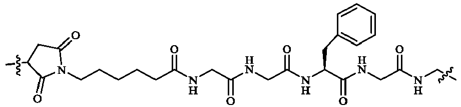 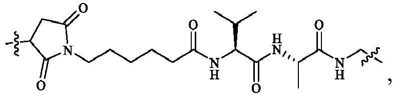 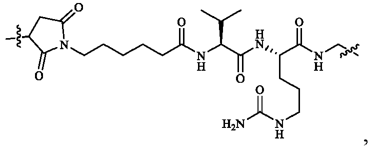 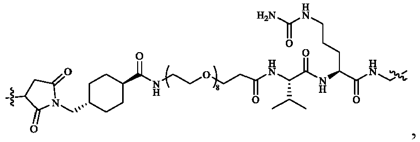 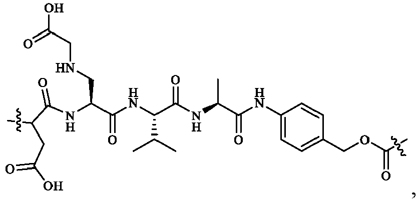 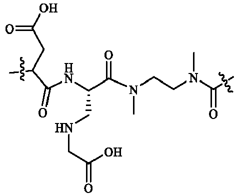 or

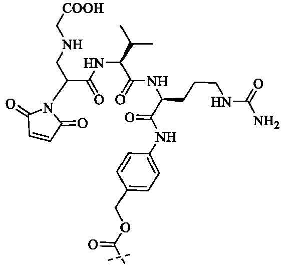

24. The compound of formula II, III or pharmaceutically acceptable salts or solvates thereof according to claim 9, further comprising a structure comprising the following: 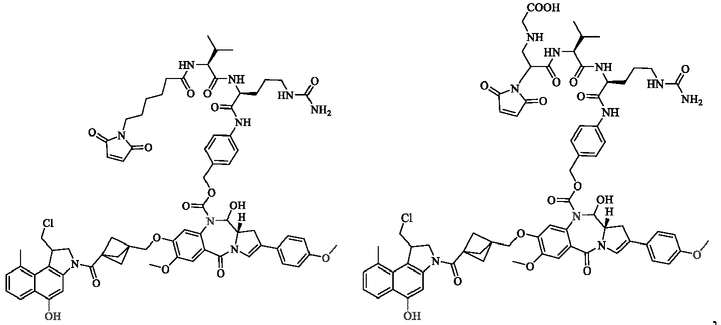 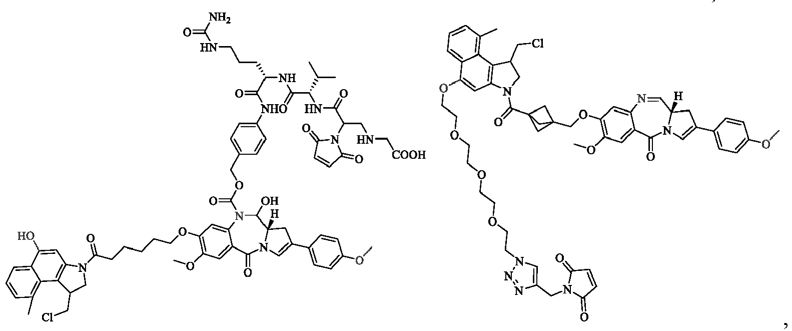 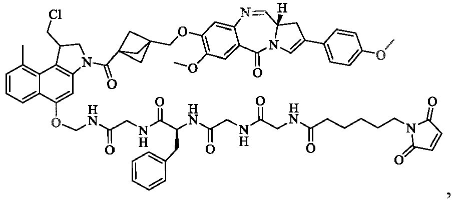 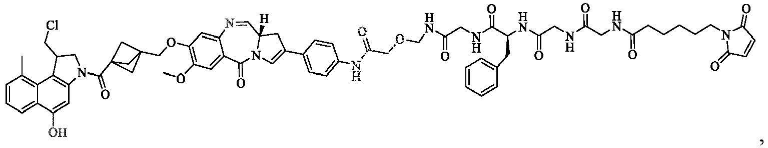 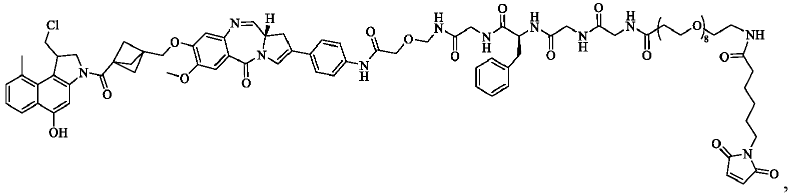 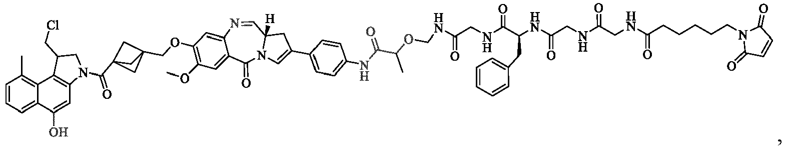 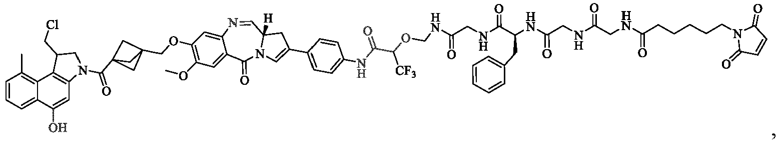 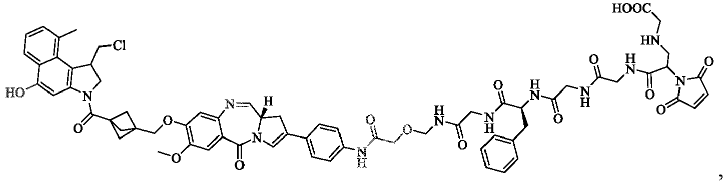 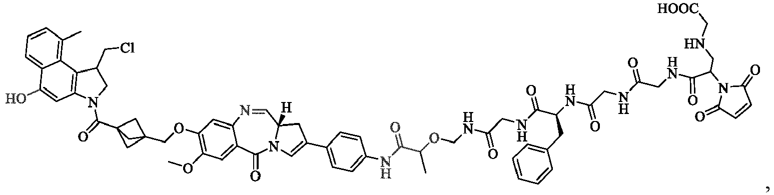 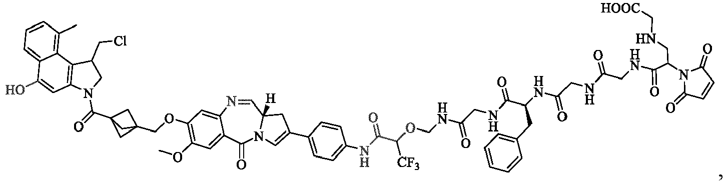 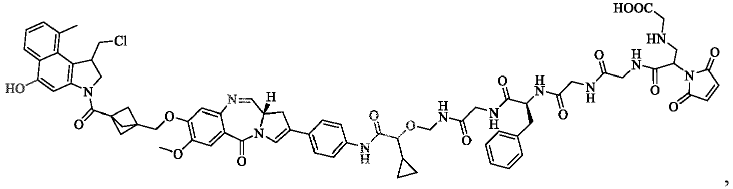 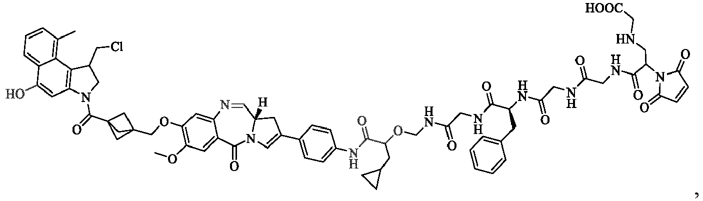 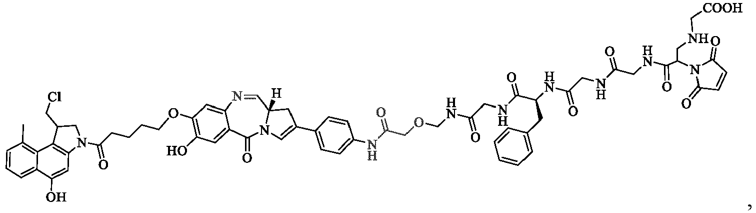 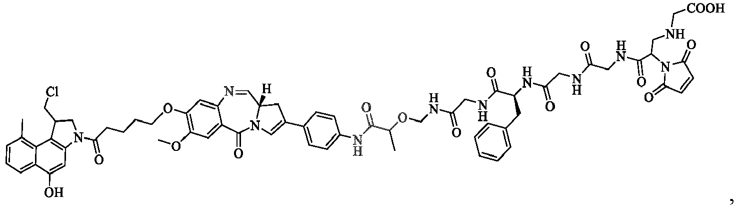 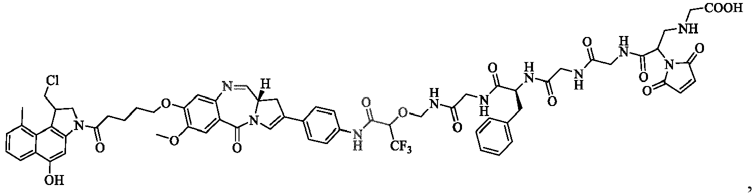 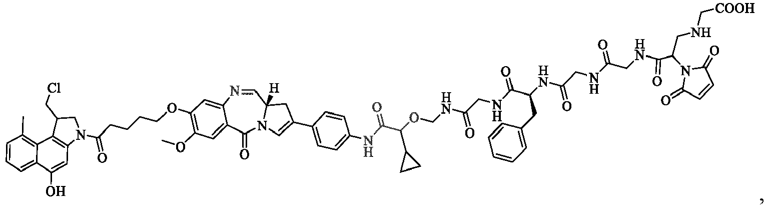 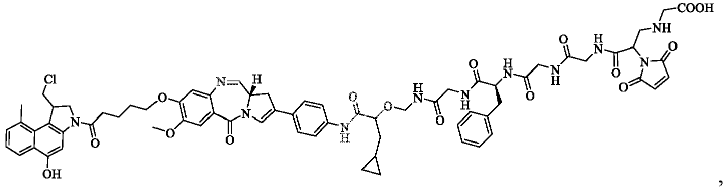 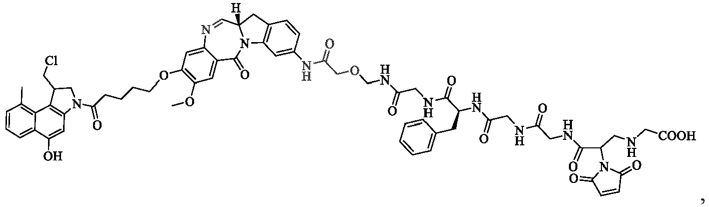 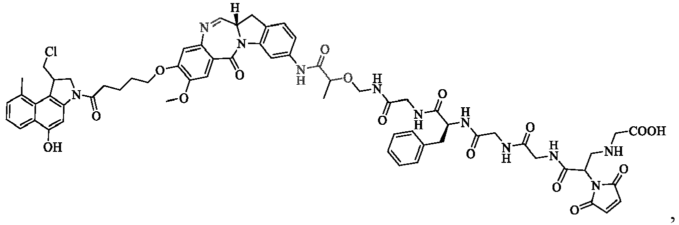 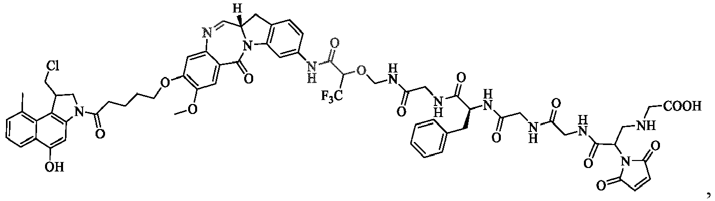 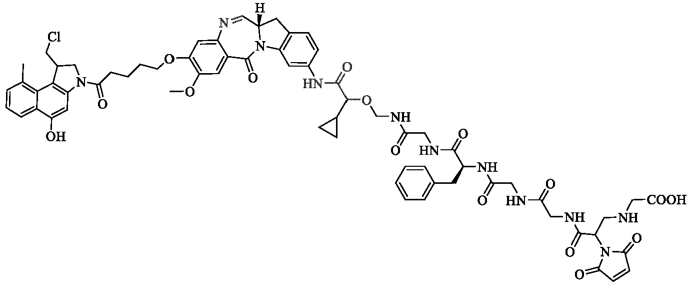 or

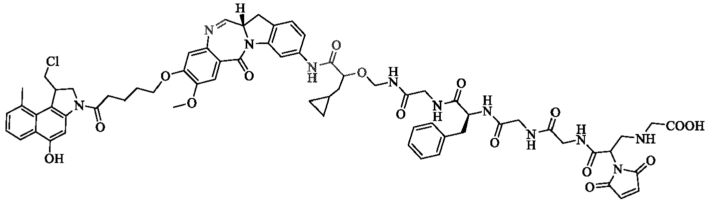

25. The ligand-drug conjugate or a pharmaceutically acceptable salt, deuterated derivative, solvate thereof according to claim 1, comprising the following: 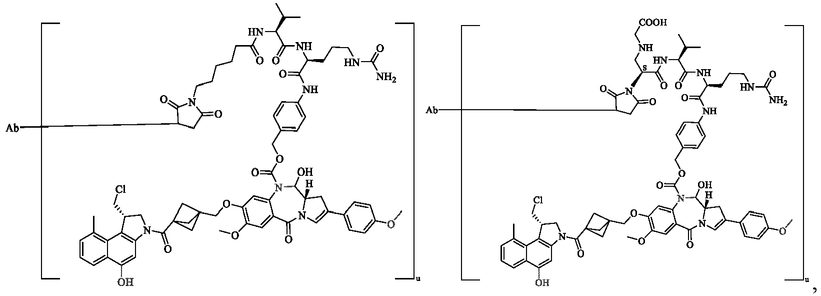 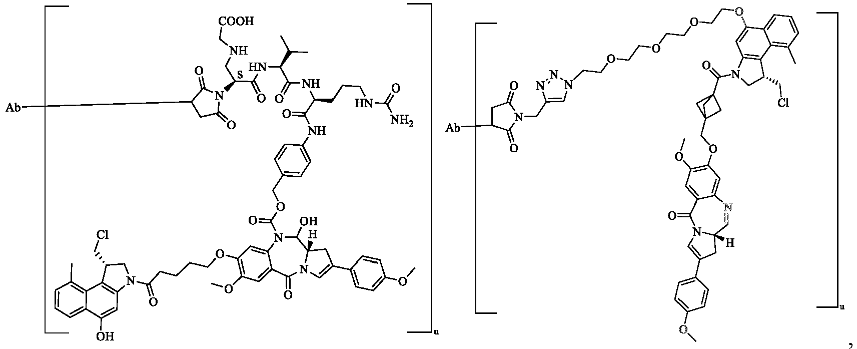 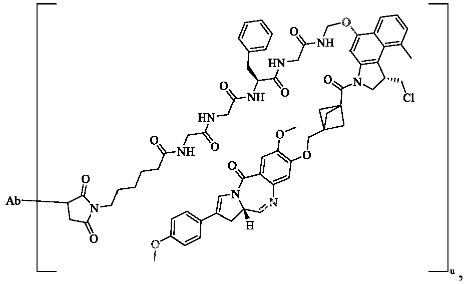 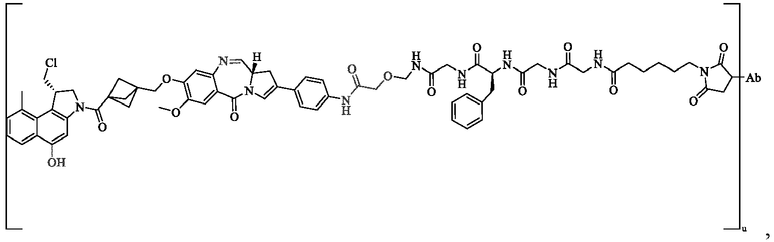 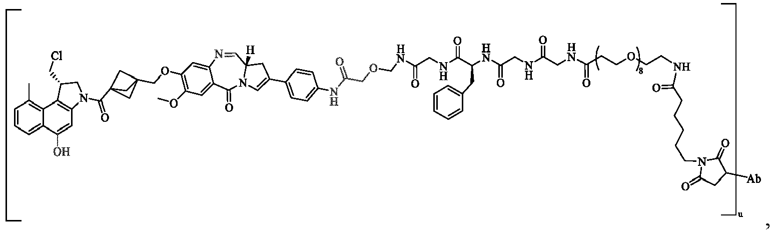 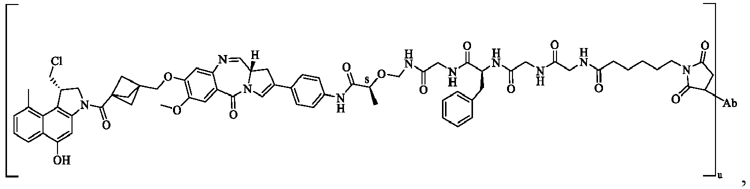 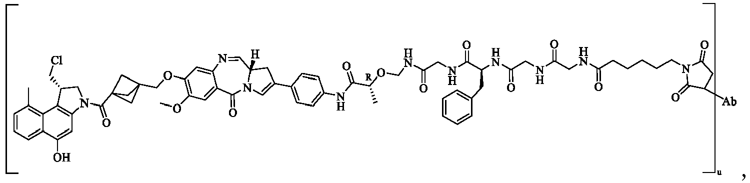 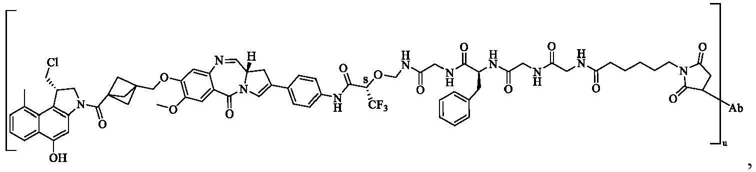 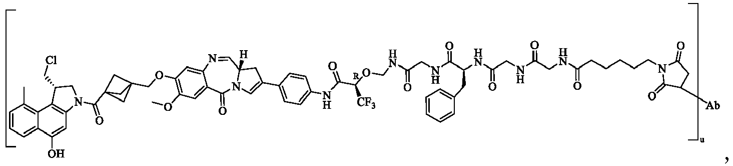 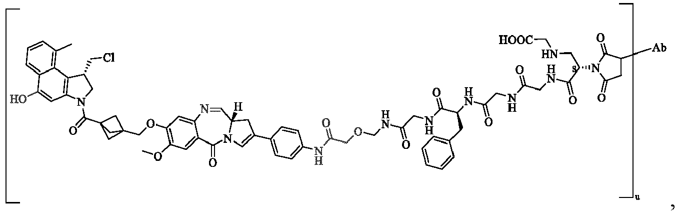 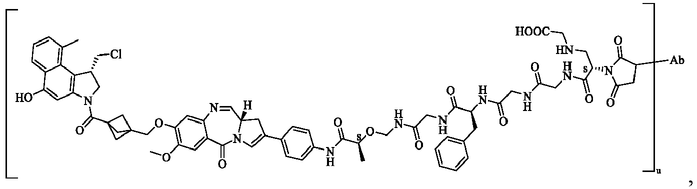 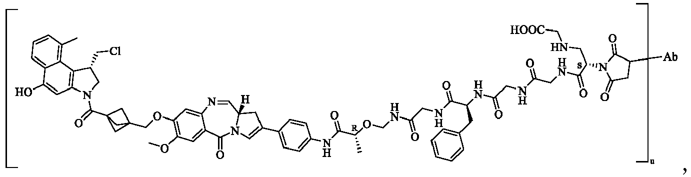 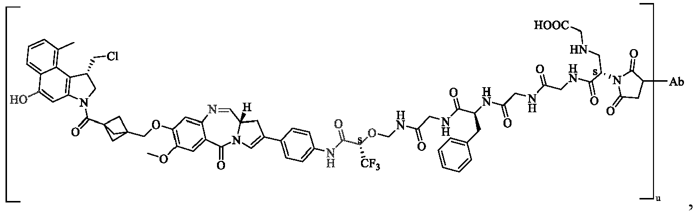 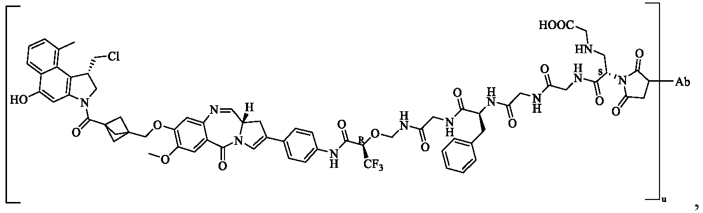 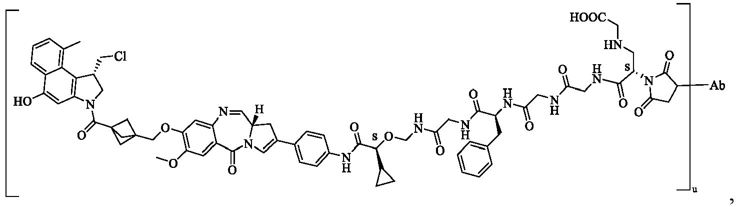 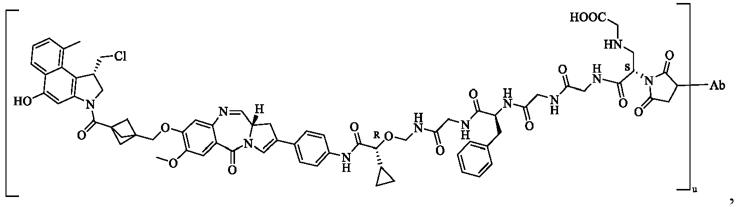 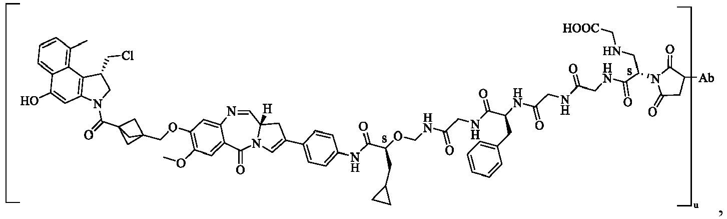 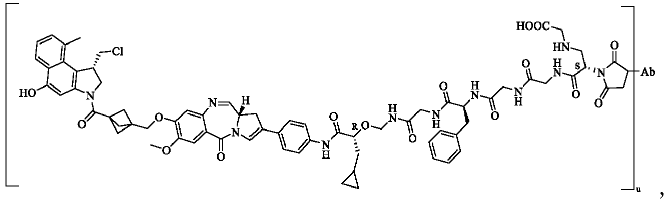 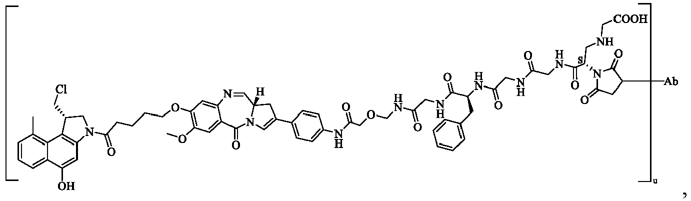 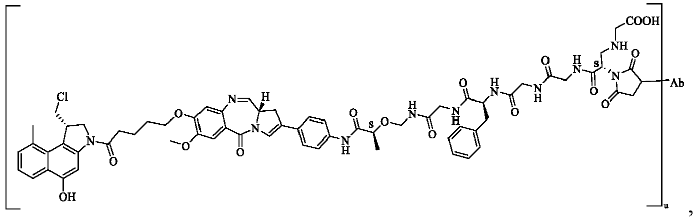 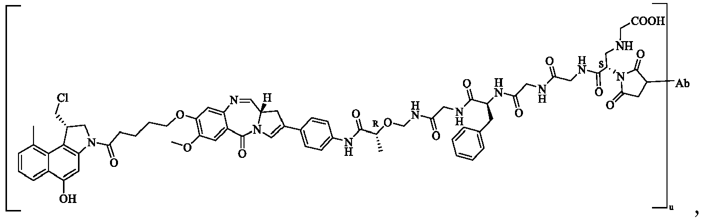 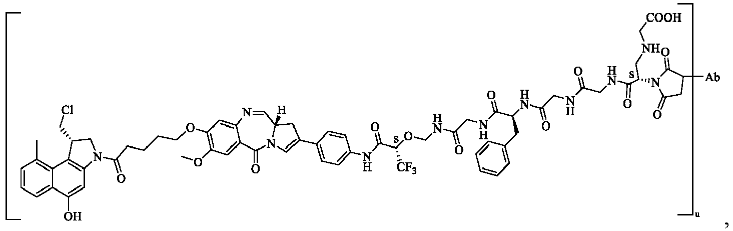 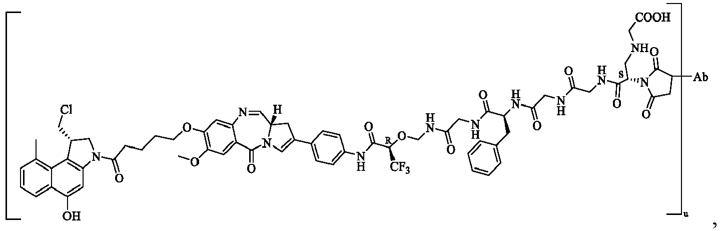 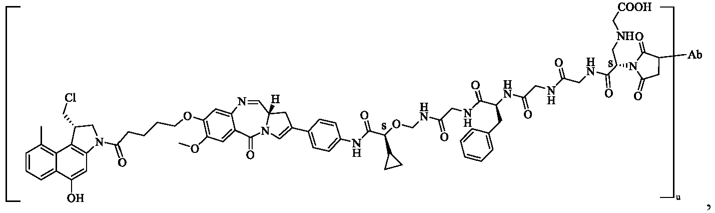 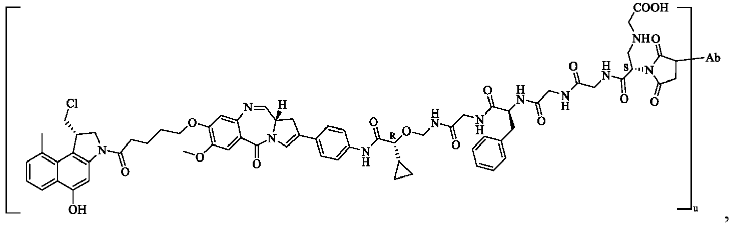 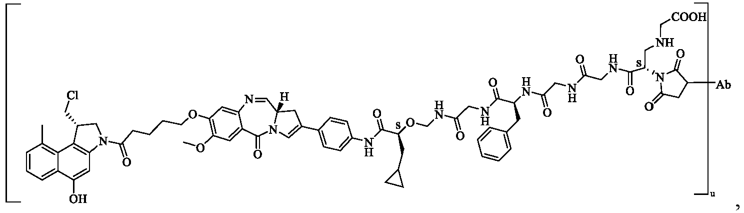 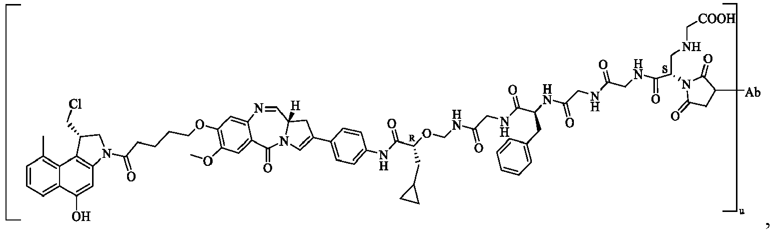 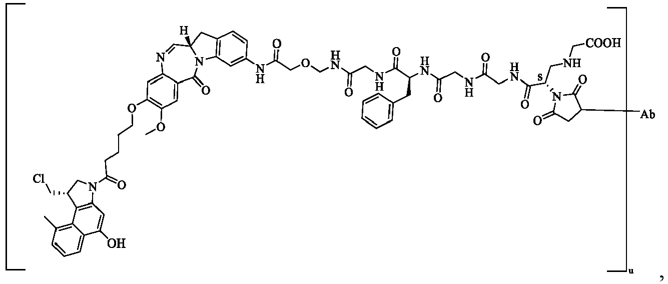 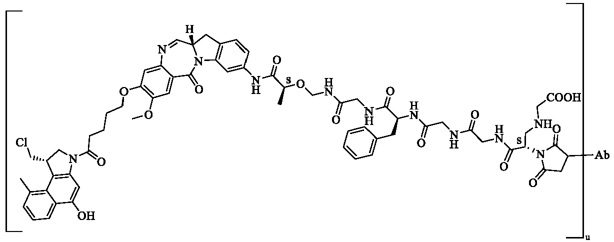 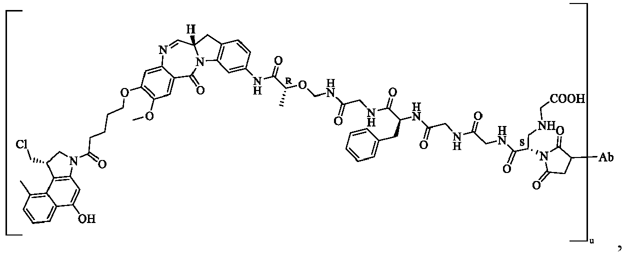 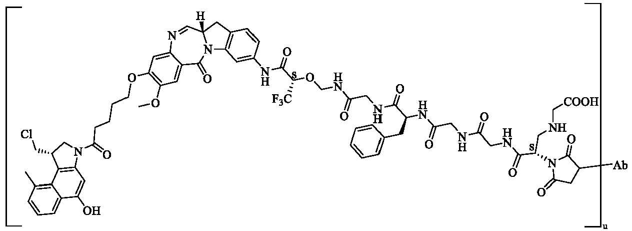 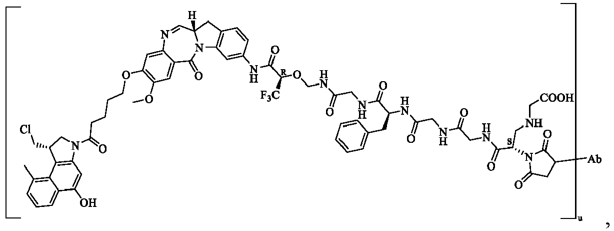 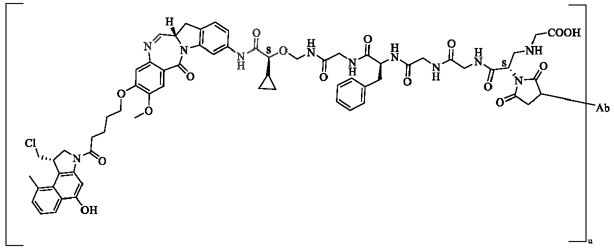 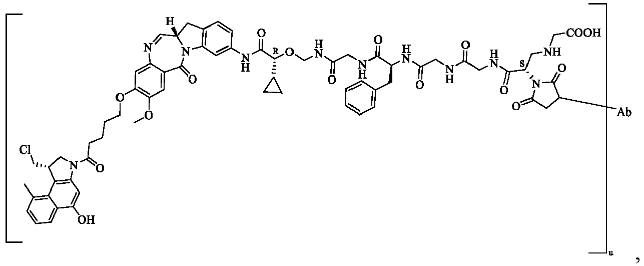 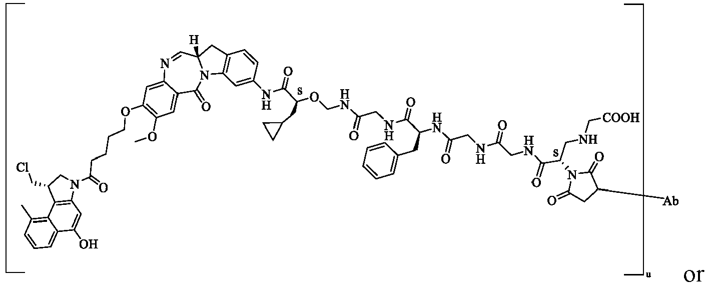 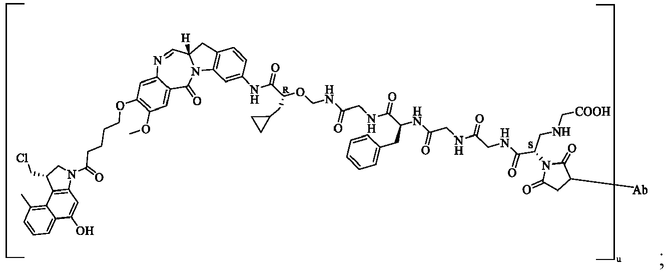 where u is selected from integers 1-10.
